# EUROPÄISCHE PATENTSCHRIFT

(11) **EP 2 099 751 B1**
(45) Veröffentlichungstag und Bekanntmachung des Hinweises auf die Patenterteilung: **30.05.2018**
(21) Anmeldenummer: 07856209.7
(22) Anmeldetag: 22.11.2007
(51) Int. Cl.: C07D 209/54, C07D 307/94, C07D 309/28, C07D 317/72, C07D 319/08, C07D 493/10, C07D 495/10, C07D 491/113, C07D 491/107, A01N 43/08, A01N 43/36, A01N 43/38, C07C 57/58, C07C 69/65, C07C 69/74

(54) **BIPHENYLSUBSTITUIERTE SPIROCYCLISCHE KETOENOLE**
BIPHENYL-SUBSTITUTED SPIROCYCLIC KETOENOLS
CÉTOÉNOLS SPIROCYCLIQUES SUBSTITUÉS PAR LE BIPHÉNYLE

(30) Priorität: 04.12.2006 DE 102006057036
(43) Veröffentlichungstag der Anmeldung: 16.09.2009
(73) Patentinhaber: Bayer Intellectual Property GmbH, 40789 Monheim am Rhein (DE)
(72) Erfinder: BRETSCHNEIDER, Thomas, 53797 Lohmar (DE); FISCHER, Reiner, 40789 Monheim (DE); PONTZEN, Rolf, 42799 Leichlingen (DE); ARNOLD, Christian, 40764 Langenfeld (DE); GÖRGENS, Ulrich, 40882 Ratingen (DE); MALSAM, Olga, 51503 Rösrath (DE); RECKMANN, Udo, 51063 Köln (DE); SANWALD, Erich, 24159 Kiel (DE); LEHR, Stefan, 65835 Liederbach (DE); DITTGEN, Jan, 60316 Frankfurt (DE); FEUCHT, Dieter, 65760 Eschborn (DE); HILLS, Martin, 65510 Idstein (DE); ROSINGER, Chris, 65719 Hofheim am Taunus (DE)
(74) Vertreter: BIP Patents
(86) Internationale Anmeldenummer: PCT/EP2007/010103
(87) Internationale Veröffentlichungsnummer: WO 2008/067911

(56) Entgegenhaltungen:
- EP-A- 0 596 298
- WO-A-99/48869
- WO-A-03/059065
- WO-A-2005/016873
- WO-A-2006/089633
- DE-A1- 2 623 464
- US-A- 3 928 330
- SHENGLOU DENG, DONGZHI LIU: "Studies on Phosphoroheterocycle Chemistry II: A Simple and New Route to 1,3,2-Diazaphospholidine-4-thione 2 sulfide Derivatives" SYNTHESIS, Nr. 16, 2001, Seiten 2445-2449, XP002470087
- NORBERT DE KIMPE ET AL: "Synthesis of 1-Amino-2,2-Dialkylcyclopropanecarboxylic Acids from beta-Chloroaldimines" TETRAHEDRON, Bd. 47, Nr. 26, 1991, Seiten 4723-4738, XP002470088

## Beschreibung

Die vorliegende Erfindung betrifft neue biphenylsubstituierte spirocyclische Ketoenole, mehrere Verfahren zu ihrer Herstellung und ihre Verwendung als Schädlingsbekämpfungsmittel und/oder Herbizide. Gegenstand der Erfindung sind auch selektiv herbizide Mittel, die Biphenyl- substituierte spirocyclische Ketoenole einerseits und eine die Kulturpflanzenverträglichkeit verbessernde Verbindung andererseits enthalten.

Die vorliegende Erfindung betrifft weiterhin die Steigerung der Wirkung von Pflanzenschutzmitteln enthaltend insbesondere biphenylsubstituierte spirocyclische Ketoenole, durch die Zugabe von Ammonium- oder Phosphoniumsalzen und gegebenenfalls Penetrationsförderern, die entsprechenden Mittel, Verfahren zu ihrer Herstellung und ihre Anwendung im Pflanzenschutz als Insektizide und/oder Akarizide und/oder zur Verhinderung von unerwünschten Pflanzenwuchs.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211 und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 95/01 997, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/16748, WO 99/24437, WO 99/43649, WO 99/48869 und WO 99/55673, WO 01/17972, WO 01/23354, WO 01/74770, WO 03/013249, WO 04/007448, WO 04/024688, WO 04/065366, WO 04/080962, WO 04/111042, WO 05/044791, WO 05/044796, WO 05/048710, WO 05/049596, WO 05/066125, WO 05/092897, WO 06/000355, WO 06/029799, WO 06/056281, WO 06/056282, WO 06/089633, WO 07/048545, und WO 07/073856, DE-05/059892, DE-06/007882, DE-06/018828, DE-06/025874, DE-06/050148).

Außerdem sind biphenylsubstituierte 1H-Pyrrolidin-dion Derivate mit fungizider Wirkung bekannt (WO 03/059065).

Es ist bekannt, dass bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A-0 647 637, WO 95/26 345, WO 96/20 196, WO 96/25 395, WO 96/35 664, WO 97/01 535, WO 97/02 243, WO 97/36 868, WO 98/05638, WO 98/25928, WO 99/16748, WO 99/43649, WO 99/48869, WO 99/55673, WO 01/17972, WO 01/23354, WO 01/74770, WO 03/013 249, WO 04/024 688, WO 04/080 962, WO 04/111 042, WO 05/092897, WO 06/000355, WO 06/029799, WO 06/089633, WO 07/048545, WO 07/073856, DE-06/007882 bekannt.

Die Wirksamkeit und Wirkungsbreite dieser Verbindungen ist jedoch insbesondere bei niedrigen Aufwandmengen und Konzentrationen nicht immer völlig zufriedenstellend. Weiterhin ist die Pflanzenverträglichkeit dieser Verbindungen gegenüber den Kulturpflanzen nicht immer ausreichend. Außerdem sind die toxikologischen Eigenschaften und/oder Umwelteigenschaften dieser Verbindungen nicht immer völlig zufriedenstellend.

Es wurden nun neue Verbindungen der Formel (I) 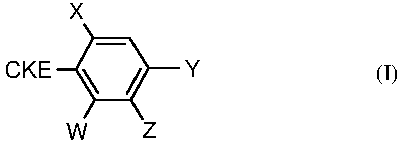 gefunden,
in welcher
- W: für Wasserstoff oder Methyl steht,
- X: für Chlor oder Methyl steht,
- Y: für Wasserstoff oder Methyl steht,
- Z: für den Rest 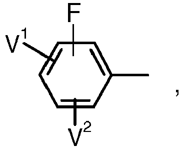 steht
- V¹: für Wasserstoff, Fluor, Chlor, Methyl, Methoxy oder Trifluormethyl steht,
- V²: für Wasserstoff oder Fluor steht,
- CKE: für die Gruppe 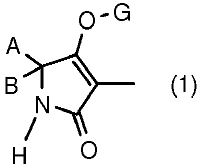 steht,
A, B und das Kohlenstoffatom, an das sie gebunden sind, stehen für gesättigtes C₆-Cycloalkyl, in welchem ein Ringglied durch Sauerstoff ersetzt ist und welches gegebenenfalls einfach durch Methyl oder Ethyl, substituiert ist,
- G: für Wasserstoff (a) oder für eine der Gruppen 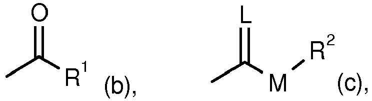 oder E (f) steht,
in welchen
- L: für Sauerstoff steht,
- M: für Sauerstoff steht und
- E: für ein Metallionenäquivalent oder ein Ammoniumion steht,
R¹ für jeweils gegebenenfalls einfach durch Fluor oder Chlor substituiertes C₁-C₆-Alkyl, C₂-C₆-Alkenyl, C₁-C₂-Alkoxy-C₁-alkyl, C₁-C₂-Alkylthio-C₁-alkyl oder jeweils gegebenenfalls einfach durch Fluor, Chlor, Methyl oder Methoxy substituiertes Cyclopropyl oder Cyclohexyl, für gegebenenfalls einfach durch Fluor, Chlor, Brom, Cyano, Nitro, Methyl, Methoxy, Trifluormethyl oder Trifluormethoxy substituiertes Phenyl steht,
R² für jeweils gegebenenfalls einfach durch Fluor substituiertes C₁-C₈-Alkyl, C₂-C₆-Alkenyl oder C₁-C₄-Alkoxy-C₂-C₃-alkyl, Phenyl oder Benzyl steht.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutung (1) der Gruppe CKE ergibt sich folgende hauptsächliche Struktur (I-1): 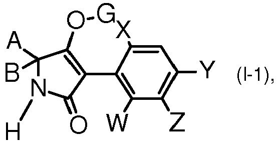 worin
A, B, G, W, X, Y und Z die oben angegebene Bedeutung haben.

Unter Einbeziehung der verschiedenen Bedeutungen (a), (b), (c) und (f) der Gruppe G ergeben sich folgende hauptsächliche Strukturen (I-1-a), (I-1-b), (I-1-c), (I-1-f), wenn CKE für die Gruppe (1) steht, 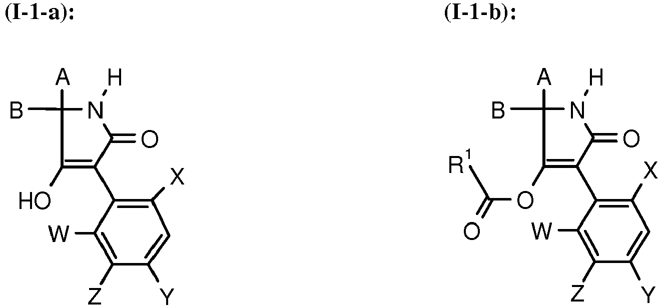 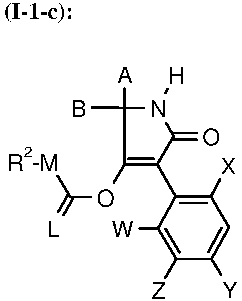 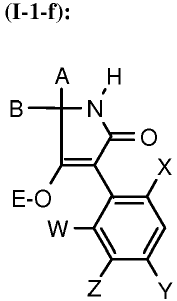 worin
A, B, E, L, M, W, X, Y, Z, R¹, R² die oben angegebenen Bedeutungen besitzen.

Weiterhin wurde gefunden, daß man die neuen Verbindungen der Formel (I) nach einem der im folgenden beschriebenen Verfahren erhält:
(A) Man erhält substituierte 3-Biphenylpyrrolidin-2,4-dione bzw. deren Enole der Formel (I-1-a) 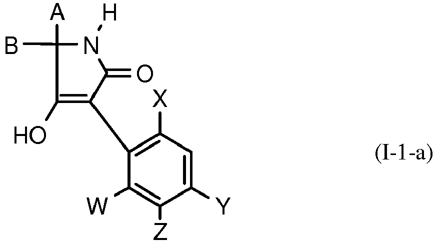 in welcher
   A, B, W, X, Y und Z die oben angegebenen Bedeutungen haben,
   wenn man
   N-Acylaminosäureester der Formel (II) 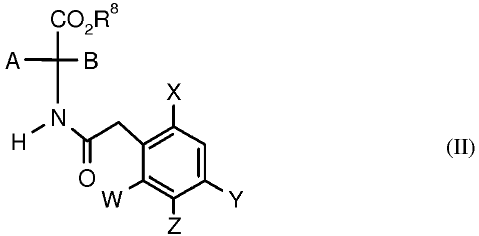 in welcher
   A, B, W, X, Y und Z die oben angegebenen Bedeutungen haben,
   und
   R⁸ für Alkyl (bevorzugt C₁-C₆-Alkyl) steht,
   in Gegenwart eines Verdünnungsmittels und in Gegenwart einer Base intramolekular kondensiert.
(C) Weiterhin wurde gefunden, daß man Verbindungen der oben gezeigten Formeln (I-1-a), (I-1-b), (I-1-c), (I-1-f), in welchen A, B, G, W, X, Y und Z die oben angegebene Bedeutung haben, erhält, wenn man Verbindungen der Formel (I-1'-a), (I-1'-b), (I-1'-c), (I-1'-f), (**I-1'-a**), (I-1'-b), (I-1'-c), (I-1'-f) 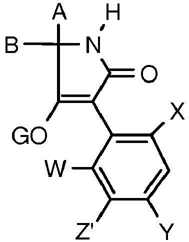 in welchen
   A, B, G, W, X und Y die oben angegebene Bedeutung haben und
   Z' für Chlor, Brom, Jod, bevorzugt für Brom steht,
   mit Boronsäuren oder Boronsäure-Derivaten der Formel (IV) 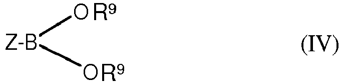 in welcher
   - R⁹: für Wasserstoff, C₁-C₆-Alkyl oder C₂-C₆-Alkandiyl steht
   und
   Z die oben angegebene Bedeutung hat,
in Gegenwart eines Lösungsmittels, einer Base und eines Katalysators umsetzt, wobei als Katalysator insbesondere Palladiumsalze oder Palladiumkomplexe in Frage kommen.

Außerdem wurde gefunden
- (D): daß man die Verbindungen der oben gezeigten Formel (I-1-b), in welchen A, B, R¹, W, X, Y und Z die oben angebenen Bedeutungen haben, erhält, wenn man Verbindungen der oben gezeigten Formel (I-1-a), in welchen A, B, W, X, Y und Z die oben angegebenen Bedeutungen haben, jeweils
- (α): mit Säurehalogeniden der Formel (V) 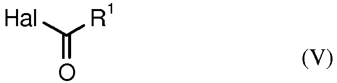 in welcher
- R¹: die oben angegebene Bedeutung hat und
- Hal: für Halogen (insbesondere Chlor oder Brom) steht
oder
(β) mit Carbonsäureanhydriden der Formel (VI)

   R¹-CO-O-CO-R¹ (VI)

   in welcher
   - R¹: die oben angegebene Bedeutung hat,
gegebenenfalls in Gegenwart eines Verdünnungsmittels und gegebenenfalls in Gegenwart eines Säurebindemittels umsetzt;
(E) daß man die Verbindungen der oben gezeigten Formel (I-1-c), in welchen A, B, R², M, W, X, Y und Z die oben angegebenen Bedeutungen haben und L für Sauerstoff steht, erhält, wenn man Verbindungen der oben gezeigten Formel (I-1-a), in welchen A, B, W, X, Y und Z die oben angegebenen Bedeutungen haben, jeweils
   mit Chlorameisensäureestern der Formel (VII)

   R²-M-CO-Cl (VII)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
   (
   (I) daß man Verbindungen der oben gezeigten Formel (I-1-f), in welchen A, B, E, W, X, Y und Z die oben angegebenen Bedeutungen haben, erhält, wenn man Verbindungen der oben gezeigten Formel (I-1-a), in welchen A, B, W, X, Y und Z die oben angegebenen Bedeutungen haben, jeweils
mit Metallverbindungen oder Aminen der Formeln (XI) oder (XII)

Me(OR¹⁰)ₜ (XI)

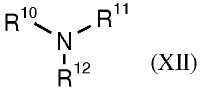

in welchen
Me für ein ein- oder zweiwertiges Metall (bevorzugt ein Alkali- oder Erdalkalimetall wie Lithium, Natrium, Kalium, Magnesium oder Calcium),
t für die Zahl 1 oder 2 und
R¹⁰, R¹¹, R¹² unabhängig voneinander für Wasserstoff oder Alkyl (bevorzugt C₁-C₈-Alkyl) stehen,
gegebenenfalls in Gegenwart eines Verdünnungsmittels umsetzt,

Weiterhin wurde gefunden, dass die neuen Verbindungen der Formel (I) eine gute Wirksamkeit als Schädlingsbekämpfungsmittel, vorzugsweise als Insektizide und/oder Akarizide und/oder Herbizide aufweisen, darüber hinaus häufig insbesondere gegenüber Kulturpflanzen, sehr gut pflanzenverträglich sind und/oder über günstige toxikologische und/oder umweltrelevante Eigenschaften verfügen.

Überraschenderweise wurde nun auch gefunden, dass bestimmte biphenyl-substituierte, spirocyclische Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
(a') mindestens ein biphenylsubstituiertes, spirocyclisches Ketoenol der Formel (I), in welcher CKE, W, X, Y und Z die oben angegebene Bedeutung haben und
(b') zumindest eine die Kulturpflanzen-Verträglichkeit verbessernde Verbindung aus der folgenden Gruppe von Verbindungen:
   4-Dichloracetyl-1-oxa-4-aza-spiro[4.5]-decan (AD-67, MON-4660), 1-Dichloracetyl-hexa-hydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlorbenzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-a-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazol-carboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyl-oxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlorphenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethylsulfonyl-benzol, 1-[4-(N-2-Methoxybenzoyl-sulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxybenzoylsulfamoyl)-phenyl] -3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyll-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa) 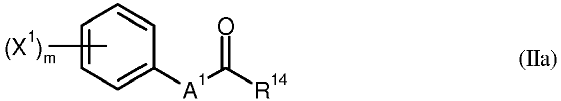 oder der allgemeinen Formel (IIb) 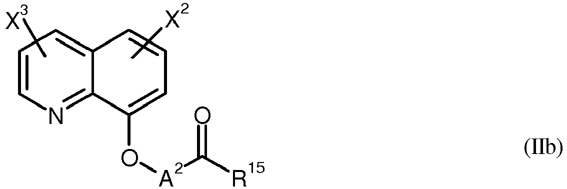 oder der Formel (IIc) 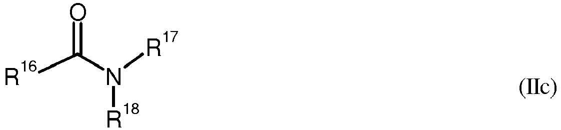 wobei
- m: für eine Zahl 0, 1, 2, 3, 4 oder 5 steht,
- A¹: für eine der nachstehend skizzierten divalenten heterocyclischen Gruppierungen steht, 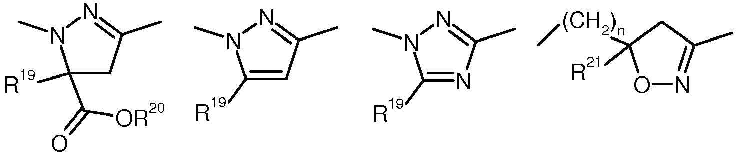
- n: für eine Zahl 0, 1, 2, 3, 4 oder 5 steht,
- A²: für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
- R¹⁴: für Hydroxy, Mercapto, Amino, C₁-C₆-Alkoxy, C₁-C₆-Alkylthio, C₁-C₆-Alkylamino oder Di-(C₁-C₄-alkyl)-amino steht,
- R¹⁵: für Hydroxy, Mercapto, Amino, C₁-C₇-Alkoxy, C₁-C₆-Alkenyloxy, C₁-C₆-Alkenyloxy-C₁-C₆-alkoxy, C₁-C₆-Alkylthio, C₁-C₆-Alkylamino oder Di-(C₁-C₄-alkyl)-amino steht,
- R¹⁶: für gegebenenfalls durch Fluor, Chlor und/oder Brom substituiertes C₁-C₄-Alkyl steht,
- R¹⁷: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
- R¹⁸: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht, R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅-Oxaalkandiyl steht,
- R¹⁹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- R²⁰: für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
- R²¹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- X¹: für Nitro, Cyano, Halogen, C₁-C₄-Alkyl, C₁-C₄-Halogenalkyl, C₁-C₄-Alkoxy oder C₁-C₄-Halogenalkoxy steht,
- X²: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
- X³: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 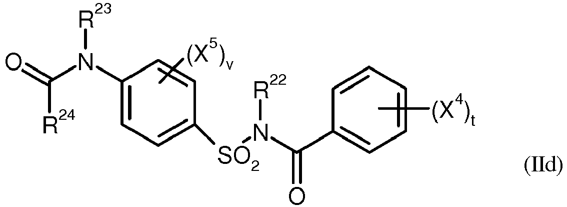 oder der allgemeinen Formel (IIe) 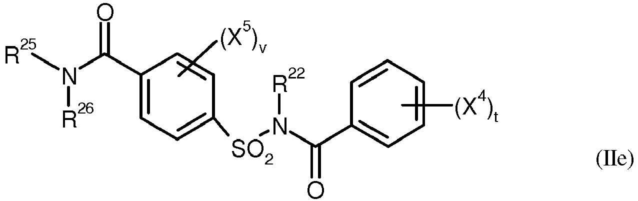 wobei
- t: für eine Zahl 0, 1, 2, 3, 4 oder 5 steht,
- v: für eine Zahl 0, 1, 2, 3, 4 oder 5 steht,
- R²²: für Wasserstoff oder C₁-C₄-Alkyl steht,
- R²³: für Wasserstoff oder C₁-C₄-Alkyl steht,
- R²⁴: für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkylthio oder C₃-C₆-Cycloalkylamino steht,
- R²⁵: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
- R²⁶: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxaalkandiyl steht,
- X⁴: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
- X⁵: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß insbesonders bevorzugt werden die Verbindungen der Formel (I), in welchen eine Kombination der vorstehend als insbesonders bevorzugt aufgeführten Bedeutungen vorliegt.

Hervorgehoben sind Verbindungen mit

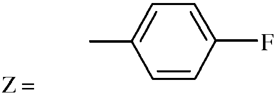

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Beispielen genannten Verbindungen die folgenden Verbindungen der Formel (I) genannt:

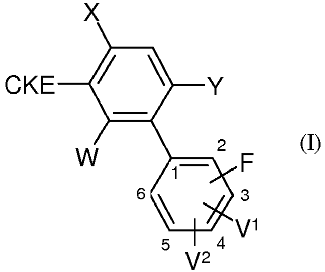

**Tabelle 1**

| **W** | **X** | **Y** | **F** | **V¹** | **V²** |
|---|---|---|---|---|---|
| H | Cl | H | 2 | H | H |
| H | Cl | H | 3 | H | H |
| H | Cl | H | 4 | H | H |
| H | Cl | H | 2 | 4-F | H |
| H | Cl | H | 2 | 4-Cl | H |
| H | Cl | H | 2 | 4-CH₃ | H |
| H | Cl | H | 2 | 4-OCH₃ | H |
| H | Cl | H | 3 | 4-F | H |
| H | Cl | H | 3 | 4-Cl | H |
| H | Cl | H | 3 | 4-CH₃ | H |
| H | Cl | H | 3 | 4-OCH₃ | H |
| H | Cl | H | 4 | 3-Cl | H |
| H | Cl | H | 4 | 3-CH₃ | H |
| H | Cl | H | 4 | 3-OCH₃ | H |
| H | Cl | H | 2 | 4-F | 5-F |
| H | Cl | H | 2 | 4-F | 6-F |
| H | Cl | H | 2 | 4-Cl | 5-F |
| H | Cl | H | 2 | 5-Cl | 4-F |
| H | Cl | H | 3 | 4-F | 5-F |
| H | CH₃ | H | 2 | H | H |
| H | CH₃ | H | 3 | H | H |
| H | CH₃ | H | 4 | H | H |
| H | CH₃ | H | 2 | 4-F | H |
| H | CH₃ | H | 2 | 4-Cl | H |
| H | CH₃ | H | 2 | 4-CH₃ | H |
| H | CH₃ | H | 2 | 4-OCH₃ | H |
| H | CH₃ | H | 3 | 4-F | H |
| H | CH₃ | H | 3 | 4-Cl | H |
| H | CH₃ | H | 3 | 4-CH₃ | H |
| H | CH₃ | H | 3 | 4-OCH₃ | H |
| H | CH₃ | H | 4 | 3-Cl | H |
| H | CH₃ | H | 4 | 3-CH₃ | H |
| H | CH₃ | H | 4 | 3-OCH₃ | H |
| H | CH₃ | H | 2 | 4-F | 5-F |
| H | CH₃ | H | 2 | 4-F | 6-F |
| H | CH₃ | H | 2 | 4-Cl | 5-F |
| H | CH₃ | H | 2 | 5-Cl | 4-F |
| H | CH₃ | H | 3 | 4-F | 5-F |
| CH₃ | CH₃ | H | 2 | H | H |
| CH₃ | CH₃ | H | 3 | H | H |
| CH₃ | CH₃ | H | 4 | H | H |
| CH₃ | CH₃ | H | 2 | 4-F | H |
| CH₃ | CH₃ | H | 2 | 4-Cl | H |
| CH₃ | CH₃ | H | 2 | 4-CH₃ | H |
| CH₃ | CH₃ | H | 2 | 4-OCH₃ | H |
| CH₃ | CH₃ | H | 3 | 4-F | H |
| CH₃ | CH₃ | H | 3 | 4-Cl | H |
| CH₃ | CH₃ | H | 3 | 4-CH₃ | H |
| CH₃ | CH₃ | H | 3 | 4-OCH₃ | H |
| CH₃ | CH₃ | H | 4 | 3-Cl | H |
| CH₃ | CH₃ | H | 4 | 3-CH₃ | H |
| CH₃ | CH₃ | H | 4 | 3-OCH₃ | H |
| CH₃ | CH₃ | H | 2 | 4-F | 5-F |
| CH₃ | CH₃ | H | 2 | 4-F | 6-F |
| CH₃ | CH₃ | H | 2 | 4-Cl | 5-F |
| CH₃ | CH₃ | H | 2 | 5-Cl | 4-F |
| CH₃ | CH₃ | H | 3 | 4-F | 5-F |
| H | CH₃ | CH₃ | 2 | H | H |
| H | CH₃ | CH₃ | 3 | H | H |
| H | CH₃ | CH₃ | 4 | H | H |
| H | CH₃ | CH₃ | 2 | 4-F | H |
| H | CH₃ | CH₃ | 2 | 4-Cl | H |
| H | CH₃ | CH₃ | 2 | 4-CH₃ | H |
| H | CH₃ | CH₃ | 2 | 4-OCH₃ | H |
| H | CH₃ | CH₃ | 3 | 4-F | H |
| H | CH₃ | CH₃ | 3 | 4-Cl | H |
| H | CH₃ | CH₃ | 3 | 4-CH₃ | H |
| H | CH₃ | CH₃ | 3 | 4-OCH₃ | H |
| H | CH₃ | CH₃ | 4 | 3-Cl | H |
| H | CH₃ | CH₃ | 4 | 3-CH₃ | H |
| H | CH₃ | CH₃ | 4 | 3-OCH₃ | H |
| H | CH₃ | CH₃ | 2 | 4-F | 5-F |
| H | CH₃ | CH₃ | 2 | 4-F | 6-F |
| H | CH₃ | CH₃ | 2 | 4-Cl | 5-F |
| H | CH₃ | CH₃ | 2 | 5-Cl | 4-F |
| H | CH₃ | CH₃ | 3 | 4-F | 5-F |
| CH₃ | CH₃ | CH₃ | 2 | H | H |
| CH₃ | CH₃ | CH₃ | 3 | H | H |
| CH₃ | CH₃ | CH₃ | 4 | H | H |
| CH₃ | CH₃ | CH₃ | 2 | 4-F | H |
| CH₃ | CH₃ | CH₃ | 2 | 4-Cl | H |
| CH₃ | CH₃ | CH₃ | 2 | 4-CH₃ | H |
| CH₃ | CH₃ | CH₃ | 2 | 4-OCH₃ | H |
| CH₃ | CH₃ | CH₃ | 3 | 4-F | H |
| CH₃ | CH₃ | CH₃ | 3 | 4-Cl | H |
| CH₃ | CH₃ | CH₃ | 3 | 4-CH₃ | H |
| CH₃ | CH₃ | CH₃ | 3 | 4-OCH₃ | H |
| CH₃ | CH₃ | CH₃ | 4 | 3-Cl | H |
| CH₃ | CH₃ | CH₃ | 4 | 3-CH₃ | H |
| CH₃ | CH₃ | CH₃ | 4 | 3-OCH₃ | H |
| CH₃ | CH₃ | CH₃ | 2 | 4-F | 5-F |
| CH₃ | CH₃ | CH₃ | 2 | 4-F | 6-F |
| CH₃ | CH₃ | CH₃ | 2 | 4-Cl | 5-F |
| CH₃ | CH₃ | CH₃ | 2 | 5-Cl | 4-F |
| CH₃ | CH₃ | CH₃ | 3 | 4-F | 5-F |

Als erfindungsgemäße Wirkstoffe kommen insbesondere bevorzugt Verbindungen aus mit den in Tabelle 1 genannten Restekombinationen für W, X, Y, F, V¹ und V² mit den in Tabelle 2 genannten Restekombinationen für A und B in Frage.
CKE = (1)

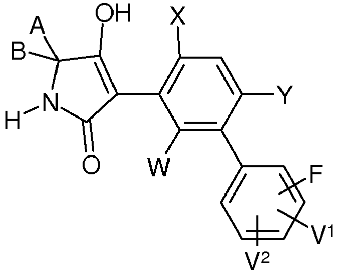

**Tabelle 2**

| **A** | **B** |
|---|---|
| -(CH₂)₂- | |
| -(CH₂)₄- | |
| -(CH₂)₅- | |
| -(CH₂)₆- | |
| -(CH₂)₇- | |
| -CH₂-CHCH₃-O-(CH₂)₂- | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH₂)₃- | |
| -CH₂-CHOCH₃-(CH₂)₂- | |
| -CH₂-CHOC₂H₅-(CH₂)₂- | |
| -CH₂-CHOC₃H₇-(CH₂)₂- | |
| -CH₂-CHOC₄H₉-(CH₂)₂- | |
| -CH₂-CHO(CH₂)₂OCH₃-(CH₂)₂- | |
| 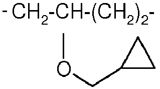 | |
| -CH₂-CHOCH₃-(CH₂)₃ | |
| -CH₂-CHOC₂H₅-(CH₂)₃ | |
| -CH₂-CHOC₃H₇-(CH₂)₃ | |
| -CH₂-CHOC₄H₉-(CH₂)₃ | |
| -CH₂-CHO(CH₂)₂OCH₃-(CH₂)₃ | |
| 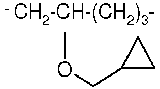 | |
| -(CH₂)₂-CHCH₃-(CH₂)₂ | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-C(CH₃)₂-(CH₂)₂ | |
| -CH₂-(CHCH₃)₂-(CH₂)₂- | |
| 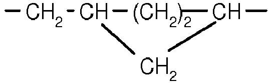 | |
| 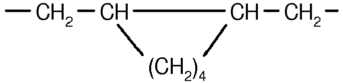 | |
| 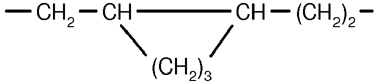 | |
| 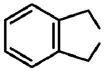 | |
| 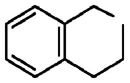 | |
| 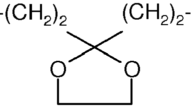 | |
| 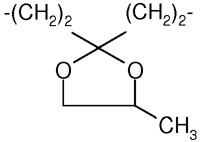 | |
| 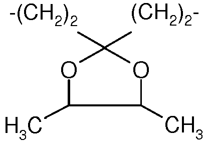 | |
| 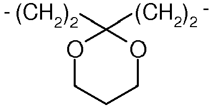 | |
| 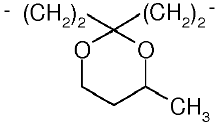 | |
| 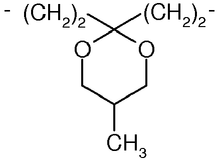 | |
| 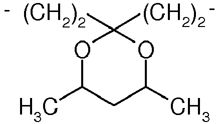 | |
| 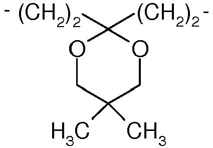 | |
| 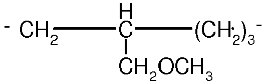 | |
| 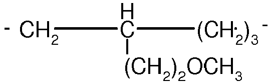 | |
| 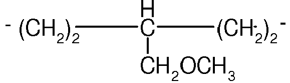 | |
| 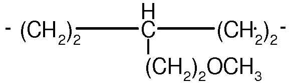 | |
| 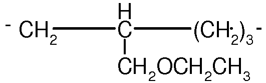 | |
| 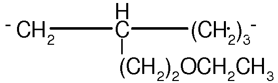 | |
| 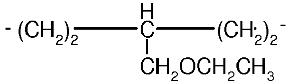 | |
| 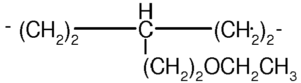 | |

Bevorzugte Bedeutungen der oben in Zusammenhang mit den die Kulturpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.
- m: steht bevorzugt für die Zahlen 0, 1, 2, 3 oder 4.
- A¹: steht bevorzugt für eine der nachstehend skizzierten divalenten heterocyclischen Gruppierungen 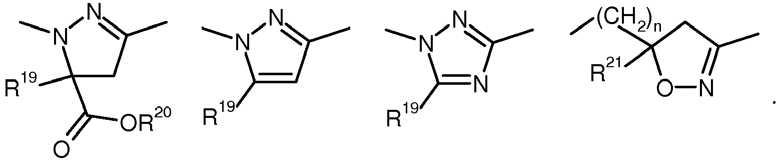
- n: steht bevorzugt für die Zahlen 0, 1, 2, 3 oder 4.
- A²: steht bevorzugt für jeweils gegebenenfalls durch Methyl, Ethyl, Methoxycarbonyl, Ethoxycarbonyl oder Alkyloxycarbonyl substituiertes Methylen oder Ethylen.
- R¹⁴: steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁵: steht bevorzugt für Hydroxy, Mercapto, Amino, Methoxy, Ethoxy, n- oder i-Propoxy, n-, i-, s- oder t-Butoxy, 1-Methyl-hexyloxy, Allyloxy, 1-Allyloxymethyl-ethoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁶: steht bevorzugt für jeweils gegebenenfalls durch Fluor, Chlor und/oder Brom substituiertes Methyl, Ethyl, n- oder i-Propyl.
- R¹⁷: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.
- R¹⁸: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl, oder zusammen mit R¹⁷ für einen der Reste -CH₂-O-CH₂-CH₂- und -CH₂-CH₂-O-CH₂-CH₂-, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.
- R¹⁹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.
- R²⁰: steht bevorzugt für Wasserstoff, jeweils gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s-oder t-Butyl.
- R²¹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s-oder t-Butyl, Cyclopropyl, Cyclobutyl, Cyclopentyl, Cyclohexyl oder Phenyl.
- X¹: steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X²: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X³: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- t: steht bevorzugt für die Zahlen 0, 1, 2, 3 oder 4.
- v: steht bevorzugt für die Zahlen 0, 1, 2, 3 oder 4.
- R²²: steht bevorzugt für Wasserstoff, Methyl, Ethyl, n- oder i-Propyl.
- R²³: steht bevorzugt für Wasserstoff, Methyl, Ethyl, n- oder i-Propyl.
- R²⁴: steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclo-propylamino, Cyclobutylamino, Cyclopentylamino oder Cyclohexylamino.
- R²⁵: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.
- R²⁶: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, oder zusammen mit R²⁵ für jeweils gegebenenfalls durch Methyl oder Ethyl substituiertes Butan-1,4-diyl (Trimethylen), Pentan-1,5-diyl, 1-Oxa-butan-1,4-diyl oder 3-Oxa-pentan-1,5-diyl.
- X⁴: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.
- X⁵: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle aufgeführt.

**Tabelle: Beispiele für die Verbindungen der Formel (IIa)**

| | | | |
|---|---|---|---|
| 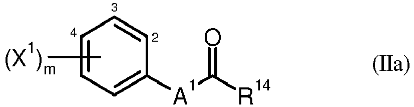 | | | |

| **Beispiel-Nr.** | **(Positionen) (X¹)ₘ** | **A¹** | **R¹⁴** |
|---|---|---|---|
| IIa-1 | (2) Cl, (4) Cl | 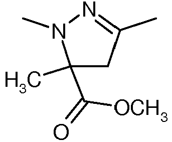 | OCH₃ |
| IIa-2 | (2) Cl, (4) Cl | 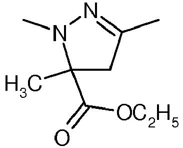 | OCH₃ |
| IIa-3 | (2) Cl, (4) Cl | 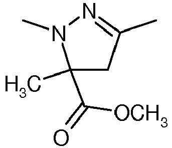 | OC₂H₅ |
| IIa-4 | (2) Cl, (4) Cl | 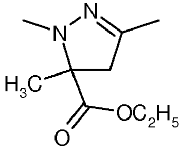 | OC₂H₅ |
| IIa-5 | (2) Cl | 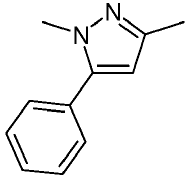 | OCH₃ |
| IIa-6 | (2) Cl, (4) Cl | 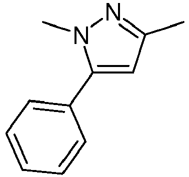 | OCH₃ |
| IIa-7 | (2) F | 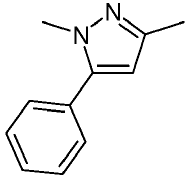 | OCH₃ |
| IIa-8 | (2) F | 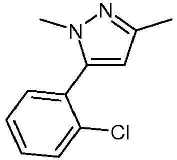 | OCH₃ |
| IIa-9 | (2) Cl, (4) Cl | 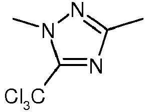 | OC₂H₅ |
| IIa-10 | (2) Cl, (4) CF₃ | 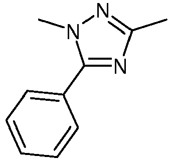 | OCH₃ |
| IIa-11 | (2) Cl | 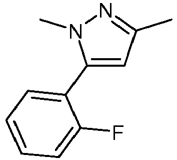 | OCH₃ |
| IIa-12 | - | 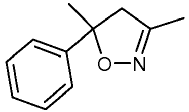 | OC₂H₅ |
| Ha-13 | (2) Cl, (4) Cl | 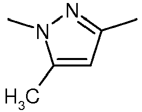 | OC₂H₅ |
| IIa-14 | (2) Cl, (4) Cl | 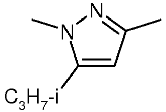 | OC₂H₅ |
| IIa-15 | (2) Cl, (4) Cl | 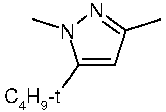 | OC₂H₅ |
| IIa-16 | (2) Cl, (4) Cl | 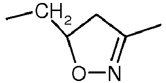 | OC₂H₅ |
| IIa-17 | (2) Cl, (4) Cl | 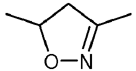 | OC₂H₅ |
| IIa-18 | - | 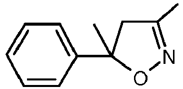 | OH |

Beispiele für die als erfindungsgemäße Herbizid-Safener ganz besonders bevorzugten Verbindungen der Formel (IIb) sind in der nachstehenden Tabelle aufgeführt.

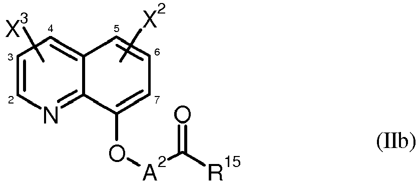

**Tabelle: Beispiele für die Verbindungen der Formel (IIb)**

| **Beispiel-Nr.** | **(Position) X²** | **(Position) X³** | **A²** | **R¹⁵** |
|---|---|---|---|---|
| IIb-1 | (5) Cl | - | CH₂ | OH |
| IIb-2 | (5) Cl | - | CH₂ | OCH₃ |
| IIb-3 | (5) Cl | - | CH₂ | OC₂H₅ |
| IIb-4 | (5) Cl | - | CH₂ | OC₃H₇-n |
| IIb-5 | (5) Cl | - | CH₂ | OC₃H₇-i |
| IIb-6 | (5) Cl | - | CH₂ | OC₄H₉-n |
| IIb-7 | (5) Cl | - | CH₂ | OCH(CH₃)C₅H₁₁-n |
| IIb-8 | (5) Cl | (2) F | CH₂ | OH |
| IIb-9 | (5) Cl | (2) Cl | CH₂ | OH |
| IIb-10 | (5) Cl | - | CH₂ | OCH₂CH=CH₂ |
| IIb-11 | (5) Cl | - | CH₂ | OC₄H₉-i |
| IIb-12 | (5) Cl | - | CH₂ | 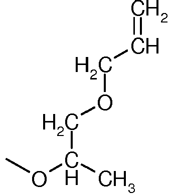 |
| IIb-13 | (5) Cl | - | 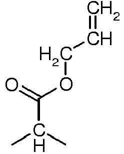 | OCH₂CH=CH₂ |
| IIb-14 | (5) Cl | - | 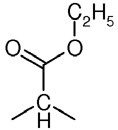 | OC₂H₅ |
| IIb-15 | (5) Cl | - | 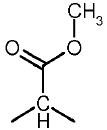 | OCH₃ |

Beispiele für die als erfindungsgemäße Herbizid-Safener ganz besonders bevorzugten Verbindungen der Formel (IIc) sind in der nachstehenden Tabelle aufgeführt.

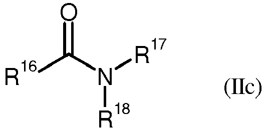

**Tabelle: Beispiele für die Verbindungen der Formel (IIc)**

| **Beispiel-Nr.** | **R¹⁶** | **N(R¹⁷R¹⁸)** |
|---|---|---|
| IIc-1 | CHCl₂ | N(CH₂CH=CH₂)₂ |
| IIc-2 | CHCl₂ | 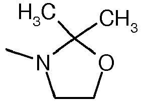 |
| IIc-3 | CHCl₂ | 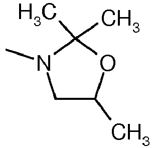 |
| IIc-4 | CHCl₂ | 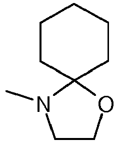 |
| IIc-5 | CHCl₂ | 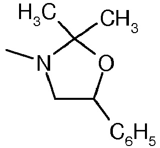 |
| IIc-6 | CHCl₂ | 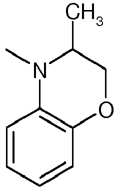 |
| IIc-7 | CHCl₂ | 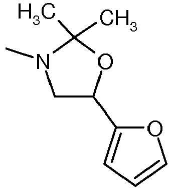 |

Beispiele für die als erfindungsgemäße Herbizid-Safener ganz besonders bevorzugten Verbindungen der Formel (IId) sind in der nachstehenden Tabelle aufgeführt.

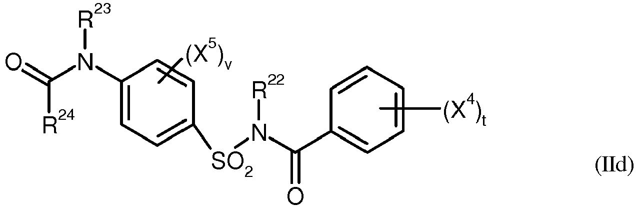

**Tabelle: Beispiele für die Verbindungen der Formel (IId)**

| **Beispiel-Nr.** | **R²²** | **R²³** | **R²⁴** | **(Positionen) (X⁴)ₜ** | **(Positionen) (X⁵)ᵥ** |
|---|---|---|---|---|---|
| IId-1 | H | H | CH₃ | (2) OCH₃ | - |
| IId-2 | H | H | C₂H₅ | (2) OCH₃ | - |
| IId-3 | H | H | C₃H₇-n | (2) OCH₃ | - |
| IId-4 | H | H | C₃H₇-i | (2) OCH₃ | - |
| IId-5 | H | H | 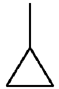 | (2) OCH₃ | - |
| IId-6 | H | H | CH₃ | (2) OCH₃ | - |
| | | | | (5) CH₃ | |
| IId-7 | H | H | C₂H₅ | (2) OCH₃ | - |
| | | | | (5) CH₃ | |
| IId-8 | H | H | C₃H₇-n | (2) OCH₃ | - |
| | | | | (5) CH₃ | |
| IId-9 | H | H | C₃H₇-i | (2) OCH₃ | - |
| | | | | (5) CH₃ | |
| IId-10 | H | H | 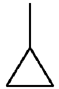 | (2) OCH₃ | - |
| | | | | (5) CH₃ | |
| IId-11 | H | H | OCH₃ | (2) OCH₃ | - |
| | | | | (5) CH₃ | |
| IId-12 | H | H | OC₂H₅ | (2) OCH₃ | - |
| | | | | (5) CH₃ | |
| IId-13 | H | H | OC₃H₇-i | (2) OCH₃ | - |
| | | | | (5) CH₃ | |
| IId-14 | H | H | SCH₃ | (2) OCH₃ | - |
| | | | | (5) CH₃ | |
| IId-15 | H | H | SC₂H₅ | (2) OCH₃ | - |
| | | | | (5) CH₃ | |
| IId-16 | H | H | SC₃H₇-i | (2) OCH₃ | - |
| | | | | (5) CH₃ | |
| IId-17 | H | H | NHCH₃ | (2) OCH₃ | - |
| | | | | (5) CH₃ | |
| IId-18 | H | H | NHC₂H₅ | (2) OCH₃ | - |
| | | | | (5) CH₃ | |
| IId-19 | H | H | NHC₃H₇-i | (2) OCH₃ | - |
| | | | | (5) CH₃ | |
| IId-20 | H | H | 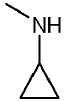 | (2) OCH₃ | - |
| | | | | (5) CH₃ | |
| IId-21 | H | H | NHCH₃ | (2) OCH₃ | - |
| IId-22 | H | H | NHC₃H₇-i | (2) OCH₃ | - |
| IId-23 | H | H | N(CH₃)₂ | (2) OCH₃ | - |
| IId-24 | H | H | N(CH₃)₂ | (3) CH₃ | - |
| | | | | (4) CH₃ | |
| IId-25 | H | H | CH₂-O-CH₃ | (2) OCH₃ | - |

Beispiele für die als erfindungsgemäße Herbizid-Safener ganz besonders bevorzugten Verbindungen der Formel (IIe) sind in der nachstehenden Tabelle aufgeführt.

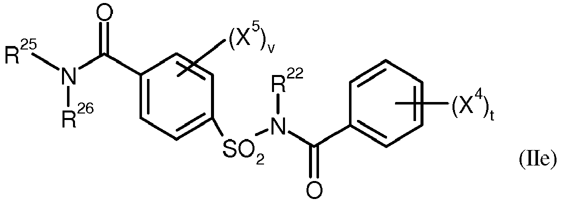

**Tabelle: Beispiele für die Verbindungen der Formel (IIe)**

| **Beispiel-Nr.** | **R²²** | **R²⁵** | **R²⁶** | **(Positionen) (X⁴)ₜ** | **(Positionen) (X⁵)ᵥ** |
|---|---|---|---|---|---|
| IIe-1 | H | H | CH₃ | (2) OCH₃ | - |
| IIe-2 | H | H | C₂H₅ | (2) OCH₃ | - |
| IIe-3 | H | H | C₃H₇-n | (2) OCH₃ | - |
| IIe-4 | H | H | C₃H₇-i | (2) OCH₃ | - |
| IIe-5 | H | H | 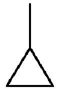 | (2) OCH₃ | - |
| IIe-6 | H | CH₃ | CH₃ | (2) OCH₃ | - |
| IIe-7 | H | H | CH₃ | (2) OCH₃ | - |
| | | | | (5) CH₃ | |
| IIe-8 | H | H | C₂H₅ | (2) OCH₃ | - |
| | | | | (5) CH₃ | |
| IIe-9 | H | H | C₃H₇-n | (2) OCH₃ | - |
| | | | | (5) CH₃ | |
| IIe-10 | H | H | C₃H₇-i | (2) OCH₃ | - |
| | | | | (5) CH₃ | |
| IIe-11 | H | H | 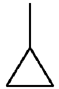 | (2) OCH₃ | - |
| | | | | (5) CH₃ | |
| IIe-12 | H | CH₃ | CH₃ | (2) OCH₃ | - |
| | | | | (5) CH₃ | |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyr-diethyl, Furilazole, Fenclorim, Cumyluron, Dymron, Dimepiperate und die Verbindungen IIe-5 und IIe-11 am meisten bevorzugt, wobei Cloquintocet-mexyl und Mefenpyr-diethyl, aber auch Isoxadifen-ethyl besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt und/oder können nach an sich bekannten Verfahren hergestellt werden (vgl. DE-A-19621522/US-A-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795/US-A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle aufgeführt.

**Tabelle: Beispiele für die erfindungsgemäßen Kombinationen**

| **Wirkstoffe der Formel (I)** | **Safener** |
|---|---|
| I-1-a | Cloquintocet-mexyl |
| I-1-a | Fenchlorazole-ethyl |
| I-1-a | Isoxadifen-ethyl |
| I-1-a | Mefenpyr-diethyl |
| I-1-a | Furilazole |
| I-1-a | Fenclorim |
| I-1-a | Cumyluron |
| I-1-a | Daimuron /Dymron |
| I-1-a | Dimepiperate |
| I-1-a | IIe-11 |
| I-1-a | IIe-5 |
| I-1-b | Cloquintocet-mexyl |
| I-1-b | Fenchlorazole-ethyl |
| I-1-b | Isoxadifen-ethyl |
| I-1-b | Mefenpyr -diethyl |
| I-1-b | Furilazole |
| I-1-b | Fenclorim |
| I-1-b | Cumyluron |
| I-1-b | Daimuron /Dymron |
| I-1-b | Dimepiperate |
| I-1-b | IIe-11 |
| I-1-b | IIe-5 |
| I-1-c | Cloquintocet -mexyl |
| I-1-c | Fenchlorazole-ethyl |
| I-1-c | Isoxadifen-ethyl |
| I-1-c | Mefenpyr-diethyl |
| I-1-c | Furilazole |
| I-1-c | Fenclorim |
| I-1-c | Cumyluron |
| I-1-c | Daimuron /Dymron |
| I-1-c | Dimepiperate |
| I-1-c | IIe-5 |
| I-1-c | IIe-11 |
| | |
| | |
| | |
| | |
| | |
| | |
| | |
| | |
| | |
| | |
| | |
| | |
| | |
| | |
| | |
| | |
| | |
| | |
| | |
| | |
| I-1-f | Cloquintocet -mexyl |
| I-1-f | Fenchlorazole-ethyl |
| I-1-f | Isoxadifen-ethyl |
| I-1-f | Mefenpyr -diethyl |
| I-1-f | Furilazole |
| I-1-f | Fenclorim |
| I-1-f | Cumyluron |
| I-1-f | Daimuron /Dymron |
| I-1-f | Dimepiperate |
| I-1-f | IIe-5 |
| I-1-f | IIe-11 |
| | |
| | |
| | |
| | |
| | |
| | |
| | |
| | |
| | |

Es wurde nun überraschend gefunden, dass die oben definierten Wirkstoffkombinationen aus biphenylsubstituierten spirocyclischen Ketoenole der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von biphenylsubstituierten spirocyclischen Ketoenolen auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat, Phosphinothricin und bestimmte Cyclische Ketoenole beschrieben (US 6 645 914, EP-A2 0 036 106, WO 07/068427). Eine entsprechende Wirkung bei Insektiziden ist für bestimmte Cyclische Ketoenole in der WO 07/068428 beschrieben..

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun völlig überraschend gefunden, dass sich die Wirkung von Insektiziden und/oder Akariziden und/oder Herbiziden aus der Klasse der biphenylsubstituierten, spirocyclischen Ketoenole der Formel (I) durch den Zusatz von Ammonium- oder Phosphoniumsalzen zur Anwendungslösung oder durch den Einbau dieser Salze in eine Formulierung enthaltend biphenylsubstituierte, spirocyclische Ketoenole, der Formel (I) deutlich steigern lässt. Gegenstand der vorliegenden Erfindung ist also die Verwendung von Ammonium- oder Phosphoniumsalzen zur Wirkungssteigerung von Pflanzenschutzmitteln, die insektizid und / oder akarizid wirksame biphenylsubstituierte, spirocyclische Ketoenole der Formel (I) als Wirkstoff enthalten. Gegenstand der Erfindung sind ebenfalls Mittel, die herbizide und/oder akarizid und/oder insektizid wirksame biphenylsubstituierte, spirocyclische Ketoenole der Formel (I) und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Die Verbindungen der Formel (I) besitzen eine breite insektizide und/oder akarizide und/oder herbizide Wirkung, die Wirkung und/oder Pflanzenverträglichkeit lässt im Einzelnen aber zu wünschen übrig. Diese Eigenschaften können jedoch durch den Zusatz von Ammonium- oder Phosphoniumsalzen insgesamt oder partiell verbessert werden.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend Wirkstoffe aus der Klasse der biphenylsubstituierten, spirocyclischen Ketoenole der Formel (1) steigern, werden durch Formel (III') definiert 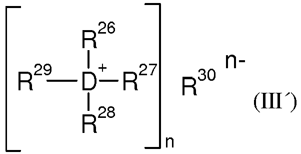 in welcher
D für Stickstoff oder Phosphor steht,
D bevorzugt für Stickstoff steht,
R^{26'}, R²⁷, R²⁸ und R²⁹ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
R^{26'}, R²⁷, R²⁸ und R²⁹ bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
R^{26'}, R²⁷, R²⁸ und R²⁹ besonders bevorzugt unabhängig voneinander für Wasserstoff, Methyl, Ethyl, n-Propyl, i-Propyl, n-Butyl, i-Butyl, s-Butyl oder t-Butyl stehen,
R^{26'}, R²⁷, R²⁸ und R²⁹ ganz besonders bevorzugt für Wasserstoff stehen,
n für 1, 2, 3 oder 4 steht,
n bevorzugt für 1 oder 2 steht,
R³⁰ für ein anorganisches oder organisches Anion steht,
R³⁰ bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat oder Oxalat steht,
R³⁰ besonders bevorzugt für Laktat, Sulfat, Nitrat, Thiosulfat, Thiocyanat, Oxalat oder Formiat steht.
R³⁰ ganz besonders bevorzugt für Sulfat steht.

Erfindungsgemäß hervorgehobene Kombinationen von Wirkstoff, Salz und Penetrationsförderer sind in folgender Tabelle aufgeführt. "Penetrationsförderer gemäß Test" bedeutet dabei, dass jede Verbindung geeignet ist, die in dem Test für die Kutikelpenetration (Baur et al., 1997, Pesticide Science 51, 131-152) als Penetrationsförderer wirkt.

Die Ammonium- und Phosphoniumsalze der Formel (III') können in einem breiten Konzentrationsbereich zur Steigerung der Wirkung von Pflanzenschutzmitteln enthaltend biphenylsubstituierte cyclische Ketoenole der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1-50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die insektizid wirksame, biphenylsubstituierte cyclische Ketoenole der Formel (I) als Wirkstoff enthalten. Gegenstand der Erfindung sind ebenfalls Mittel, die herbizid und/oder akarizid und/oder insektizid wirksame biphenylsubstituierte cyclische Ketoenole der Formel (I), Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel (IV')

R-O-(-AO)ᵥ-R' (IV')

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (IV'-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (IV'-b)

in welcher
- R: die oben angegebene Bedeutung hat,
- R': die oben angegebene Bedeutung hat,
- EO: für -CH₂-CH₂-O- steht,

- PO: für 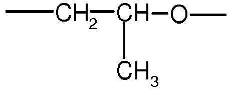 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (IV'-c)

in welcher
- R: die oben angegebene Bedeutung hat,
- R': die oben angegebene Bedeutung hat,
- EO: für -CH₂-CH₂-O- steht,
- PO: für 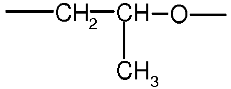 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (IV'-d)

in welcher
R und R' die oben angegebenen Bedeutungen haben,
- EO: für CH₂-CH₂-O- steht,

- BO: für 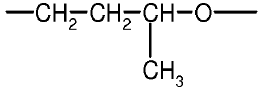 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (IV'-e)

in welcher
R und R' die oben angegebenen Bedeutungen haben,
- BO: für 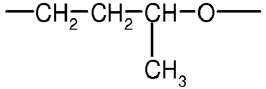 steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (IV'-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (IV-c) sei 2-Ethyl-hexyl-alkoxylat der Formel 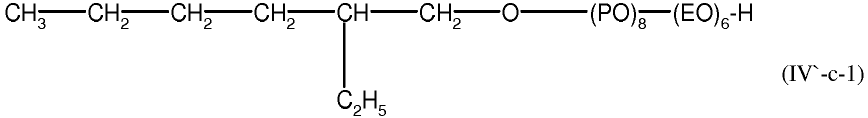 in welcher
- EO: für -CH₂-CH₂-O- steht,
- PO: für 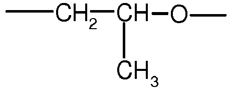 steht und
die Zahlen 8 und 6 Durchschnittswerte darstellen, genannt.

Als Beispiel für ein Alkanol-Alkoxylat der Formel (IV-d) sei die Formel

CH₃-(CH₂)₁₀-O-(-EO-)₆-(-BO-)₂-CH₃ (IV'-d-1)

in welcher
- EO: für CH₂-CH₂-O- steht,
- BO: für 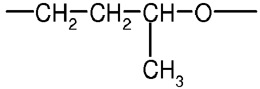 steht und
die Zahlen 10, 6 und 2 Durchschnittswerte darstellen, genannt.

Besonders bevorzugte Alkanol-Alkoxylate der Formel (IV'-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (IV'-f-1)

CH₃-(CH₂)ₜ-CH₂-O-(-CH₂-CH₂-O-)ᵤ-H (IV'-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäße Pflanzenschutzmittel können auch weitere Komponente, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Verwendet man gemäß Verfahren (A) N-[6-Methyl-3(4-fluor-phenyl)-phenylacetyl]-1-amino-cyclohexan-carbonsäureethylester als Ausgangsstoff, so kann der Verlauf des erfindungsgemäßen Verfahrens durch folgendes Reaktionsschema wiedergegeben werden:

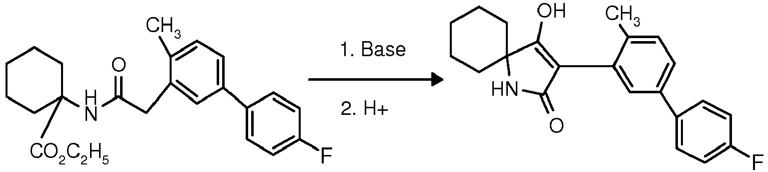

Verwendet man gemäß Verfahren (C) 3-[(2,6-Dimethyl-3-brom)-phenyl]-4,4-(pentamethylen)-pyrrolidin-2,4-dion und 4-Fluorphenylboronsäure als Ausgangsprodukte, so kann der Reaktionsverlauf durch folgendes Schema wiedergegeben werden:

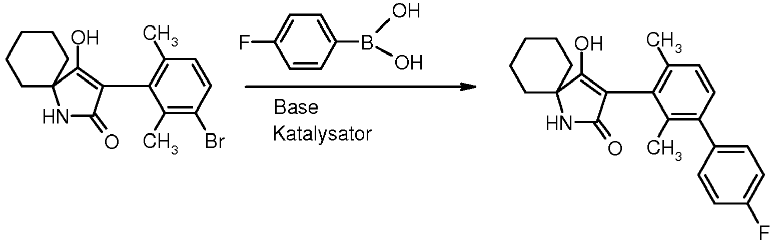

Verwendet man gemäß Verfahren (Da) 9-[(2-Chlor-5-(4-fluor-phenyl))-phenyl]- 4-oxa-7-aza-bicyclo[5.4.0]decan-8,10-dion und Pivaloylchlorid als Ausgangsstoffe, so kann der Verlauf des erfindungsgemäßen Verfahrens durch folgendes Reaktionsschema wiedergegeben werden:

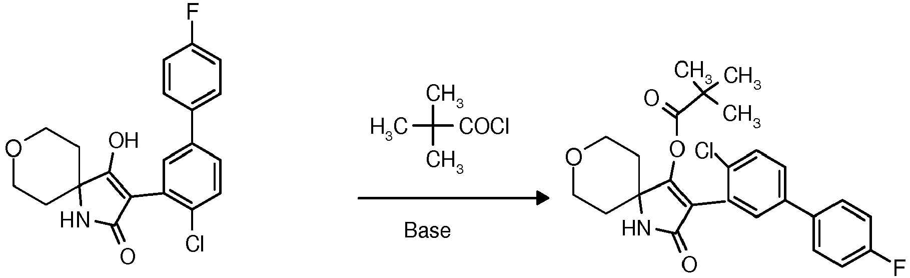

Verwendet man gemäß Verfahren (D) (Variante β) 3-[(6-Methyl-3-(4-fluor-phenyl))-phenyl]-4-hydroxy-5,5-pentamethylen-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

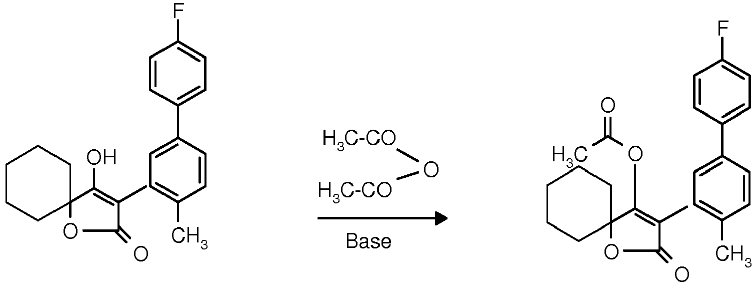

Verwendet man gemäß Verfahren (E) 9-[2,6-Dimethyl-3-(4-fluorphenyl)-phenyl]-4-oxa-7-aza-bicyclo-[5.4.0]-decan-8,10-dion und Chlorameisensäureethylester als Ausgangsverbindungen, so kann der Verlauf des erfindungsgemäßen Verfahrens durch folgendes Reaktionsschema wiedergegeben werden:

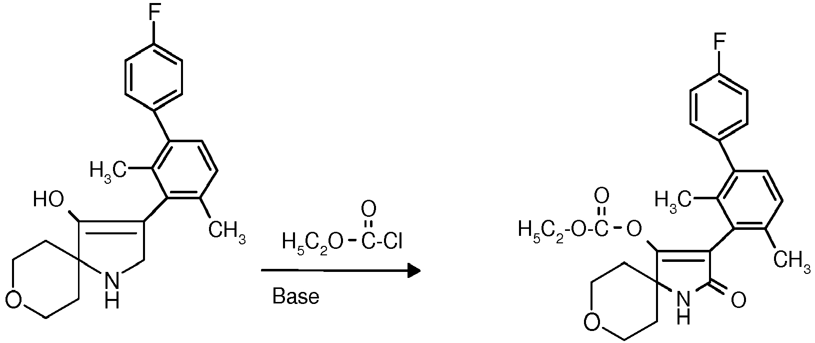

Verwendet man gemäß Verfahren (I) 9-[2-Methyl-5-(3,4-difluor-phenyl)-phenyl]-4-oxa-7-aza-bicyclo[5.4.0]-decan-8,10-dion und NaOH als Komponenten, so kann der Verlauf des erfindungsgemäßen Verfahrens durch folgendes Reaktionsschema wiedergegeben werden:

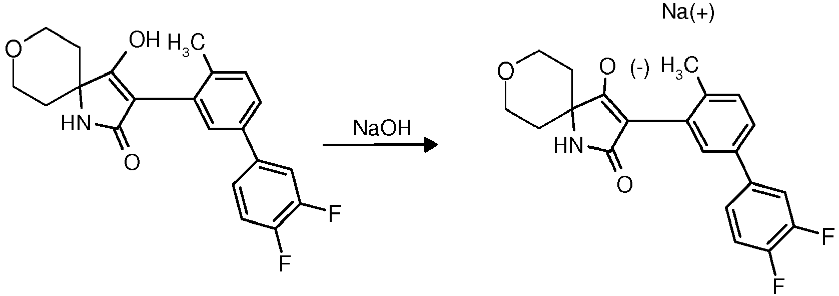

Die beim erfindungsgemäßen Verfahren (A) als Ausgangsstoffe benötigten Verbindungen der Formel (II) 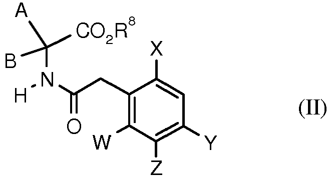 in welcher
- A, B, W, X, Y, Z und R⁸: die oben angegebenen Bedeutungen haben,
sind neu.

Man erhält die Acylaminosäureester der Formel (II) beispielsweise, wenn man Aminosäurederivate der Formel (XV) 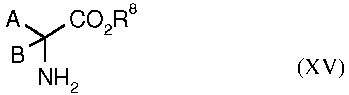 in welcher
- A, B und R⁸: die oben angegebenen Bedeutungen haben,
mit substituierten Phenylessigsäurederivaten der Formel (XVI) 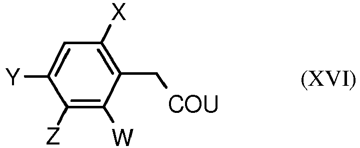 in welcher
W, X, Y und Z die oben angegebenen Bedeutungen haben und
U für eine durch Carbonsäureaktivierungsreagenzien wie Carbonyldiimidazol, Carbonyldiimide (wie z. B. Dicyclohexylcarbodiimid), Phosphorylierungs-reagenzien (wie z. B. POCl₃, BOP-Cl), Halogenierungsmittel, wie z. B. Thionylchlorid, Oxalylchlorid, Phosgen oder Chlorameisensäureester eingeführte Abgangsgruppe steht,
acyliert (Chem. Reviews 52, 237-416 (1953); Bhattacharya, Indian J. Chem. 6, 341-5, 1968)
oder wenn man Acylaminosäuren der Formel (XVII) 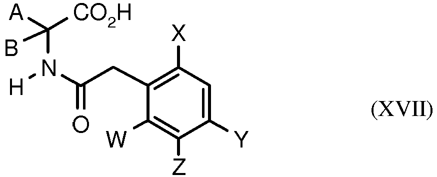 in welcher
- A, B, W, X, Y und Z: die oben angegebenen Bedeutungen haben,
verestert (Chem. Ind. (London) 1568 (1968)).

Die Verbindungen der Formel (XVII) 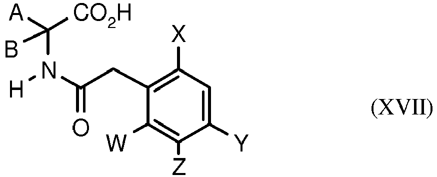 in welcher
- A, B, W, X, Y und Z: die oben angegebenen Bedeutungen haben,
sind neu.

Man erhält die Verbindungen der Formel (XVII), wenn man Aminosäuren der Formel (XVIII) 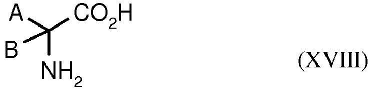 in welcher
- A und B: die oben angegebenen Bedeutungen haben,
mit substituierten Phenylessigsäurehalogeniden der Formel (XVI) 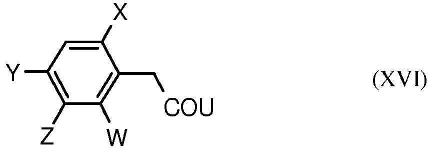 in welcher
- U, W, X, Y und Z: die oben angegebenen Bedeutungen haben
beispielsweise nach Schotten-Baumann acyliert (Organikum, VEB Deutscher Verlag der Wissenschaften, Berlin 1977, S. 505).

Die Verbindungen der Formel (XVI) sind neu. Sie lassen sich nach im Prinzip bekannten Verfahren darstellen (s. z.B. H. Henecka, Houben-Weyl, Methoden der Organischen Chemie, Bd. 8, S. 467-469 (1952) oder nach den eingangs zitierten Patentanmeldungen).

Man erhält die Verbindungen der Formel (XVI) beispielsweise, indem man substituierte Phenylessigsäuren der Formel (XIX) 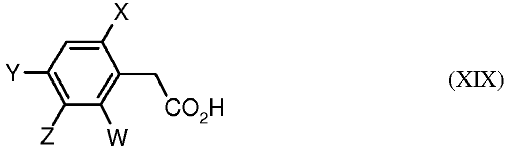 in welcher
W, X, Y und Z die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) oder Phosphorylierungs-reagenzien (z.B. POCl₃, BOP-Cl) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XV) und (XVIII) sind teilweise aus den eingangs zitierten Patentanmeldungen bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XVIIIa), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 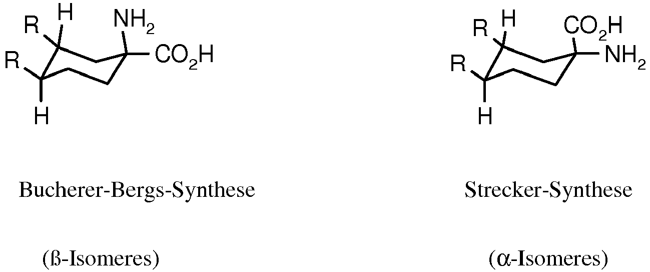 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 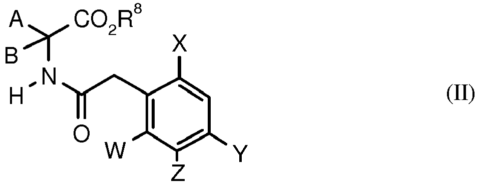 in welcher
- A, B, W, X, Y, Z und R⁸: die oben angegebenen Bedeutungen haben,
herstellen, indem man Aminonitrile der Formel (XX) 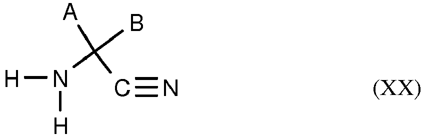 in welcher
- A und B: die oben angegebenen Bedeutungen haben,
mit substituierten Phenylessigsäurehalogeniden der Formel (XVI) 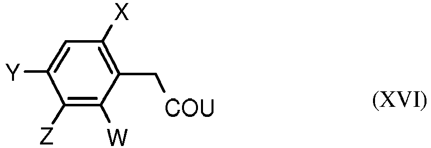 in welcher
W, X, Y und Z die oben angegebenen Bedeutungen haben,
zu Verbindungen der Formel (XXI) 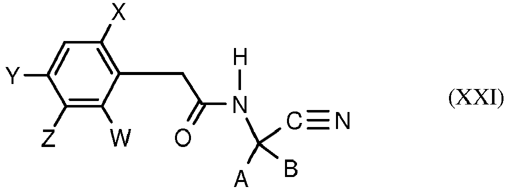 in welcher
- A, B, W, X, Y und Z: die oben angegebenen Bedeutungen haben,
umsetzt,
und diese anschließend einer sauren Alkoholyse unterwirft (EP-A-595130).

Die Verbindungen der Formel (XXI) sind ebenfalls neu.

Die Verbindungen der Formel (XIX) sind teilweise bekannt aus WO 2005/016873 oder lassen sich nach den dort beschriebenen Verfahren herstellen.

Beispielsweise erhält man die Verbindungen der Formel (XIX), 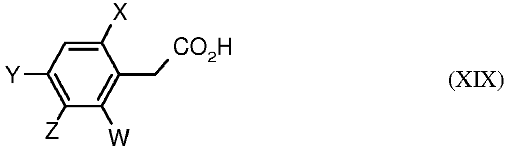 in welcher
W, X, Y und Z die oben angegebenen Bedeutungen haben,
α) wenn man Verbindungen der Formel (XIX-a) 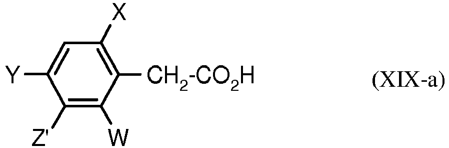 in welcher
   - X und Y: die oben angegebene Bedeutung haben,
   - Z': für Chlor, Brom oder Jod, bevorzugt für Brom steht,
   mit Boronsäuren oder Boronsäurederivaten der Formel (IV) 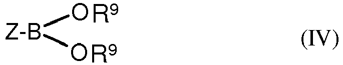 in welcher
   - Z und R⁹: die oben angegebene Bedeutung haben,
   in Gegenwart eines Lösungsmittels, einer Base und eines Katalysators (bevorzugt eines Palladiumsalzes oder Palladiumkomplexes, wie z.B. Palladium-tetrakis(triphenylphosphin)) umsetzt oder
β) wenn man Phenylessigsäureester der Formel (XXIII) 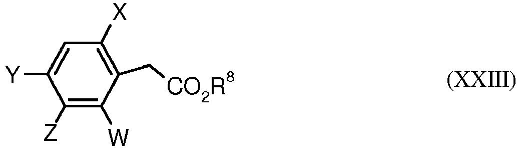 in welcher
   - W, X, Y, Z und R⁸: die oben angegebene Bedeutung haben,
   in Gegenwart von Säuren oder Basen, in Gegenwart eines Lösungsmittels unter allgemein bekannten Standardbedingungen verseift oder
γ) wenn man Phenylessigsäuren der Formel (XIX-b) 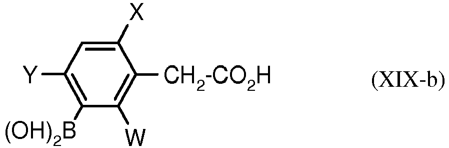 in welcher
   W, X und Z die oben angegebene Bedeutung haben,
   mit Halogenverbindungen der Formel (XXIV),

   Z-Hal (XXIV)

   in welcher
   - Z: die oben angegebene Bedeutung hat und
   - Hal: für Chlor, Brom oder Iod, bevorzugt für Brom und Iod steht,
in Gegenwart eines Lösungsmittels, einer Base und eines Katalysators (bevorzugt eines Palladiumsalzes oder einer der oben genannten Palladiumkomplexe) umsetzt.

Die Verbindungen der Formeln (IV) und (XXIV) sind teilweise bekannt, teilweise käuflich oder lassen sich nach im Prinzip bekannten Verfahren herstellen. Die Phenylessigsäuren der Formel (XIX-a) sind teilweise aus WO 97/01 535, WO 97/36 868 und WO 98/05 638 bekannt oder lassen sich nach den dort beschriebenen Verfahren herstellen. Die Verbindungen der Formel (XIX-b) sind teilweise aus WO 05/016873 bekannt oder lassen sich nach den dort beschriebenen Verfahren herstellen.

Die Verbindungen der Formel (XXIII) sind teilweise bekannt aus WO 2005/016873 oder lassen sich nach den dort beschriebenen Verfahren herstellen.

Die Verbindungen der Formel (XXIII) 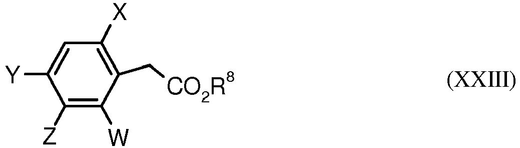 in welcher
- W, X, Y, Z und R⁸: die oben angegebene Bedeutung haben,
erhält man beispielsweise,
wenn man Phenylessigsäureester der Formel (XXIII-a) 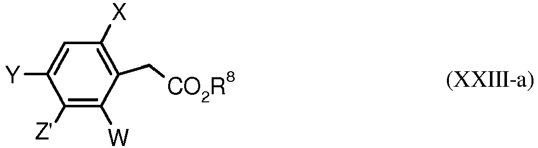 in welcher
- R⁸, W, X, Y und Z': die oben angegebene Bedeutung haben,
mit Boronsäuren oder Boronsäurederivaten der Formel (IV) 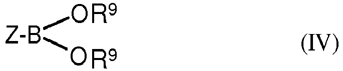 in welcher
- Z und R⁹: die oben angegebene Bedeutung haben,
in Gegenwart eines Lösungsmittels, einer Base und eines Katalysators (bevorzugt eines Palladiumsalzes oder einer der oben genannten Palladiumkomplexe) umsetzt.

Die Phenylessigsäureester der Formel (XXIII-a) sind teilweise aus den Anmeldungen WO 97/01535, WO 97/36868 und WO 98/0563 bekannt oder lassen sich nach den dort beschriebenen Verfahren herstellen.

Die bei dem obigen Verfahren (C) als Ausgangsstoffe benötigten Verbindungen der Formel (I-1'-a), (I-1'-b), (I-1'-c), (I-1'-f), in welchen A, B, W, X und Y die oben angegebene Bedeutung haben und Z' für Chlor, Brom oder Jod, bevorzugt für Brom steht, sind teilweise bekannt (WO 96/35 664, WO 97/02 243 und WO 98/05 638) oder lassen sich gemäß den dort beschriebenen Verfahren herstellen.

Die Boronsäuren und Boronsäurederivate der Formel (IV) 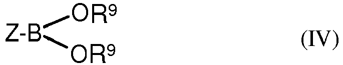 in welcher
- Z und R⁹: die oben angegebene Bedeutung hat,
sind teilweise käuflich oder lassen sich nach allgemein bekannten Verfahren in einfacher Weise herstellen.

Die zur Durchführung der erfindungsgemäßen Verfahren (D), (E), (I) außerdem als Ausgangsstoffe benötigten Säurehalogenide der Formel (V), Carbonsäureanhydride der Formel (VI), Chlorameisensäureester der Formel (VII), und Metallhydroxide, Metallalkoxide oder Amine der Formel (XI) und (XII) sind allgemein bekannte Verbindungen der Organischen bzw. Anorganischen Chemie.

Das Verfahren (A) ist dadurch gekennzeichnet, daß man Verbindungen der Formel (II), in welcher A, B, W, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natriumethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa doppeltäquimolaren Mengen ein. Es ist jedoch auch möglich, die eine oder andere Komponente in einem größeren Überschuß (bis zu 3 Mol) zu verwenden.

Zur Durchführung des erfindungsgemäßen Verfahrens (C) sind Palladium(0)-Komplexe als Katalysator geeignet. Bevorzugt wird beispielsweise Tetrakis-(triphenylphosphin)palladium. Gegebenenfalls können auch Palladium(II)-Salze eingesetzt werden, beispielsweise PdCl₂, Pd(NO₃)₂.

Als Säureakzeptoren zur Durchführung des erfindungsgemäßen Verfahrens (C) kommen anorganische oder organische Basen in Frage. Hierzu gehören vorzugsweise Erdalkalimetall- oder Alkalimetallhydroxide, -acetate, -carbonate oder -hydrogencarbonate, wie beispielsweise Natrium-, Kalium-, Barium- oder Ammoniumhydroxid, Natrium-, Kalium-, Calcium- oder Ammoniumacetat, Natrium-, Kalium- oder Ammoniumcarbonat, Natriumhydrogen- oder Kaliumhydrogencarbonat, Alkalifluoride, wie beispielsweise Cäsiumfluorid, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (C) kommen Wasser, organische Lösungsmittel und beliebige Mischungen davon in Betracht. Beispielhaft seien genannt: aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-amylether, Dioxan, Tetrahydrofuran, 1,2-Dimethoxyethan, 1,2-Diethoxyethan, Diethylenglykoldimethylether oder Anisol; Alkohole, wie Methanol, Ethanol, n- oder i-Propanol, n-, iso-, sek.- oder tert.-Butanol, Ethandiol, Propan-1,2-diol, Ethoxyethanol, Methoxyethanol, Diethylenglykolmonomethylether, Diethylenglykolmonomethylether; Wasser.

Die Reaktionstemperatur kann bei dem erfindungsgemäßen Verfahren (C) innerhalb eines größeren Bereiches variiert werden. Im allgemeinen arbeitet man bei Temperaturen zwischen 0°C und +140°C, bevorzugt zwischen 50°C und +100°C.

Bei der Durchführung des erfindungsgemäßen Verfahrens (C) werden die Boronsäure(n)-Derivate der Formel (IV), in welcher Z die oben angegebene Bedeutung hat und Verbindungen der Formel (I-1'-a), in welchen A, B, W, X, Y und Z' die oben angegebene Bedeutung haben, im molaren Verhältnis 1:1 bis 3:1, vorzugsweise 1:1 bis 2:1 eingesetzt. Vom Katalysator setzt man im allgemeinen 0,005 bis 0,5 Mol, vorzugsweise 0,01 Mol bis 0,1 Mol pro Mol der Verbindungen der Formeln (I-1-a) bis (I-8-a) ein. Die Base setzt man im allgemeinen in einem Überschuß ein.

Das Verfahren (D-α) ist dadurch gekennzeichnet, daß man Verbindungen der Formel (I-1-a)jeweils mit Carbonsäurehalogeniden der Formel (V) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (D-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (D-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (D-α) innerhalb eines größeren Bereiches variiert werden. Im allgemeinen arbeitet man bei Temperaturen zwischen - 20°C und +150°C, vorzugsweise zwischen 0°C und 100°C.

Bei der Durchführung des erfindungsgemäßen Verfahrens (D-α) werden die Ausgangsstoffe der Formel (I-1-a) und das Carbonsäurehalogenid der Formel (V) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (D-β) ist dadurch gekennzeichnet, daß man Verbindungen der Formel (I-1-a)mit Carbonsäureanhydriden der Formel (VI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (D-β) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (D-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (D-β) innerhalb eines größeren Bereiches variiert werden. Im allgemeinen arbeitet man bei Temperaturen zwischen - 20°C und +150°C, vorzugsweise zwischen 0°C und 100°C.

Bei der Durchführung des erfindungsgemäßen Verfahrens (D-β) werden die Ausgangsstoffe der Formel (I-1-a) und das Carbonsäureanhydrid der Formel (VI) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (E) ist dadurch gekennzeichnet, daß man Verbindungen der Formel (I-1-a)jeweils mit Chlorameisensäureestern der Formel (VII) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (E) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (E) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (E) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (E) wird im allgemeinen unter Normaldruck durchgeführt.

Bei der Durchführung des erfindungsgemäßen Verfahrens (E) werden die Ausgangsstoffe der Formel (I-1-a) und der entsprechende Chlorameisensäureester der Formel (VII) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das Verfahren (I) ist dadurch gekennzeichnet, daß man Verbindungen der Formel (I-1-a)mit Metallhydroxiden bzw. Metallalkoxiden der Formel (XI) oder Aminen der Formel (XII), gegebenenfalls in Gegenwart eines Verdünnungsmittels, umsetzt.

Als Verdünnungsmittel können bei dem erfindungsgemäßen Verfahren (I) vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (I) wird im allgemeinen unter Normaldruck durchgeführt.

Die Reaktionstemperaturen liegen im allgemeinen zwischen -20°C und 100°C, vorzugsweise zwischen 0°C und 50°C.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp.

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp.

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Reticulitermes spp., Odontotermes spp.

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im Allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Besonders günstige Mischpartner sind z.B. die folgenden:

### Fungizide:

Inhibitoren der Nucleinsäure Synthese
   Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure
Inhibitoren der Mitose und Zellteilung
   Benomyl, Carbendazim, Diethofencarb, Fuberidazole, Pencycuron, Thiabendazol, Thiophanat-methyl, Zoxamid
Inhibitoren der Atmungskette Komplex I
   Diflumetorim
Inhibitoren der Atmungskette Komplex II
   Boscalid, Carboxin, Fenfuram, Flutolanil, Furametpyr, Mepronil, Oxycarboxin, Penthiopyrad, Thifluzamid
Inhibitoren der Atmungskette Komplex III
   Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin, Trifloxystrobin
Entkoppler
   Dinocap, Fluazinam
Inhibitoren der ATP Produktion
   Fentinacetat, Fentinchlorid, Fentinhydroxid, Silthiofam
Inhibitoren der Aminosäure- und Proteinbiosynthese
   Andoprim, Blasticidin-S, Cyprodinil, Kasugamycin, Kasugamycinhydrochlorid Hydrat, Mepanipyrim, Pyrimethanil
Inhibitoren der Signal-Transduktion
   Fenpiclonil, Fludioxonil, Quinoxyfen
Inhibitoren der Fett- und Membran Synthese
   Chlozolinat, Iprodion, Procymidon, Vinclozolin
   Ampropylfos, Kalium-Ampropylfos, Edifenphos, Iprobenfos (IBP), Isoprothiolan, Pyrazophos
   Tolclofos-methyl, Biphenyl
   Iodocarb, Propamocarb, Propamocarb hydrochlorid
Inhibitoren der Ergosterol Biosynthese
   Fenhexamid,
   Azaconazol, Bitertanol, Bromuconazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadimefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefurazoat, Prochloraz, Triflumizol, Viniconazol,
   Aldimorph, Dodemorph, Dodemorphacetat, Fenpropimorph, Tridemorph, Fenpropidin, Spiroxamin,
   Naftifin, Pyributicarb, Terbinafin
Inhibitoren der Zellwand Synthese
   Benthiavalicarb, Bialaphos, Dimethomorph, Flumorph, Iprovalicarb, Polyoxins, Polyoxorim, Validamycin A
Inhibitoren der Melanin Biosynthese
   Capropamid, Diclocymet, Fenoxanil, Phtalid, Pyroquilon, Tricyclazol
Resistenzinduktion
   Acibenzolar-S-methyl, Probenazol, Tiadinil
Multisite
   Captafol, Captan, Chlorothalonil, Kupfersalze wie: Kupferhydroxid, Kupfernaphthenat, Kupferoxychlorid, Kupfersulfat, Kupferoxid, Oxin-Kupfer und Bordeaux Mischung, Dichlofluanid, Dithianon, Dodin, Dodin freie Base, Ferbam, Folpet, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
Unbekannter Mechanismus
   Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, N-(4-Chlor-2-nitrophenyl)-N-ethyl-4-methyl-benzenesulfonamid, 2-Amino-4-methyl-N-phenyl-5-thiazolecarboxamid, 2-Chlor-N-(2,3-dihydro-1,1,3-trimethyl-1H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)- benzacetat, 4-Chlor-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy] -3-methoxyphenyl]ethyl]-3-methyl-2-[(methylsulfonyl)amino]-butanamid, 5-Chlor-7-(4-methylpiperidin-1-yl)-6-(2,4,6-trifluorophenyl)-[1,2,4]triazolo[1,5-a]pyrimidin, 5-Chlor-6-(2,4,6-trifluorophenyl)-N-[(1R)-1,2,2-trimethyl-propyl][1,2,4]triazolo[1,5-a]pyrimidin-7-amin, 5-Chlor-N-[(1R)-1,2-dimethylpropyl]-6-(2,4,6-trifluorophenyl) [1,2,4]triazolo[1,5-a]pyrimidin-7-amine, N-[1-(5-Brom-3-chloro-pyridin-2-yl)ethyl]-2,4-dichloronicotinamid, N-(5-Brom-3-chlorpyridin-2-yl)methyl-2,4-dichlornicotinamid, 2-Butoxy-6-iod-3-propyl-benzopyranon-4-on, N-{(Z)-[(cyclopropyl-methoxy) imino][6-(difluormethoxy)-2,3-difluorphenyl]methyl}-2-benzacetamid, N-(3-Ethyl-3,5,5-trimethyl-cyclohexyl)-3-formylamino-2-hydroxy-benzamid, 2-[[[[1-[3(1Fluor-2-phenylethyl)oxy] phenyl] ethyliden]amino]oxy]methyl]-alpha-(methoxyimino)-N-methyl-alphaE-benzacetamid, N-{2-[3-Chlor-5-(trifluormethyl)pyridin-2-yl]ethyl}-2-(trifluoromethyl)-benzamid, N-(3',4'-dichlor-5-fluorbiphenyl-2-yl)-3-(difluormethyl)-1-methyl-1H-pyrazol-4-carboxamid, N-(6-Methoxy-3-pyridinyl)-cyclopropan carboxamid, 1-[(4-Methoxyphenoxy)-methyl]-2,2-dimethylpropyl-1H-imidazol-1-carbonsäure, O-[1-[(4-Methoxyphenoxy)-methyl]-2,2-dimethylpropyl]-1H-imidazol- 1- carbothioic acid, 2-(2-{[6-(3-Chlor-2-methyl-phenoxy)-5-fluorpyrimidin-4-yl]oxy}phenyl)-2-(methoxyimino)-N-methylacetamid

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

### Acetylcholinesterase (AChE) Inhibitoren

Carbamate,
   zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
Organophosphate,
   zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
Natrium-Kanal-Modulatoren / Spannungsabhängige Natrium-Kanal-Blocker
   Pyrethroide,
   zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda- Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
   DDT
   Oxadiazine,
   zum Beispiel Indoxacarb
   Semicarbazone,
   zum Beispiel Metaflumizone (BAS 3201)
Acetylcholin-Rezeptor-Agonisten/-Antagonisten
   Chloronicotinyle,
   zum Beispiel Acetamiprid, Clothianidin, Dinotefuran, Imidacloprid, Nitenpyram, Nithiazine, Thiacloprid, Thiamethoxam
   Nicotine, Bensultap, Cartap
Acetylcholin-Rezeptor-Modulatoren
   Spinosyne,
   zum Beispiel Spinosad
GABA-gesteuerte Chlorid-Kanal-Antagonisten
   Organochlorine,
   zum Beispiel Camphechlor, Chlordane, Endosulfan, Gamma-HCH, HCH, Heptachlor, Lindane, Methoxychlor
   Fiprole,
   zum Beispiel Acetoprole, Ethiprole, Fipronil, Pyrafluprole, Pyriprole, Vaniliprole
Chlorid-Kanal-Aktivatoren
   Mectine,
   zum Beispiel Avermectin, Emamectin, Emamectin-benzoate, Ivermectin, Milbemycin
Juvenilhormon-Mimetika,
   zum Beispiel Diofenolan, Epofenonane, Fenoxycarb, Hydroprene, Kinoprene, Methoprene, Pyriproxifen, Triprene
Ecdysonagonisten/disruptoren
   Diacylhydrazine,
   zum Beispiel Chromafenozide, Halofenozide, Methoxyfenozide, Tebufenozide
Inhibitoren der Chitinbiosynthese
   Benzoylharnstoffe,
   zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
   Buprofezin
   Cyromazine
Inhibitoren der oxidativen Phosphorylierung, ATP-Disruptoren
   Diafenthiuron
   Organozinnverbindungen,
   zum Beispiel Azocyclotin, Cyhexatin, Fenbutatin-oxide
Entkoppler der oxidativen Phoshorylierung durch Unterbrechung des H-Protongradienten
   Pyrrole,
   zum Beispiel Chlorfenapyr
   Dinitrophenole,
   zum Beispiel Binapacyrl, Dinobuton, Dinocap, DNOC
Seite-I-Elektronentransportinhibitoren
   METI's,
   zum Beispiel Fenazaquin, Fenpyroximate, Pyrimidifen, Pyridaben, Tebufenpyrad, Tolfenpyrad
   Hydramethylnon
   Dicofol
Seite-II-Elektronentransportinhibitoren
   Rotenone
Seite-III-Elektronentransportinhibitoren
   Acequinocyl, Fluacrypyrim
Mikrobielle Disruptoren der Insektendarmmembran
   Bacillus thuringiensis-Stämme
Inhibitoren der Fettsynthese
   Tetronsäuren,
   zum Beispiel Spirodiclofen, Spiromesifen
   Tetramsäuren,
   zum Beispiel Spirotetramat
Carboxamide,
   zum Beispiel Flonicamid
Oktopaminerge Agonisten,
   zum Beispiel Amitraz
Inhibitoren der Magnesium-stimulierten ATPase,
   Propargite
Ryanodinrezeptor-Effektoren
   a) Benzoesäuredicarboxamide,
      zum Beispiel Flubendiamide
   b) Anthranilamide, z.B.
      Rynaxapyr (3-bromo-N-{4-chloro-2-methyl-6-[(methylamino)carbonyl]phenyl}-1-(3-chloropyridin-2-yl)-1H-pyrazole-5-carboxamide)
Nereistoxin-Analoge,
   zum Beispiel Thiocyclam hydrogen oxalate, Thiosultap-sodium
Biologika, Hormone oder Pheromone
   Azadirachtin, Bacillus spec., Beauveria spec., Codlemone, Metarrhizium spec., Paecilomyces spec., Thuringiensin, Verticillium spec.
Wirkstoffe mit unbekannten oder nicht spezifischen Wirkmechanismen
   Begasungsmittel,
   zum Beispiel Aluminium phosphide, Methyl bromide, Sulfuryl fluoride Fraßhemmer,
   zum Beispiel Cryolite, Flonicamid, Pymetrozine
   Milbenwachstumsinhibitoren,
   zum Beispiel Clofentezine, Etoxazole, Hexythiazox
   Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp.

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.

Aus der Ordnung der Siphonapterida z.B. Pulex spp., Ctenocephalides spp., Xenopsylla spp., Ceratophyllus spp.

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp.

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp.

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp.

Aus der Ordnung der Chilopoda z.B. Geophilus spp.

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propeller Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe/Wirkstoffkombinationen können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe/Wirkstoffkombinationen können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe/Wirkstoffkombinationen ist jedoch keineswegs auf diese Gattungen beschränkt, sondern erstreckt sich in gleicher Weise auch auf andere Pflanzen.

Die erfindungsgemäßen Wirkstoffe/Wirkstoffkombinationen eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I)/Wirkstoffkombinationen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe/Wirkstoffkombinationen können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe/Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe/Wirkstoffkombinationen können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise

Acetochlor, Acifluorfen (-sodium), Aclonifen, Alachlor, Alloxydim (-sodium), Ametryne, Amicarbazone, Amidochlor, Amidosulfuron, Aminopyralid, Anilofos, Asulam, Atrazine, Azafenidin, Azimsulfuron, Beflubutamid, Benazolin (-ethyl), Benfuresate, Bensulfuron (-methyl), Bentazon, Benzcarbazone, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, HOK-201, Imazamethabenz-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, KIH 485, Lactofen, Lenacil, Linuron, MCPA, Mecoprop, Mefenacet, Mesosulfurone, Mesotrione, Metamifop, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Orthosulfamuron, Oryzalin, Oxadiargyl, Oxadiazon, Oxasulfuron, Oxaziclomefone, Oxyfluorfen, Paraquat, Pelargonsäure, Pendimethalin, Pendralin, Penoxsulam, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrasulfotole, Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrimisulfan, Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tembotrione, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Topramezone, Tralkoxydim, Triallate, Triasulfuron, Tribenuron (-methyl), Triclopyr, Tridiphane, Trifluralin, Trifloxysulfuron, Triflusulfuron (-methyl), Tritosulfuron und

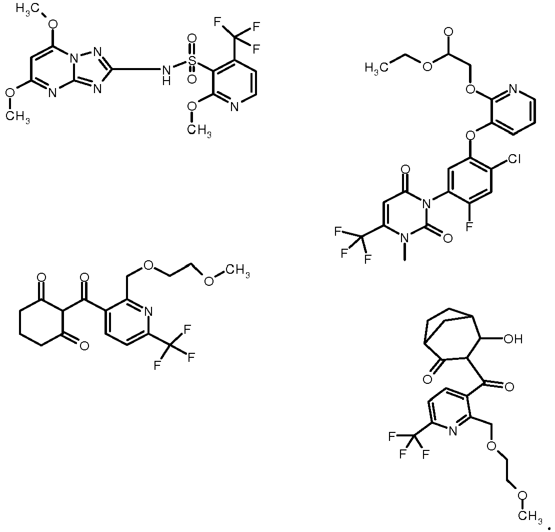

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe bzw. Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe bzw. Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im Allgemeinen entfallen auf 1 Gewichtsteil Wirkstoff der Formel (I) Salzen 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im Allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im Allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstoffkombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Der Begriff Wirkstoff umfasst die genanntenWirkstoffkombinationen ebenso.

Die Herstellung und die Verwendung der erfindungsgemäßen Wirkstoffe geht aus den nachfolgenden Beispielen hervor.

### Herstellungsbeispiele

### Beispiel I-1-a-1

### Verfahren A

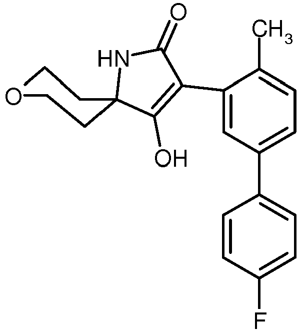

Zu 1,83 g (15,5 mmol) Kalium-tert-butylat in 6 ml N,N-Dimethylacetamid werden bei 80°C 2,4 g der Verbindung gemäß Bsp. II-1 in 6 ml N,N-Dimethylacetamid zugetropft und eine Stunde bei 80°C gerührt.

Nach dünnschichtchromatographischer Probe wird das Reaktionsgemisch auf 80 ml Eiswasser gegeben und bei 0-10°C mit IN Salzsäure auf pH 2 gebracht. Der Niederschlag wird abgesaugt, nachgewaschen und getrocknet.
Roh-Ausbeute: 2,35 g beiges Pulver
Nach präparativer HPLC-Trennung erhält man das Produkt in einer
Ausbeute: 173 mg (7,7% d. Theorie) Fp. 94°C

### Beispiel I-1-a-2

### Verfahren A

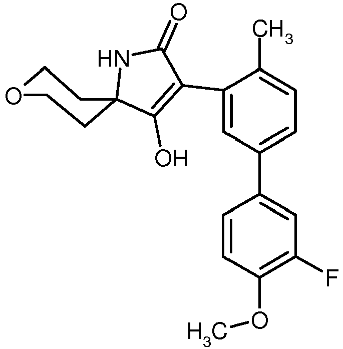

Zu 1,3 g (10,5 mmol) Kalium-tert-butylat in 4 ml N,N-Dimethylacetamid werden bei 60°C 1,95 g (4,7 mmol) der Verbindung gemäß Bsp. II-2 in 4,0 ml N,N-Dimethylacetamid zugetropft und eine Stunde bei 60°C gerührt.

Nach dünnschichtchromatographischer Probe auf 80 ml Eiswasser geben und bei 0-10°C mit IN Salzsäure auf pH 2 bringen, absaugen, nachwaschen und trocknen. Die Aufreinigung erfolgte durch Säulenchromatographie an Kieselgel mit Essigsäure-ethylester als Laufmittel.
Ausbeute: 0,92 g (48,8% d. Theorie), Fp. 177°C

### Beispiel I-1-a-24

### Verfahren C

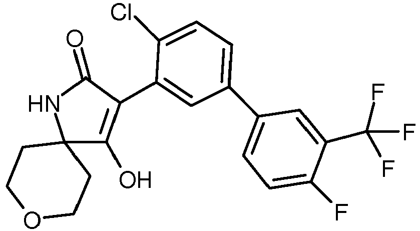

0,717 g der Verbindung gemäß Bsp. I-1'-a-1, 0,448g 4-Fluor-3-trifluormethylphenylboronsäure und 0,8 g Natriumcarbonat werden in 15 ml Wasser vorgelegt, 0.05 g Palladium (II) nitrat-dihydrat zugegeben und 20 min. bei 144°C in der Mikrowelle gerührt. Nach Abkühlen wird mit verd. Salzsäure angesäuert und abgesaugt.

Es erfolgt eine MPLC-Trennung an Kieselgel mit Cyclohexan + 50-80% Essigsäureethylester als Elutionsgradienten
Ausbeute: 0,31 g (34 % d. Theorie), Fp. 257°C

In Analogie zu den Beispielen (I-1-a-1) und (I-1-a-2) und gemäß den allgemeinen Angaben zur Herstellung erhält man folgende Verbindungen der Formel (I-1-a):

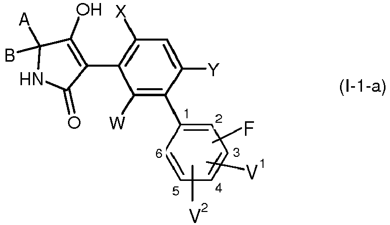

| **Bsp.-Nr.** | **W** | **X** | **Y** | **F** | **V¹** | **V²** | A | **B** | **Fp°C** | **Isomer** |
|---|---|---|---|---|---|---|---|---|---|---|
| I-1-a-3 | H | CH₃ | H | 4 | 3-F | H | -(CH₂)₂-O-(CH₂)₂- | | 241 | - |
| I-1-a-4 | CH₃ | CH₃ | H | 4 | H | H | -(CH₂)₂-O-(CH₂)₂- | | 167 | - |
| I-1-a-5 | CH₃ | CH₃ | H | 4 | 3-F | H | -(CH₂)₂-O-(CH₂)₂- | | 164 | - |
| I-1-a-6 | H | CH₃ | H | 3 | 4-Cl | H | -(CH₂)₂-O-(CH₂)₂- | | 258 | - |
| I-1-a-7 | H | CH₃ | H | 4 | H | H | -CH₂-CHOC₂H₅-(CH₂)₃- | | 102 | β |
| I-1-a-8 | H | CH₃ | H | 4 | H | H | 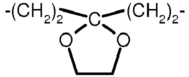 | | 290 | - |
| I-1-a-9 | CH₃ | CH₃ | H | 4 | H | H | 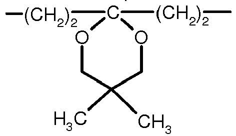 | | 299 | - |
| I-1-a-10 | CH₃ | CH₃ | H | 4 | H | H | -CH₂-O-(CH₂)₃- | | 284 | - |
| I-1-a-11 | H | CH₃ | H | 4 | H | H | -CH₂-CHCH₃-O-(CH₂)₂- | | *1.11-1.13 (2d, 3H, CHCH₃ 2.19 (d, 3H, Ar-CH₃ | β |
| | | | | | | | | | 3.72-4.06 (3m, 3H, O-CH₂, O-CH-CH₃) | |
| | | | | | | | | | 7.61-7.66 (m, 2H, Ar-H) | |
| I-1-a-12 | CH₃ | CH₃ | H | 4 | H | H | -CH₂-CHCH₃-O-(CH₂)₂- | | 168 | β |
| I-1-a-13 | H | Cl | H | 4 | H | H | -(CH₂)₂-O-(CH₂)₂- | | 279 | - |
| I-1-a-14 | H | CH₃ | H | 4 | 3-CH₃ | H | -(CH₂)₂-O-(CH₂)₂- | | 160 | - |
| I-1-a-15 | CH₃ | CH₃ | H | 4 | 3-CH₃ | H | -(CH₂)₂-O-(CH₂)₂- | | 163 | - |
| I-1-a-16 | H | CH₃ | H | 4 | 3-Cl | H | -(CH₂)₂-O-(CH₂)₂- | | 200 | - |
| I-1-a-17 | H | Cl | H | 4 | 3-Cl | H | -(CH₂)₂-O-(CH₂)₂- | | 160 | - |
| I-1-a-18 | H | CH₃ | H | 4 | 3-OCH₃ | H | -(CH₂)₂-O-(CH₂)₂- | | 141 | - |
| I-1-a-19 | H | Cl | H | 4 | 3-CH₃ | H | -(CH₂)₂-O-(CH₂)₂- | | 254 | - |
| I-1-a-20 | CH₃ | CH₃ | H | 4 | 3-Cl | H | -(CH₂)₂-O-(CH₂)₂- | | 298 | - |
| I-1-a-21 | H | Cl | H | 4 | 3-F | H | -(CH₂)₂-O-(CH₂)₂- | | 273 | - |
| I-1-a-22 | H | CH₃ | H | 4 | 3-CF₃ | H | -(CH₂)₂-O-(CH₂)₂- | | 210 | - |
| I-1-a-23 | CH₃ | CH₃ | H | 4 | 3-CF₃ | H | -(CH₂)₂-O-(CH₂)₂- | | 262 | - |
| I-1-a-24 | H | Cl | H | 4 | 3-CF₃ | H | -(CH₂)₂-O-(CH₂)₂- | | 257 | - |
| I-1-a-25 | CH₃ | CH₃ | H | 4 | 3-F | 5-F | -(CH₂)₂-O-(CH₂)₂- | | 267 | - |
| I-1-a-26 | CH₃ | CH₃ | H | 4 | 2-CH₃ | H | -(CH₂)₂-O-(CH₂)₂- | | * 1.25-1.34(m,2H,CH₂);2.14 (s,3H,Ar-CH₃);3.67-3.75 (m,2H-O-CH₂);6.88-6.9 (m,1H-Ar-H) | - |
| I-1-a-27 | H | Cl | H | 4 | 2-CH₃ | H | -(CH₂)₂-O-(CH₂)₂- | | * 1.25-1.32(m,2H, OCH₂); 2.28(s, 3H,Ar-CH₃); 3.88-3.87(m,2H,O-CH₂); 7.48(d,1H,Ar-H) | - |
| I-1-a-28 | H | CH₃ | H | 4 | 3-F | 5-F | -(CH₂)₂-O-(CH₂)₂- | | * 2.20 (s,3H,Ar-CH₃); 3.68-3.75 (m,2H,O-CH₂); 3.85-3.89 (m,2H,O-CH₂); 7.29-7.31 (d,1H,Ar-H) | - |
| I-1-a-29 | H | CH₃ | H | 4 | 2-F | H | -(CH₂)₂-O-(CH₂)₂- | | * 1.25-1.32 (m,2H,CH₂); 2.21 (s,3H,Ar-CH₃); 3.84-3.88 (m,2H-OCH₂); 7.49-7.55 (m,1H,Ar-H) | - |
| I-1-a-30 | H | Cl | H | 4 | 3-F | 5-F | -(CH₂)₂-O-(CH₂)₂- | | 300 | - |
| I-1-a-31 | H | CH₃ | H | 4 | 2-CH₃ | H | -(CH₂)₂-O-(CH₂)₂- | | * 1.25-1.31 (m,2H,CH₂); 2.21,2.26 (2s,je 3H,Ar-CH₃);3.84-3.87 (m,2H,O-CH₂); 7.00-7.05 (m,2H,Ar-H) | - |
| I-1-a-32 | H | Cl | H | 4 | 3-OCH₃ | H | -(CH₂)₂-O-(CH₂)₂- | | 226 | - |
| I-1-a-33 | CH₃ | CH₃ | CH₃ | 4 | H | H | -(CH₂)₂-O-(CH₂)₂- | | 298 | - |
| I-1-a-34 | CH₃ | CH₃ | H | 4 | 2-F | H | -(CH₂)₂-O-(CH₂)₂- | | 215 | - |
| I-1-a-35 | H | Cl | H | 4 | 2-F | H | -(CH₂)₂-O-(CH₂)₂- | | * 1.25-1.33 (m,2H,CH₂);3.66-3.73 (m,2H,O-CH₂); 3.84-3.87 (m,2H,OCH₂); 7.40-7.41 (m,1H,Ar-H) | - |
| I-1-a-36 | H | CH₃ | H | 3 | H | H | -(CH₂)₂-O-(CH₂)₂- | | 138 | - |
| I-1-a-37 | CH₃ | CH₃ | H | 3 | H | H | -(CH₂)₂-O-(CH₂)₂- | | 147 | - |
| I-1-a-38 | H | CH₃ | H | 3 | 5-F | H | -(CH₂)₂-O-(CH₂)₂- | | 230 | - |
| I-1-a-39 | CH₃ | CH₃ | H | 3 | 5-F | H | -(CH₂)₂-O-(CH₂)₂- | | 163 | - |
| I-1-a-40 | H | Cl | H | 2 | 5-F | H | -(CH₂)₂-O-(CH₂)₂- | | | - |
| I-1-a-41 | H | Cl | H | 3 | 5-F | H | -(CH₂)₂-O-(CH₂)₂- | | 276 | |
| I-1-a-42 | H | CH₃ | H | 2 | 5-F | H | -(CH₂)₂-O-(CH₂)₂- | | * 1.25-1.33 (m,2H,-CH₂)-; 2.21 (s,3H,Ar-CH₃); 3.68-3.74 | - |
| | | | | | | | | | (m,2H,OCH₂); 7.15-7.21 (m,1H, Ar-H) | |
| I-1-a-43 | H | CH₃ | H | 3 | 4-CH₃ | H | -(CH₂)₂-O-(CH₂)₂- | | 156 | - |
| I-1-a-44 | CH₃ | CH₃ | H | 3 | 4-CH₃ | H | -(CH₂)₂-O-(CH₂)₂- | | 191 | - |
| I-1-a-45 | H | Cl | H | 3 | 4-CH₃ | H | -(CH₂)₂-O-(CH₂)₂- | | 215 | - |
| I-1-a-46 | H | CH₃ | H | 4 | H | H | -CH₂-O-(CH₂)₃- | | 259 | - |
| I-1-a-47 | H | Cl | H | 4 | H | H | -CH₂-O-(CH₂)₃- | | 272 | - |
| I-1-a-48 | CH₃ | CH₃ | H | 4 | H | H | -CH₂-CHOCH₃-(CH₂)₃- | | 263 | cis |
| I-1-a-49 | H | CH₃ | H | 4 | H | H | -CH₂-CHOCH₃-(CH₂)₃- | | 248 | cis |
| I-1-a-50 | H | CH₃ | H | 4 | H | H | -CH₂-CHOCH₃-(CH₂)₃- | | 192 | trans |
| I-1-a-51 | CH₃ | CH₃ | H | 4 | H | H | -CH₂-CHOC₂H₅-(CH₂)₃- | | 162 | cis/trans |
| I-1-a-52 | H | CH₃ | H | 4 | H | H | -(CH₂)₅- | | 243 | - |
| I-1-a-53 | CH₃ | CH₃ | H | 4 | H | H | -(CH₂)₅- | | 286 | - |
| I-1-a-54 | CH₃ | CH₃ | H | 4 | H | H | -CH₂-CHOCH₃-(CH₂)₃- | | 264 | trans |
| I-1-a-55 | CH₃ | CH₃ | H | 4 | H | H | -CH₂-CHOCH₃-(CH₂)₂- | | 230 | cis/trans ca. 1:1 |
| I-1-a-56 | H | CH₃ | H | 4 | H | H | -CH₂-CHOCH₃-(CH₂)₂- | | * 2.19 (s,3H,Ar-CH₃); 3.24, 3.25 (2s, zusammen 3H, OCH₃); 7.42.7.45 (m,1H,Ar-H) | cis/trans ca. 1:1 |
| I-1-a-57 | CH₃ | CH₃ | H | 4 | H | H | 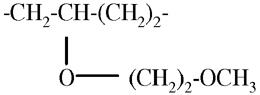 | | 225-227 | cis |
| I-1-a-58 | CH₃ | CH₃ | H | 4 | H | H | 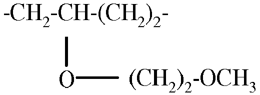 | | ** 2.25 (Pseudo-d, 3H, Ar CH₃); 4.22 (m, 1H, CH O-(CH₂)₂-O) | trans |
| I-1-a-59 | H | CH₃ | H | 4 | 3-F | H | -CH₂-O-(CH₂)₃- | | 246 | - |
| I-1-a-60 | CH₃ | CH₃ | H | 4 | 3-F | H | -CH₂-O-(CH₂)₃- | | 273 | - |
| I-1-a-61 | H | Cl | H | 4 | 3-Cl | H | -CH₂-O-(CH₂)₃- | | 269 | - |
| I-1-a-62 | H | CH₃ | H | 4 | 3-F | H | 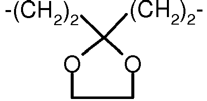 | | 269 | - |
| I-1-a-63 | H | CH₃ | H | 4 | 3-Cl | H | 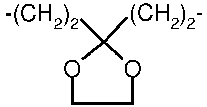 | | 250 | - |
| I-1-a-64 | H | CH₃ | H | 4 | 2-F | 3-F | -(CH₂)₂-O-(CH₂)₂- | | *2.22 (s, 3H, ArCH₃) 3.84-3.88 (m, 2H, O-CH₂) | |
| | | | | | | | | | 7.26-7.44 (m, 6H, ArH) | |
| I-1-a-65 | CH₃ | CH₃ | H | 4 | 2-F | 3-F | -(CH₂)₂-O-(CH₂)₂- | | | |
| I-1-a-66 | CH₃ | CH₃ | H | 4 | 2-F | 5-F | -(CH₂)₂-O-(CH₂)₂- | | *2.14 (s, 3H, ArCH₃) | |
| | | | | | | | | | 7.05 (d, 1H, ArH) | |
| | | | | | | | | | 7.16 (d, 1H, ArH) | |
| I-1-a-67 | H | Cl | H | 4 | 2-F | 5-F | -(CH₂)₂-O-(CH₂)₂- | | | |
| I-1-a-68 | H | CH₃ | H | 4 | 2-F | 3-F | -(CH₂)₂-O-(CH₂)₂- | | *2.22 (s, 3H, ArCH₃) | |
| | | | | | | | | | 3.68-3.76 (m, 2H, O-CH₂) | |
| | | | | | | | | | 7.29-7.37 (m, 4H, ArH) | |
| I-1-a-69 | CH₃ | CH₃ | H | 4 | 2-F | 3-F | -(CH₂)₂-O-(CH₂)₂- | | *2.16 (2s, je 3H, ArCH₃) | |
| | | | | | | | | | 3.65-3.72 (m, 2H, O-CH₂) | |
| | | | | | | | | | 7.07-7.11 (m, 2H, ArH) | |
| I-1-a-70 | H | Cl | H | 4 | 2-F | 3-F | -(CH₂)₂-O-(CH₂)₂- | | | |

| | | | | | | | | | | |
|---|---|---|---|---|---|---|---|---|---|---|
| * ¹H-NMR (400 MHz, d₆-DMSO): Verschiebung δ in ppm | | | | | | | | | | |

### Beispiel (I-1-b-1)

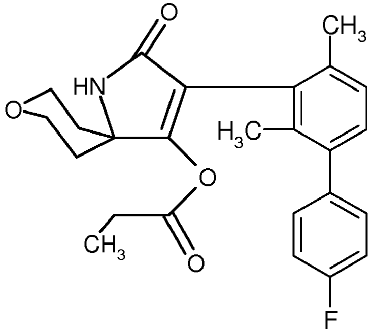

Zu 0,78 g (2 mmol) der Verbindung gemäß Beispiel (I-1-a-4) in 50 ml Essigsäureethylester gibt man 0,28 ml (2 mmol) Triethylamin und 20 mg 4-N,N-Dimethylamino-pyridin. Unter Rückfluss werden 0.19 g (2 mmol) Propionsäurechlorid in 5 ml Essigester zugetropft. Nach beendeter Reaktion (Dünnschichtkontrolle) wird das Reaktionsgemisch im Vakuum eingedampft und der Rückstand durch Reversed Phase Chromatographie im Laufmittelsystem Wasser/Acetonitril gereinigt. Man erhält 0,5 g (=̂58 % d. Theorie), Fp. 234°C.

In Analogie zu Beispiel (I-1-b-1) und gemäß den allgemeinen Angaben zur Herstellung erhält man folgende Verbindungen der Formel (I-1-b):

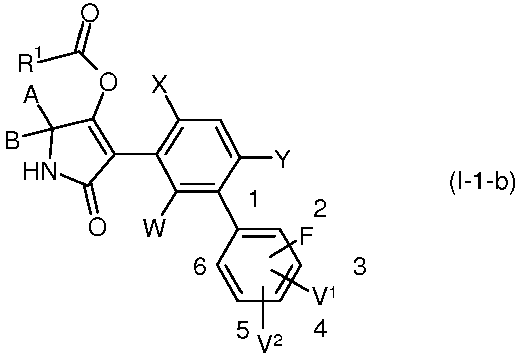

| **Bsp.-Nr.** | **W** | **X** | **Y** | **F** | **V¹** | **V²** | **A** | **B** | **R¹** | **Fp°C** | **Isomer** |
|---|---|---|---|---|---|---|---|---|---|---|---|
| I-1-b-2 | CH₃ | CH₃ | H | 4 | H | H | -(CH₂)₂-O-(CH₂)₂- | | CH₃ | 160 | - |
| I-1-b-3 | CH₃ | CH₃ | H | 4 | H | H | -(CH₂)₂-O-(CH₂)₂- | | i-C₃H₇ | 219 | - |
| I-1-b-4 | H | Cl | H | 4 | H | H | -(CH₂)₂-O-(CH₂)₂- | | i-C₃H₇ | 226 | - |
| I-1-b-5 | H | CH₃ | H | 4 | H | H | -(CH₂)₂-O-(CH₂)₂- | | CH₃ | 249 | - |
| I-1-b-6 | H | CH₃ | H | 4 | H | H | -(CH₂)₂-O-(CH₂)₂- | | i-C₃H₇ | 249 | - |
| I-1-b-7 | CH₃ | CH₃ | H | 4 | H | H | -(CH₂)₂-O-(CH₂)₂- | | 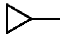 | 233 | - |
| I-1-b-8 | H | CH₃ | H | 4 | H | H | -(CH₂)₂-O-(CH₂)₂- | | 4-Cl-Ph* | 203 | - |
| I-1-b-9 | H | CH₃ | H | 4 | H | H | -(CH₂)₂-O-(CH₂)₂- | | 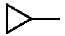 | 207 | - |
| I-1-b-10 | CH₃ | CH₃ | H | 4 | H | H | -(CH₂)₂-O-(CH₂)₂- | | 4-Cl-Ph* | 242 | - |

| | | | | | | | | | | | |
|---|---|---|---|---|---|---|---|---|---|---|---|
| * Ph = Phenyl | | | | | | | | | | | |

### Beispiel (I-1-c-1)

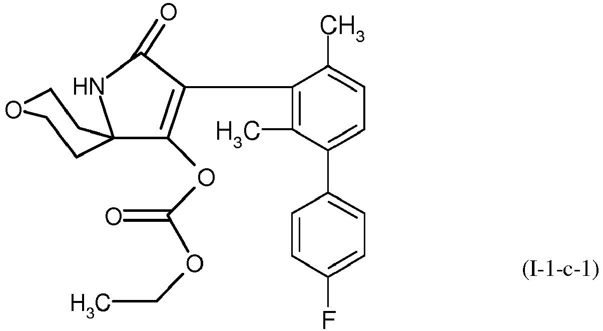

Zu 0,78 g (2 mmol) der Verbindung gemäß Beispiel (I-1-a-4) in 30 ml Dichlormethan gibt man 0,28 ml (2 mmol) Triethylamin. Bei ca. 30°C werden 0.19 ml (2 mmol) Chlorameisensäure-ethylester in 5 ml Dichlormethan zugetropft und bei 30 - 40°C nachgerührt. Nach beendeter Reaktion (Dünnschichtkontrolle) wird das Reaktionsgemisch im Vakuum eingedampft und der Rückstand durch Reversed Phase Chromatographie im Laufmittelsystem Wasser/Acetonitril gereinigt. Man erhält 0,18 g (=̂ 20 % d. Theorie), Fp. 206°C.

In Analogie zu Beispiel (I-1-c-1) und gemäß den allgemeinen Angaben zur Herstellung erhält man folgende Verbindungen der Formel (I-1-c):

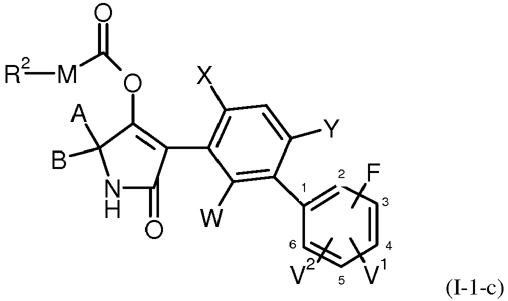

| **Bsp.-Nr.** | **W** | **X** | **Y** | **F** | **V¹** | **V²** | **A** | **B** | **M** | **R²** | **Fp°C** | **Isomer** |
|---|---|---|---|---|---|---|---|---|---|---|---|---|
| I-1-c-2 | CH₃ | CH₃ | H | 4 | H | H | -(CH₂)₂-O-(CH₂)₂- | | O | CH₃ | 242 | - |
| I-1-c-3 | H | Cl | H | 4 | H | H | -(CH₂)₂-O-(CH₂)₂- | | O | CH₃ | 230 | - |
| I-1-c-4 | H | Cl | H | 4 | H | H | -(CH₂)₂-O-(CH₂)₂- | | O | C₂H₅ | 205 | - |
| I-1-c-5 | H | CH₃ | H | 4 | H | H | -(CH₂)₂-O-(CH₂)₂- | | O | C₂H₅ | 200 | - |
| I-1-c-6 | H | CH₃ | H | 4 | H | H | -(CH₂)₂-O-(CH₂)₂- | | O | *Ph-CH₂- | 188 | - |
| I-1-c-7 | CH₃ | CH₃ | H | 4 | H | H | -(CH₂)₂-O-(CH₂)₂- | | O | *Ph-CH₂- | 144 | - |

| | | | | | | | | | | | | |
|---|---|---|---|---|---|---|---|---|---|---|---|---|
| *Ph = Phenyl | | | | | | | | | | | | |

### Beispiel (I-1-f-1)

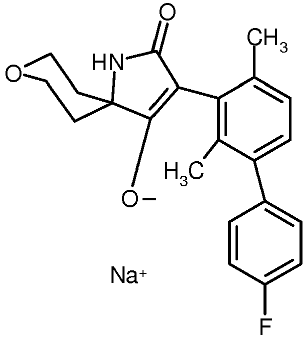

3 ml IN Natronlauge und 7 ml Wasser werden vorgelegt, dann 1,1 g der Verbindung gemäß Bsp. I-1-a-4 portionsweise zugeben, lösen, danach bis zur Trockene einrotieren.
Ausbeute: 1g (80 % d. Theorie)
¹H-NMR (400 MHz, d₆-DMSO): δ = 1.09-1.16 (m,2H,CH₂), 1.91-2.00 (m,2H,CH₂), 2.02, 2.217 (2s, je 3H, Ar-CH₃), 3.55-3.63 (m, 2H, OCH₂), 3.78-3.84 (m, 2H-O-CH₂), 6.75-6.77 (d, 1H, ArH), 6.90-6.95 (m,1H,Ar-H), 7.15-7.20 (m, 2H, Ar-H), 7.23-7.28 (m,2H,AR-H) ppm.

In Analogie zu Beispiel (I-1-f-1) und gemäß den allgemeinen Angaben zur Herstellung erhält man folgende Verbindungen der Formel (I-1-f):

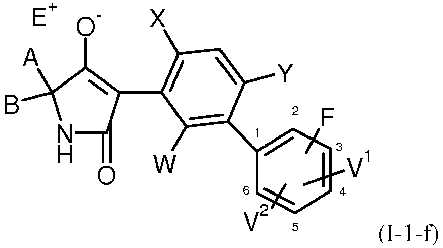

| **Bsp.-Nr.** | **W** | **X** | **Y** | **F** | **V¹** | **V²** | **A** | **B** | **E** | **NMR-Daten** | **Isomer** |
|---|---|---|---|---|---|---|---|---|---|---|---|
| I-1-f-2 | CH₃ | CH₃ | H | 4 | H | H | -(CH₂)₂-O-(CH₂)₂- | | K⁺ | * 2.01,2.16(2s,je 3H,Ar-CH₃) | |
| | | | | | | | | | | 3.55-3.63(m,2H,OCH₂) | |
| | | | | | | | | | | 6.75-6.77(m,1H,Ar-H) | |
| | | | | | | | | | | 6.89-6.91(m,1H,Ar-H) | |
| I-1-f-3 | H | CH₃ | H | 4 | H | H | -(CH₂)₂-O-(CH₂)₂- | | Na⁺ | * 1.90-1.98(m,2H,CH₂) | |
| | | | | | | | | | | 2.23(s,3H,Ar-CH₃) | |
| | | | | | | | | | | 3.56-3.63(m,2H,OCH₂) | |
| | | | | | | | | | | 7.02-7.08(m,2H,Ar-H) | |
| I-1-f-4 | H | CH₃ | H | 4 | H | H | -(CH₂)₂-O-(CH₂)₂- | | K⁺ | * 1.90-1.97(m,2H,CH₂) | |
| | | | | | | | | | | 2.23(s,3H,Ar-CH₃) | |
| | | | | | | | | | | 3.56-3.62(m,2H,OCH₂) | |
| | | | | | | | | | | 7.01-7.07(m,2H,Ar-H) | |
| I-1-f-5 | H | Cl | H | 4 | H | H | -(CH₂)₂-O-(CH₂)₂- | | Na⁺ | * 1.88-1.96(m,2H,CH₂) | |
| | | | | | | | | | | 3.56-3.62(m,2H,O-CH₂) | |
| | | | | | | | | | | 7.56-7.61(m,3H,Ar-H) | |

| | | | | | | | | | | | |
|---|---|---|---|---|---|---|---|---|---|---|---|
| * ¹H-NMR (400 MHz, d₆-DMSO): Verschiebungen δ in ppm | | | | | | | | | | | |

### Beispiel (I-1'-a-1)

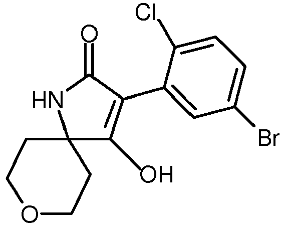

14,1 g Kalium-tert-butylat wird in 50 ml N,N-Dimethylacetamid vorgelegt, bei 50°C wird 22,3 g der Verbindung gemäß Bsp. II-1'-1 gelöst in 50 ml DMA zugetropft und 1 h bei 50°C nachgerührt. Das Reaktionsgemisch wird auf Eiswasser gegossen, mit verd. Salzsäure angesäuert und abgesaugt. Es wird erst mehrmals mit Wasser nachgewaschen und anschließend 2x mit MTBE aufgeschlämmt und abgesaugt. Die Mutterlauge wird nach 4 Tagen erneut filtriert und mit MTBE nachgewaschen.
Ausbeute: 20 g (98 % d. Theorie) Fp. 143°C
MTBE = Methyl-tert.-butylether

In Analogie zu Beispiel (I-1'-a-1) und gemäß den allgemeinen Angaben zur Herstellung in der eingangs erwähnten Literatur erhält man folgende Beispiele der Formel (I-1'-a)

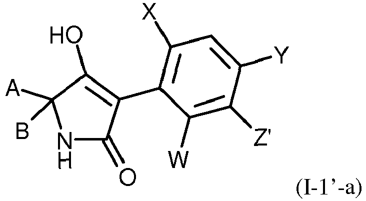

| **Bsp.-Nr.** | **W** | **X** | **Y** | **Z'** | **Fp°C** |
|---|---|---|---|---|---|
| I-1-a'-2 | H | CH₃ | H | Br | 152 |
| I-1-a'-3 | CH₃ | CH₃ | H | Br | 184 |

### Beispiel (II-1'-1)

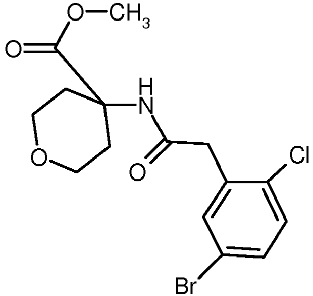

24.9 g 2-Chlor-5-brom-phenylessigsäure wird in 36,5 ml Thionylchlorid bei 80°C bis zur Beendigung der Gasentwicklung gerührt und anschließend eingeengt. Der Rückstand wird in Toluol aufgenommen und nochmals einrotiert (= Säurechlorid). 21.5 g 4-Amino-tetrahydropyran-4-carbonsäuremethylester-hydrochlorid wird in 80 ml Essigester vorgelegt, bei 0°C 110 ml Natronlauge zugegeben, und anschließend unter schnellem Rühren gleichzeitig das Säurechlorid auf 100 ml mit Essigester aufgefüllt und die restliche Natronlauge zugetropft. Nach Beendigung der Reaktion wird das Produkt abgesaugt (1). Das Filtrat wird mit Wasser und Essigester ausgeschüttelt, die organische Phase über Natriumsulfat getrocknet, filtriert und eingeengt. Der Rückstand wird in wenig Essigester verrührt und abgesaugt (2).
Ausbeute 1 + 2: 22.7 g (= 58 % d. Theorie) Fp. 151°C

### Beispiel II-1

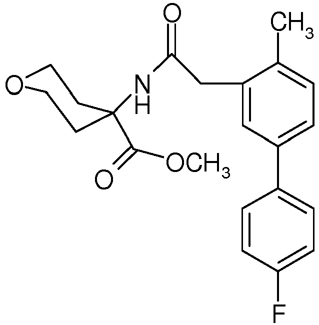

Unter Argon werden 3,23 g 4-Amino-tetrahydropyran-4-carbonsäuremethylester-Hydrochlorid (16,5 mmol) und 75 ml wasserfreies Tetrahydrofuran vorgelegt.

Bei 20°C werden 4,6 ml (33 mmol) Triethylamin zugetropft.

Man rührt 5 Minuten nach und versetzt bei 20°C mit 4,2 g 2-Methyl-5-(4-fluorphenyl)-phenylessigsäure (15 mmol). Nach 15 Minuten tropft man 3,45 ml Triethylamin (25 mmol) dazu und sofort danach 0,93 ml Phosphoroxychlorid (10 mmol), die Lösung soll mäßig sieden. 30 Minuten unter Rückfluss nachrühren.

Nach dünnschichtchromatographischer Kontrolle wird das Lösungsmittel einrotiert und säulenchromatoghraphisch an Kieselgel gereinigt (Dichlormethan: Essigsäureethylester = 3:1)
Ausbeute: 2,19 g (33% d. Theorie), Fp. 113°C

In Analogie zu Beispiel (II-1) und gemäß den allgemeinen Angaben zur Herstellung erhält man folgende Verbindungen der Formel (II):

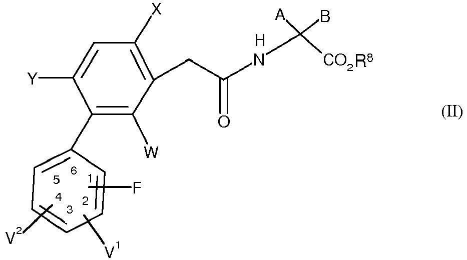

| **Bsp.-Nr.** | **W** | **X** | **Y** | **F** | **V¹** | **V²** | **A** | **B** | **R⁸** | **Fp °C** | **Isomer** |
|---|---|---|---|---|---|---|---|---|---|---|---|
| II-2 | H | CH₃ | H | 4 | 3-F | H | -(CH₂)₂-O-(CH₂)₂- | | CH₃ | 117 | - |
| II-3 | CH₃ | CH₃ | H | 4 | H | H | -(CH₂)₂-O-(CH₂)₂- | | CH₃ | 66 | - |
| II-4 | CH₃ | CH₃ | H | 4 | 3-F | H | -(CH₂)₂-O-(CH₂)₂- | | CH₃ | Wachs | - |
| II-5 | H | CH₃ | H | 3 | 4-Cl | H | -(CH₂)₂-O-(CH₂)₂- | | CH₃ | 176 | - |
| II-6 | H | CH₃ | H | 4 | H | H | -CH₂-CHCH₃-O-(CH₂)₂- | | CH₃ | glasartiger Feststoff | β |
| II-7 | CH₃ | CH₃ | H | 4 | H | H | -CH₂-CHCH₃-O-(CH₂)₂- | | CH₃ | 75 | β |
| II-8 | H | CH₃ | H | 4 | H | H | 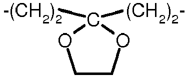 | | CH₃ | 89 | - |
| II-9 | CH₃ | CH₃ | H | 4 | H | H | 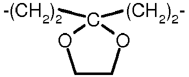 | | CH₃ | 68 | - |
| II-10 | CH₃ | CH₃ | H | 4 | H | H | 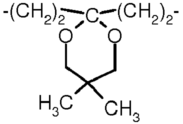 | | CH₃ | 121 | - |
| II-11 | H | Cl | H | 4 | Hl | H | -(CH₂)₂-O-(CH₂)₂- | | CH₃ | Öl | - |
| II-12 | H | CH₃ | H | 4 | H | H | -CH₂-CHOC₂H₅-(CH₂)₃- | | CH₃ | 125 | β |
| II-13 | CH₃ | CH₃ | H | 4 | H | H | -CH₂-CHOC₂H₅-(CH₂)₃- | | CH₃ | 62 | β |
| II-14 | H | CH₃ | H | 4 | H | H | -CH₂-CHOCH₃-(CH₂)₃- | | CH₃ | Harz | Gemisch |
| II-15 | CH₃ | CH₃ | H | 4 | H | H | -CH₂-CHOCH₃-(CH₂)₃- | | CH₃ | Harz | Gemisch |
| II-16 | H | CH₃ | H | 4 | H | H | -(CH₂)₅- | | CH₃ | 137 | - |
| II-17 | CH₃ | CH₃ | H | 4 | H | H | -(CH₂)₅- | | CH₃ | 145 | - |
| II-18 | CH₃ | CH₃ | CH₃ | 4 | H | H | -(CH₂)₂-O-(CH₂)₂- | | CH₃ | 158 | - |
| II-19 | CH₃ | CH₃ | H | 4 | H | H | 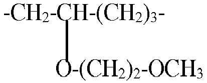 | | CH₃ | * 3.23, 3.24 (2S, zusammen 3H, OCH₃), 3.53 (s,3H, CO₂CH₃), 7.02-7.07 (m,1H,Ar-H). | cis:trans ca. 2:1 |
| II-20 | H | CH₃ | H | 4 | H | H | 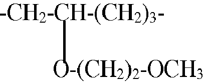 | | CH₃ | * 2.27, 2.29 (2s, zusammen 3H, Ar-CH₃), 3.21 (2s, zusammen 3H, O CH₃), 3.53 (2s, zusammen 3H, CO₂CH₃), 7.20-7.26 (m, 3H,ArH) | cis:trans ca. 1:1 |
| II-21 | H | Cl | H | 4 | H | H | 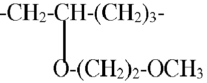 | | CH₃ | * 3.22, 3.23 (2S, zusammen 3H, O CH₃), 3.35-3.49 (m, 4H, O-CH₂-CH₂-O), 3.53, 3.54 (2s, zusammen 3H, CO₂ CH₃), 7.25-7.30 (m, 2H, Ar-H) | cis:trans ca. 1:1 |
| II-22 | H | Cl | H | 4 | H | H | -CH₂-O-(CH₂)₃- | | CH₃ | Öl | - |
| II-23 | CH₃ | CH₃ | H | 4 | H | H | -CH₂-O-(CH₂)₃- | | CH₃ | Wachs | - |
| II-24 | H | CH₃ | H | 4 | H | H | -CH₂-O-(CH₂)₃- | | CH₃ | 111 | - |
| II-25 | CH₃ | CH₃ | H | 4 | H | H | -CH₂-CHOCH₃-(CH₂)₂- | | C₂H₅ | Öl | cis:trans ca. 1:1 |
| II-26 | H | CH₃ | H | 4 | H | H | -CH₂-CHOCH₃-(CH₂)₂- | | C₂H₅ | Öl | cis:trans ca. 1:1 |
| II-27 | CH₃ | CH₃ | H | 4 | H | H | 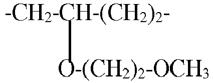 | | C₂H₅ | * 3.66 (m,2H,CH₂-Ar), 4.03 (m,1H,CHO-(CH₂)₂), 4.15 (a, 2H, O-CH₂-CH₃) | cis |
| II-28 | CH₃ | CH₃ | H | 4 | H | H | 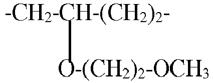 | | C₂H₅ | * 3.66 (m,2H,CH₂-Ar), 4.15 (m, 3H, O-CH₂-CH₃, CHO-(CH₂)₂-) | trans |

| | | | | | | | | | | | |
|---|---|---|---|---|---|---|---|---|---|---|---|
| * ¹H-NMR (400 MHz, CDCl₃): Verschiebungen δ in ppm | | | | | | | | | | | |

In Analogie zu den in WO 2005/016873 beschriebenen Verfahren zur Herstellung von Verbindungen der Formel (XIX) und (XXIII) wurden folgende neue Verbindungen der Formel (XIX) und (XXIII) erhalten :

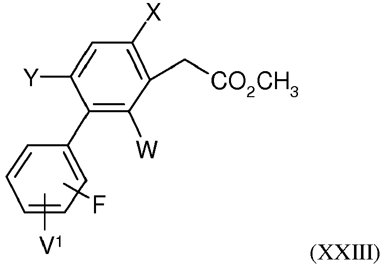

| **Bsp.-Nr.** | **W** | **X** | **Y** | **F** | **V¹** | **Fp. °C** |
|---|---|---|---|---|---|---|
| XXIII-1 | H | CH₃ | H | 4 | 3-F | * Öl |
| XXIII-2 | H | CH₃ | H | 3-F | 4-Cl | * Öl |
| XXIII-3 | CH₃ | CH₃ | H | 4-F | 3-F | * Öl |
| XXIII-4 | CH₃ | CH₃ | H | 3-F | 4-Cl | * Öl |

| | | | | | | |
|---|---|---|---|---|---|---|
| * Die Verbindungen wurden ohne weitere Charakterisierung direkt zu den Verbindungen (XIX) verseift. | | | | | | |

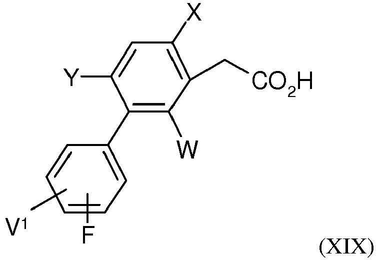

| **Bsp.-Nr.** | **W** | **X** | **Y** | **F** | **V¹** | **¹H-NMR** |
|---|---|---|---|---|---|---|
| XIX-1 | H | CH₃ | H | 4 | 3-F | *2.28 (s, 3H, Ar-CH₃) |
| | | | | | | 3.65 (s, 2H, CH₂) |
| | | | | | | 7.25 (d, 1H, Ar-H) |
| XIX-2 | H | CH₃ | H | 3 | 4-Cl | *2.28 (s, 3H, Ar-CH₃) |
| | | | | | | 3.66 (s, 2H, Ar-CH₂) |
| | | | | | | 7.27 (d, 2H, Ar-H) |
| | | | | | | 7.47-7.50 (m, 1H, Ar-H) |
| XIX-3 | CH₃ | CH₃ | H | 4 | 3-F | *2.13 (s, 3H, Ar-CH₃) |
| | | | | | | 2.31 (s, 3H, Ar-CH₃) |
| | | | | | | 3.68 (s, 2H, CH₂) |
| | | | | | | 6.98 (d, 1H, Ar-H) |
| | | | | | | 7.39-7.46 (m, 1H, Ar-H) |
| XIX-4 | CH₃ | CH₃ | H | 3 | 4-Cl | *2.13 (s, 3H, Ar-CH₃) |
| | | | | | | 2.31 (s, 3H, Ar-CH₃) |
| | | | | | | 3.68 (s, 2H, Ar-CH₂) |
| | | | | | | 7.00 (d, 1H, Ar-H) |
| | | | | | | 7.08-7.12 (m, 2H, Ar-H) |
| | | | | | | 7.25-7.28 (m, 1H, Ar-H) |
| | | | | | | 7.59 (t, 1H, Ar-H) |

| | | | | | | |
|---|---|---|---|---|---|---|
| *¹H-NMR (400 MHz, d₆-DMSO): Verschiebungen δ in ppm | | | | | | |

### Bestimmung der LogP-Werte

Die Bestimmung der in der Tabelle angegebenen logP-Werte erfolgte gemäß EEC-Directive 79/831 Annex V.A8 durch HPLC (High Performance Liquid Chromatography) an einer Phasenumkehrsäule (C18). Temperatur: 43°C

Eluenten für die Bestimmung im sauren Bereich (pH 2,3): 0,1 % wässrige Phosphorsäure, Acetonitril; linearer Gradient von 10% Acetonitril bis 90 % Acetonitril

Die Eichung erfolgte mit unverzweigten Alkan-2-onen (mit 3 bis 16 Kohlenstoffatomen), deren logP-Werte bekannt sind (Bestimmung der logP-Werte anhand der Rententionszeiten durch lineare Interpolation zwischen zwei aufeinander folgenden Alkanonen).

Die lambda-max-Werte wurden anhand der UV-Spektren von 200 nm bis 400 nm in den Maxima der chromatographischen Signale ermittelt.

### Anwendungs beispiele

Beispiel A: Steigerung der Penetration in die Pflanze durch Ammonium- oder Phosphoniumsalze und synergistische Steigerung der Penetration in die Pflanze durch Ammonium- / Phosphoniumsalze in Kombination mit Penetrationsförderern

In diesem Test wurde die Penetration von Wirkstoffen durch enzymatisch isolierte Kutikeln von Apfelbaumblättern gemessen.

Verwendet wurden Blätter, die in voll entwickeltem Zustand von Apfelbäumen der Sorte Golden Delicious abgeschnitten wurden. Die Isolierung der Kutikeln erfolgte in der Weise, dass
- zunächst auf der Unterseite mit Farbstoff markierte und ausgestanzte Blattscheiben mittels Vakuuminfiltration mit einer auf einen pH Wert zwischen 3 und 4 gepufferten Pectinase-Lösung (0,2 bis 2 %ig) gefüllt wurden,
- dann Natriumazid hinzugefügt wurde und
- die so behandelten Blattscheiben bis zur Auflösung der ursprünglichen Blattstruktur und zur Ablösung der nicht zellulären Kutikula stehen gelassen wurden.

Danach wurden nur die von Spaltöffnungen und Haaren freien Kutikeln der Blattoberseiten weiter verwendet. Sie wurden mehrfach abwechselnd mit Wasser und einer Pufferlösung vom pH Wert 7 gewaschen. Die erhaltenen sauberen Kutikel wurden schließlich auf Teflonplättchen aufgezogen und mit einem schwachen Luftstrahl geglättet und getrocknet.

Im nächsten Schritt wurden die so gewonnenen Kutikelmembranen für Membran-Transport-Untersuchungen in Diffusionszellen (= Transportkammern) aus Edelstahl eingelegt. Dazu wurden die Kutikeln mit einer Pinzette mittig auf die mit Silikonfett bestrichenen Ränder der Diffusionszellen plaziert und mit einem ebenfalls gefetteten Ring verschlossen. Die Anordnung war so gewählt worden, dass die morphologische Außenseite der Kutikeln nach außen, also zur Luft, gerichtet war, während die ursprüngliche Innenseite dem Inneren der Diffusionszelle zugewandt war.

Die Diffusionszellen waren mit einer 30%igen Ethylenglykol/Wasser-Lösung befüllt. Zur Bestimmung der Penetration wurden jeweils 10 µl der Spritzbrühe der nachstehenden Zusammensetzung auf die Außenseite der Kutikula appliziert. Das Ansetzen der Spritzbrühe erfolgt mit lokalem Leitungswasser mittlerer Wasserhärte.

Nach dem Auftragen der Spritzbrühen ließ man das Wasser verdunsten, drehte die Kammern um und stellte sie in thermostatisierte Wannen, in denen die Temperatur und Luftfeuchte über der Kutikula durch einen leichten Luftstrom auf die Kutikula mit dem Spritzbelag einstellbar war (20°C, 60 % rh). In regelmäßigen Abständen wurden von einem Autosampler Aliquots entnommen und der Wirkstoffgehalt mit HPLC bestimmt.

Die Versuchsergebnisse gehen aus der folgenden Tabelle hervor. Bei den angegebenen Zahlen handelt es sich um Durchschnittswerte aus 8 bis 10 Messungen. Deutlich ist zu sehen, dass bereits Ammoniumsulfat alleine die Penetration deutlich verbessert und zusammen mit RME ein überadditiver (synergistischer) Effekt auftritt.

**Tabelle A**

| Wirkstoff | | | |
|---|---|---|---|
| | Penetration nach 24h / % | | |
| | EC | EC + RME (1 g/l) | EC + RME (1 g/l) + AS (1 g/l) |
| Beispiel I-1-a-4 | 3 | 8 | 30 |
| 500 ppm in DMF / Emulgator W 7:1 (w/w) | | | |

| | | | |
|---|---|---|---|
| RME = Rapsölmethylester (Einsatz formuliert als 500 EW, Konzentrationsangabe in g Wirkstoff /1) AS = Ammoniumsulfat EC = Emulgierbares Konzentrat | | | |

### Beispiel 1

**Phaedon-Test (PHAECO Spritzbehandlung)**

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele nach 7 d eine Wirkung von ≥ 80 % bei einer Aufwandmenge von 500 g/ha: I-1-a-1, I-1-a-2, I-1-a-3, I-1-a-4, I-1-a-5, I-1-a-6, I-1-a-10, I-1-a-11, I-1-a-21, I-1-a-22, I-1-a-23, I-1-a-24, I-1-a-25, I-1-a-28, I-1-a-27, I-1-a-29, I-1-a-30, I-1-a-31, I-1-a-32, I-1-a-33, I-1-a-34, I-1-a-35, I-1-a-37, I-1-a-38, I-1-a-39, I-1-a-41, I-1-a-52, I-1-b-5, I-1-b-6, I-1-c-3, I-1-c-4, I-1-c-5,1-1-f-1

Bei diesem Test zeigen z. B. die folgenden Verbindungen der Herstellungsbeispiele nach 7 d eine Wirkung von ≥ 80 % bei einer Aufwandmenge von 100 g/ha: I-1-a-7.

### Beispiel 2

**Spodoptera frugiperda-Test (SPODFR Spritzbehandlung)**

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele nach 7 d eine Wirkung von ≥ 80 % bei einer Aufwandmenge von 500 g/ha: I-1-a-1, I-1-a-3, I-1-a-4, I-1-a-5, I-1-a-6, I-1-a-8, I-1-a-10, I-1-a-11, I-1-a-12, I-1-a-16, I-1-a-17, I-1-a-19, I-1-a-21, I-1-a-25, I-1-a-28, I-1-a-29, I-1-a-30, I-1-a-34, I-1-a-35, I-1-a-38, I-1-a-41, I-1-a-42, I-1-a-50, I-1-a-54, I-1-a-47, I-1-a-51, I-1-a-52, I-1-a-53, I-1-a-55, I-1-b-1, I-1-b-2,I-1-b-3, I-1-b-4, I-1-b-5, I-1-b-6, I-1-c-2, I-1-c-3, I-1-c-4, I-1-c-5, I-1-f-1.

### Beispiel 3

**Myzus-Test (MYZUPE Spritzbehandlung)**

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele nach 6 d eine Wirkung von ≥ 80 % bei einer Aufwandmenge von 500 g/ha: I-1-a-1, I-1-a-2, I-1-a-3, I-1-a-4, I-1-a-5, I-1-a-6, I-1-a-7, I-1-a-8, I-1-a-9, I-1-a-10, I-1-a-15, I-1-a-16, I-1-a-17, I-1-a-18, I-1-a-20, I-1-a-21, I-1-a-22, I-1-a-23, I-1-a-24, I-1-a-25, I-1-a-27, I-1-a-28, I-1-a-29, I-1-a-30, I-1-a-31, I-1-a-32, I-1-a-33, I-1-a-34, I-1-a-35, I-1-a-36, I-1-a-37, I-1-a-38, I-1-a-39, I-1-a-40, I-1-a-41, I-1-a-42, I-1-a-43, I-1-a-44, I-1-a-46, I-1-a-47, I-1-a-48, I-1-a-49, I-1-a-50, I-1-a-51, I-1-a-52, I-1-a-53, I-1-a-54, I-1-a-55, I-1-a-56, I-1-a-11, I-1-a-12, I-1-b-1, I-1-b-2, I-1-b-3, I-1-b-4, I-1-b-5, I-1-b-6, I-1-c-1, I-1-c-2, I-1-c-3, I-1-c-4, I-1-c-5, I-1-f-1.

### Beispiel 4

**Tetranychus-Test, OP-resistent (TETRUR Spritzbehandlung)**

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele nach 6 d eine Wirkung von ≥ 80 % bei einer Aufwandmenge von 100 g/ha: I-1-a-3, I-1-a-7, I-1-a-8, I-1-a-9, I-1-a-10, I-1-a-12, I-1-a-16, I-1-a-17, I-1-a-21, I-1-a-22, I-1-a-24, I-1-a-25, I-1-a-28, I-1-a-30, I-1-a-34, I-1-a-35, I-1-a-36, I-1-a-37, I-1-a-38, I-1-a-39, I-1-a-40, I-1-a-41, I-1-a-43, I-1-a-45, I-1-a-46, I-1-a-47, I-1-a-48, I-1-a-49, I-1-a-50, I-1-a-51, I-1-a-52, I-1-a-53, I-1-a-54, I-1-a-56, I-1-b-5, I-1-b-6, I-1-b-4, I-1-b-1, I-1-b-2, I-1-b-3, I-1-c-1, I-1-c-2, I-1-c-4, I-1-c-5, I-1-f-1

Bei diesem Test zeigen z. B. die folgenden Verbindungen der Herstellungsbeispiele nach 6 d eine Wirkung von ≥ 80 % bei einer Aufwandmenge von 20 g/ha: I-1-a-1, I-1-a-4, I-1-a-5, I-1-a-6.

### Beispiel 5

**Meloidogyne-Test (MELGIN Spritzbehandlung)**

| | |
|---|---|
| Lösungsmittel: | 80 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, *Meloidogyne incognita*-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele nach 14 d eine Wirkung von ≥ 80 % bei einer Aufwandmenge von 20 ppm: I-1-a-22, I-1-a-23, I-1-a-25, I-1-a-28, I-1-a-53, I-1-a-55.

### Beispiel 6

**Aphis gossypii -Test; (APHIGO G)**

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Baumwollpflanzen (*Gossypium hirsutum*), die stark von der Baumwollblattlaus (*Aphis gossypii*) befallen sind, werden mit einer Wirkstoffzubereitung der gewünschten Konzentration angegossen.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 20 ppm nach 10 Tagen:
Beispiel Nr. I-1-a-4, I-1-a-8, I-1-a-20, I-1-a-21, I-1-a-23, I-1-a-25, I-1-a-33, I-1-a-34, I-1-a-54, I-1-a-55, I-1-b-1, I-1-b-2, I-1-b-3, I-1-c-1, I-1-c-4, I-1-f-1

### Beispiel 7

**Myzus persicae -Test; (MYZUPE G)**

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Kohlpflanzen (*Brassica oleracea*), die stark von der Grünen Pfirsichblattlaus (*Myzus persicae*) befallen sind, werden mit einer Wirkstoffzubereitung der gewünschten Konzentration angegossen.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 20 ppm nach 10 Tagen:
Beispiel Nr. I-1-a-1, I-1-a-3, I-1-a-4, I-1-a-5, I-1-a-11, I-1-a-17, I-1-a-20, I-1-a-23, I-1-a-25, I-1-a-33, I-1-a-34, I-1-a-56, I-1-b-2, I-1-b-3, I-1-b-4, I-1-c-2, I-1-c-3, I-1-c-4

### Beispiel 8

**Tetranychus-Test; OP-resistent (TETRUR G)**

| | | |
|---|---|---|
| Lösungsmittel: | 7 | Gewichtsteile Dimethylformamid |
| Emulgator: | 2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (*Phaseolus vulgaris*), die stark von allen Stadien der Gemeinen Spinnmilbe (*Tetranychus urticae*) befallen sind, werden mit einer Wirkstoffzubereitung der gewünschten Konzentration angegossen.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 20 ppm nach 14 Tagen:
Example No. I-1-a-3, I-1-a-5, I-1-a-6, I-1-a-8, I-1-a-11, I-1-a-16, I-1-a-17, I-1-a-20, I-1-a-21, I-1-a-23, I-1-a-25, I-1-a-33, I-1-a-34, I-1-a-50, I-1-a-54, I-1-a-55, I-1-b-1, I-1-b-2, I-1-b-3, I-1-b-4, I-1-b-5, I-1-b-6, I-1-c-1, I-1-c-2, I-1-c-3, I-1-c-4, I-1-c-5, I-1-f-1,

### Beispiel 9

**Lucilia cuprina-Test (LUCICU)**

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Wasser und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 100 ppm: I-1-a-3, I-1-a-4, I-1-a-6, I-1-a-12.

### Beispiel 10

**Boophilus microplus -Test (BOOPMI Injektion)**

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Lösungsmittel auf die gewünschte Konzentration.

Die Wirkstofflösung wird in das Abdomen *(Boophilus microplus)* injiziert, die Tiere werden in Schalen überführt und in einem klimatisierten Raum aufbewahrt.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele eine Wirkung von ≥ 80 % bei einer Aufwandmenge von 20 µg/Tier: I-1-a-3, I-1-a-6, I-1-a-12.

### Beispiel 11

**Heliothis virescens - Test - Behandlung transgener Pflanzen**

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

### Beispiel 12

**Grenzkonzentrations-Test / Bodeninsekten - Behandlung transgener Pflanzen**

| | |
|---|---|
| Testinsekt: | Diabrotica balteata - Larven im Boden |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 l Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel 13: Wirkungssteigerung durch Ammonium/Phosphoniumsalze in Kombination mit Penetrationsförderern

**Myzus persicae-Test**

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Für die Anwendung mit Ammonium- oder Phosphoniumsalzen und Penetrationsförderern (Rapsölmethylester 500 EW) werden diese jeweils in einer Konzentration von 1 000 ppm der Spritzbrühe hinzugegeben.

Paprikapflanzen (*Capsicum annuum*), die stark von der Grünen Pfirsichblattlaus (*Myzus persicae*) befallen sind, werden durch Tropfnassspritzung mit der Wirkstoffzubereitung in der gewünschten Konzentration behandelt.

Nach der gewünschten Zeit wird die Abtötung in % bestimmt. Dabei bedeutet 100 %, dass alle Tiere abgetötet wurden; 0 % bedeutet, dass keine Tiere abgetötet wurden.

**Tabelle**

| **Wirkstoff** | **Konzentration (ppm)** | **Abtötung (%) nach 6d** | **+ AS 1000 ppm** | **+ RME 1000 pm** | **+ AS + RME je 1000 ppm** |
|---|---|---|---|---|---|
| I-1-a-1 | 4 | 0 | 15 | 70 | 99 |
| | 0,8 | 0 | 0 | 0 | 15 |
| I-1-a-4 | 4 | 15 | 20 | 5 | 98 |
| | 0,8 | 0 | 0 | 0 | 5 |

### Beispiel 14

**Aphis gossypii -Test**

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Für die Anwendung mit Ammonium- oder Phosphoniumsalzen und Penetrationsförderern (Rapsölmethylester 500 EW) werden diese jeweils in einer Konzentration von 1 000 ppm a.i. der Spritzbrühe hinzugegeben.

Baumwollpflanzen (*Gossypium hirsutum*), die stark von der Baumwollblattlaus (*Aphis gossypii*) befallen sind, werden durch Tropfnassspritzung mit der Wirkstoffzubereitung in der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

**Tabelle**

| **Wirkstoff** | **Konzentration** (**ppm**) | **Abtötung (%) nach 6d** | **+ AS 1000 ppm** | **+ RME 1000 pm** | **+ AS + RME je 1000 ppm** |
|---|---|---|---|---|---|
| I-1-a-1 | 20 | 0 | 5 | 15 | 80 |
| | 4 | 0 | 0 | 0 | 60 |

### Beispiel 15

### Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 1/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von 3 Wochen im Vergleich zu unbehandelten Kontrollen (herbizide Wirkung in Prozent: 100% Wirkung = Pflanzen sind abgestorben, 0 % Wirkung = wie Kontrollpflanzen).

Folgende Verbindungen zeigen neben den zuvor genannten Verbindungen im Vorauflauf mit 320 g/ha a.i. gegen Echinocloa crus-galli und Setaria viridis eine Wirkung von ≥ 80 %: I-1-a-3, I-1-a-7, I-1-a-46, I-1-a-47, I-1-a-48, I-1-a-50, I-1-a-51, I-1-a-53, I-1-a-55

### Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 1/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent: 100% Wirkung = Pflanzen sind abgestorben, 0 % Wirkung = wie Kontrollpflanzen).

Neben den zuvor genannten Verbindungen zeigen folgende Verbindungen im Nachauflauf mit 80 g/ha gegen Echinocloa crus-galli und Setaria viridis eine Wirkung von ≥ 80%: I-1-a-7, I-1-a-11, I-1-a-16, I-1-a-17, I-1-a-18, I-1-a-50, I-1-a-51, I-1-a-54.

## Patentansprüche

1. Verbindungen der Formel (I) 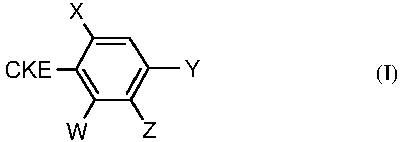 in welcher
W für Wasserstoff oder Methyl steht,
X für Chlor oder Methyl steht,
Y für Wasserstoff oder Methyl steht,
Z für den Rest 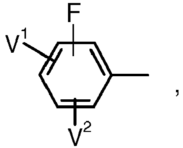 steht
V¹ für Wasserstoff, Fluor, Chlor, Methyl, Methoxy oder Trifluormethyl steht,
V² für Wasserstoff oder Fluor steht,
CKE für die Gruppe 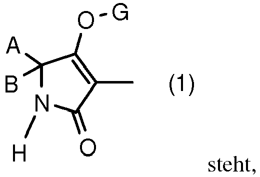
A, B und das Kohlenstoffatom, an das sie gebunden sind, stehen für gesättigtes C₆-Cycloalkyl, in welchem ein Ringglied durch Sauerstoff ersetzt ist und welches gegebenenfalls einfach durch Methyl oder Ethyl, substituiert ist,
G für Wasserstoff (a) oder für eine der Gruppen 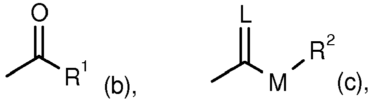 oder E (f) steht, in welchen
L für Sauerstoff steht,
M für Sauerstoff steht und
E für ein Metallionenäquivalent oder ein Ammoniumion steht,
R¹ für jeweils gegebenenfalls einfach durch Fluor oder Chlor substituiertes C₁-C₆-Alkyl, C₂-C₆-Alkenyl, C₁-C₂-Alkoxy-C₁-alkyl, C₁-C₂-Alkylthio-C₁-alkyl oder jeweils gegebenenfalls einfach durch Fluor, Chlor, Methyl oder Methoxy substituiertes Cyclopropyl oder Cyclohexyl, für gegebenenfalls einfach durch Fluor, Chlor, Brom, Cyano, Nitro, Methyl, Methoxy, Trifluormethyl oder Trifluormethoxy substituiertes Phenyl steht,
R² für jeweils gegebenenfalls einfach durch Fluor substituiertes C₁-C₈-Alkyl, C₂-C₆-Alkenyl oder C₁-C₄-Alkoxy-C₂-C₃-alkyl, Phenyl oder Benzyl steht.

2. Verfahren zur Herstellung von Verbindungen der Formel (I) gemäß Anspruch 1, **dadurch gekennzeichnet, dass** man zum Erhalt von
(A) Verbindungen der Formel (I-1-a) 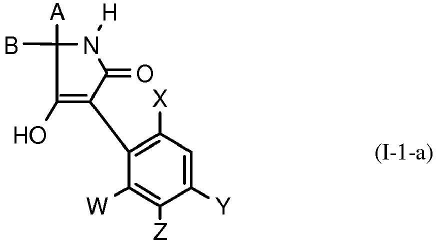 in welcher
A, B, W, X, Y und Z die in Anspruch 1 angegebenen Bedeutungen haben, N-Acylaminosäureester der Formel (II) 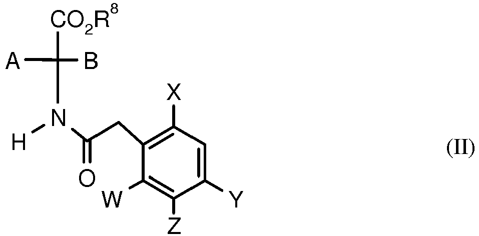 in welcher
A, B, W, X, Y und Z die in Anspruch 1 angegebenen Bedeutungen haben,
und
R⁸ für Alkyl steht,
in Gegenwart eines Verdünnungsmittels und in Gegenwart einer Base intramolekular kondensiert,
(C) Verbindungen der oben gezeigten Formeln (I-1-a), (I-1-b), (I-1-c), (I-1-f), in welchen A, B, G, W, X, Y und Z die in Anspruch 1 angegebene Bedeutung haben, Verbindungen der Formel (I-1'-a), (I-1'-b), (I-1'-c), (I-1'-f),
**(I-1'-a),** (I-1'-b), (I-1'-c), (I-1'-f) 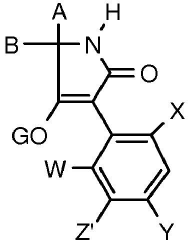 in welchen
A, B, G, W, X und Y die in Anspruch 1 angegebene Bedeutung haben und
Z' für Chlor, Brom, Jod, bevorzugt für Brom steht,
mit Boronsäuren oder Boronsäure-Derivaten der Formel (IV) 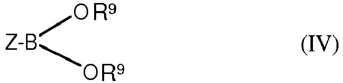 in welcher
R⁹ für Wasserstoff, C₁-C₆-Alkyl oder C₂-C₆-Alkandiyl steht
und
Z die in Anspruch 1 angegebene Bedeutung hat,
in Gegenwart eines Lösungsmittels, einer Base und eines Katalysators umsetzt, wobei als Katalysator insbesondere Palladiumsalze oder Palladiumkomplexe in Frage kommen,
(D) Verbindungen der oben gezeigten Formel (I-1-b), in welchen A, B, R¹, W, X, Y und Z die in Anspruch 1 angebenen Bedeutungen haben, Verbindungen der oben gezeigten Formeln (I-1-a), in welchen A, B, W, X, Y und Z die in Anspruch 1 angegebenen Bedeutungen haben, jeweils
(a) mit Säurehalogeniden der Formel (V) 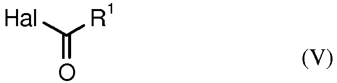 in welcher
R¹ die in Anspruch 1 angegebene Bedeutung hat und
Hal für Halogen steht
oder
(β) mit Carbonsäureanhydriden der Formel (VI)
R¹-CO-O-CO-R¹ (VI)
in welcher
R¹ die in Anspruch 1 angegebene Bedeutung hat, gegebenenfalls in Gegenwart eines Verdünnungsmittels und gegebenenfalls in Gegenwart eines Säurebindemittels umsetzt;
(E) Verbindungen der oben gezeigten Formel (I-1-c), in welchen A, B, R², M, W, X, Y und Z die in Anspruch 1 angegebenen Bedeutungen haben und L für Sauerstoff steht, Verbindungen der oben gezeigten Formeln (I-1-a), in welchen A, B, W, X, Y und Z die in Anspruch 1 angegebenen Bedeutungen haben, jeweils
mit Chlorameisensäureestern der Formel (VII)
R²-M-CO-Cl (VII)
in welcher
R² und M die in Anspruch 1 angegebenen Bedeutungen haben,
gegebenenfalls in Gegenwart eines Verdünnungsmittels und gegebenenfalls in Gegenwart eines Säurebindemittels umsetzt;
(I) Verbindungen der oben gezeigten Formeln (I-1-f), in welchen A, B, E, W, X, Y und Z die in Anspruch 1 angegebenen Bedeutungen haben, Verbindungen der oben gezeigten Formel (I-1-a), in welchen A, B, W, X, Y und Z die in Anspruch 1 angegebenen Bedeutungen haben, jeweils mit Metallverbindungen oder Aminen der Formeln (XI) oder (XII)
Me(OR¹⁰)ₜ (XI) 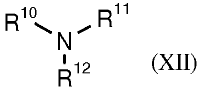
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,

3. Mittel enthaltend einen wirksamen Gehalt an einer Wirkstoffkombination umfassend als Komponenten
(a') mindestens ein biphenylsubstituiertes, spirocyclisches Ketoenol der Formel (I), 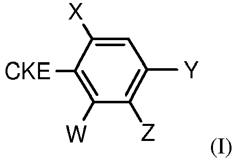 in welcher CKE, W, X, Y und Z die in Anspruch 1 angegebene Bedeutung haben
und
(b') zumindest eine die Kulturpflanzen-Verträglichkeit verbessernde Verbindung aus der folgenden Gruppe von Verbindungen:
4-Dichloracetyl-1-oxa-4-aza-spiro[4.5]-decan (AD-67, MON-4660), 1-Dichloracetyl-hexahydro-3,3, 8a-trimethylpyrrolo[1 ,2-a] -pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenyl-amino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlor-phenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro-[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethylsulfonyl-benzol, 1-[4-(N-2-Methoxybenzoylsulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxybenzoylsulfanoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa) 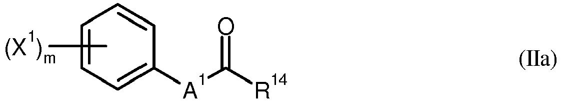 oder der allgemeinen Formel (IIb) 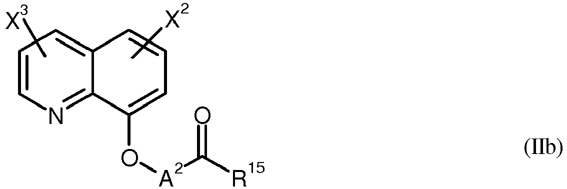 oder der Formel (IIc) 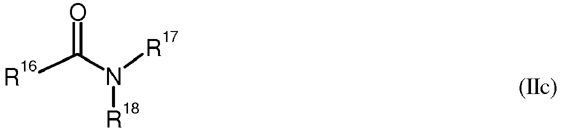 wobei
m für eine Zahl 0, 1, 2, 3, 4 oder 5 steht,
A¹ für eine der nachstehend skizzierten divalenten heterocyclischen Gruppierungen steht, 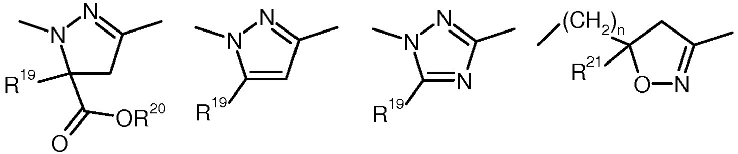
n für eine Zahl 0, 1, 2, 3, 4 oder 5 steht,
A² für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
R¹⁴ für Hydroxy, Mercapto, Amino, C₁-C₆-Alkoxy, C₁-C₆-Alkylthio, C₁-C₆-Alkylamino oder Di-(C₁-C₄-alkyl)-amino steht,
R¹⁵ für Hydroxy, Mercapto, Amino, C₁-C₇-Alkoxy, C₁-C₆-Alkenyloxy, C₁-C₆-Alkenyloxy-C₁-C₆-alkoxy, C₁-C₆-Alkylthio, C₁-C₆-Alkylaniino oder Di-(C₁-C₄-alkyl)-amino steht,
R¹⁶ für gegebenenfalls durch Fluor, Chlor und/oder Brom substituiertes C₁-C₄-Alkyl steht,
R¹⁷ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
R¹⁸ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht, R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅-Oxaalkandiyl steht,
R¹⁹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
R²⁰ für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
R²¹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
X¹ für Nitro, Cyano, Halogen, C₁-C₄-Alkyl, C₁-C₄-Halogenalkyl, C₁-C₄-Alkoxy oder C₁-C₄-Halogenalkoxy steht,
X² für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
X³ für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 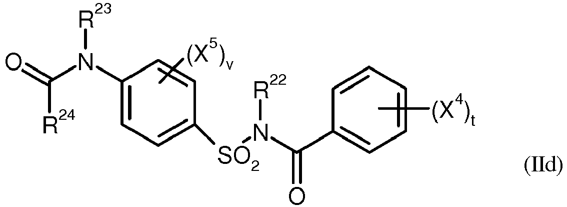
oder der allgemeinen Formel (IIe) 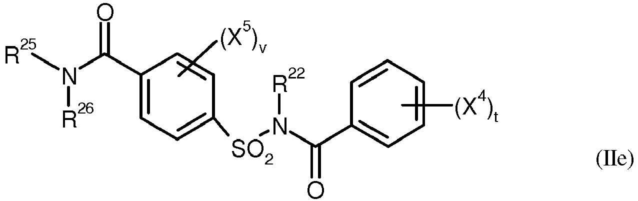 wobei
t für eine Zahl 0, 1, 2, 3, 4 oder 5 steht,
v für eine Zahl 0, 1, 2, 3, 4 oder 5 steht,
R²² für Wasserstoff oder C₁-C₄-Alkyl steht,
R²³ für Wasserstoff oder C₁-C₄-Alkyl steht,
R²⁴ für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkyl-thio oder C₃-C₆-Cycloalkylamino steht,
R²⁵ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
R²⁶ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxaalkandiyl steht,
X⁴ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
X⁵ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

4. Mittel nach Anspruch 3, bei dem die die Kulturpflanzen-Verträglichkeit verbessernde Verbindung aus der folgenden Gruppe von Verbindungen ausgewählt ist:
Cloquintocet-mexyl, Fenchlorazole-ethyl, Isoxadifen-ethyl, Mefenpyr-diethyl, Furilazole, Fenclorim, Cumyluron, Dymron oder die Verbindungen 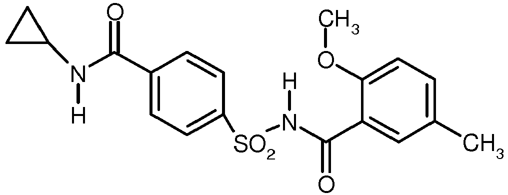 und

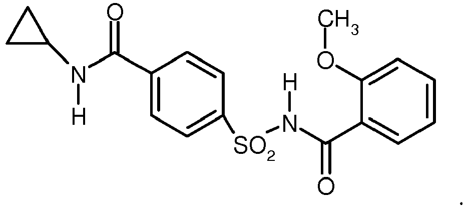

5. Mittel gemäß einem der Ansprüche 3 oder 4, bei denen die Kulturpflanzen-Verträglichkeit verbessernde Verbindung Cloquintocet-mexyl ist.

6. Mittel gemäß einem der Ansprüche 3 oder 4, bei denen die Kulturpflanzen-Verträglichkeit verbessernde Verbindung Mefenpyr-diethyl ist.

7. Zusammensetzung umfassend
- mindestens eine Verbindung der Formel (I) gemäß Anspruch 1 oder ein Mittel gemäß Anspruch 3 und
- mindestens ein Salz der Formel (III') 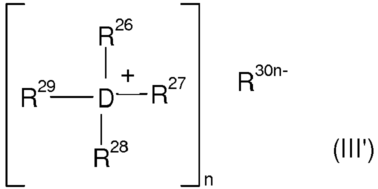 in welcher
D für Stickstoff oder Phosphor steht,
R²⁶, R²⁷, R²⁸ und R²⁹ unabhängig voneinander für Wasserstoff oder jeweils gegebenenfalls substituiertes C₁-C₈-Alkyl oder einfach oder mehrfach, ungesättigtes, gegebenenfalls substituiertes C₁-C₈-Alkylen stehen, wobei die Substituenten aus Halogen, Nitro und Cyano ausgewählt sein können,
n für 1,2,3 oder 4 steht,
R³⁰ für ein anorganisches oder organisches Anion steht.

8. Zusammensetzung gemäß Anspruch 7, **dadurch gekennzeichnet, dass** sie mindestens einen Penetrationsförderer enthält.

9. Verwendung von Verbindungen der Formel (I) gemäß Anspruch 1 zur Herstellung von Schädlingsbekämpfungsmitteln und/oder Herbiziden.

10. Schädlingsbekämpfungsmittel und/oder Herbizide, **gekennzeichnet durch** einen Gehalt an mindestens einer Verbindung der Formel (I) gemäß Anspruch 1.

11. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge und/oder ihren Lebensraum einwirken lässt, ausgenommen sind Verfahren zur chirurgischen oder therapeutischen Behandlung des menschlichen oder tierischen Körpers.

12. Verwendung von Verbindungen der Formel (I) gemäß Anspruch 1 zur Bekämpfung von tierischen Schädlingen und/oder unerwünschtem Pflanzenbewuchs, wobei die Verwendung zur chirurgischen oder therapeutischen Behandlung des menschlichen oder tierischen Körpers ausgenommen ist.

13. Verfahren zur Herstellung von Schädlingsbekämpfungsmitteln und/oder Herbiziden, **dadurch gekennzeichnet, dass** man Verbindungen der Formel (I) gemäß Anspruch 1 mit Streckmitteln und/oder oberflächenaktiven Stoffen vermischt.

14. Verfahren zum Bekämpfen von unerwünschtem Pflanzenwuchs, **dadurch gekennzeichnet, dass** man ein Mittel gemäß Anspruch 3 auf die Pflanzen oder ihre Umgebung einwirken lässt.

15. Verwendung eines Mittels gemäß Anspruch 3 zum Bekämpfen von unerwünschten Pflanzenwuchs.

16. Verfahren zur Bekämpfung von unerwünschtem Pflanzenwuchs, **dadurch gekennzeichnet, dass** man Verbindungen der Formel (I) gemäß Anspruch 1 und die die Kulturpflanzenverträglichkeit verbessernde Verbindung gemäß Anspruch 3 in zeitlich naher Abfolge getrennt auf die Pflanzen oder ihre Umgebung einwirken lässt.

17. Verfahren zur Steigerung der Wirkung von Schädlingsbekämpfungsmitteln und/oder Herbiziden enthaltend einen Wirkstoff der Formel (I) gemäß Anspruch 1 oder ein Mittel gemäß Anspruch 3, **dadurch gekennzeichnet, dass** das anwendungsfertige Mittel (Spritzbrühe) unter Einsatz eines Salzes der Formel (III') gemäß Anspruch 7 zubereitet wird, ausgenommen sind Verfahren zur chirurgischen oder therapeutischen Behandlung des menschlichen oder tierischen Körper.

18. Verfahren gemäß Anspruch 17, **dadurch gekennzeichnet, dass** die Spritzbrühe unter Einsatz eines Penetrationsförderers zubereitet wird.

19. Verbindungen der Formel (II) 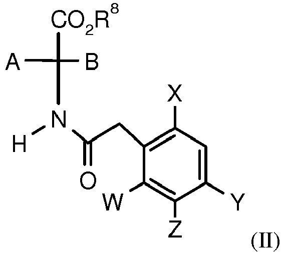 in welcher
A, B, R⁸, W, X, Y und Z die in Anspruch 1 oder 2angegebnenen Bedeutungen haben.

20. Verbindungen der Formel (XVII) 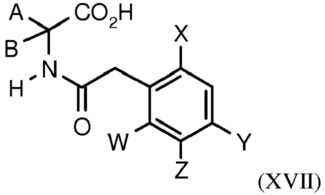 in welcher
A, B, W, X, Y und Z die in Anspruch 1 angegebenen Bedeutungen haben.

21. Verbindungen der Formel (XXI) 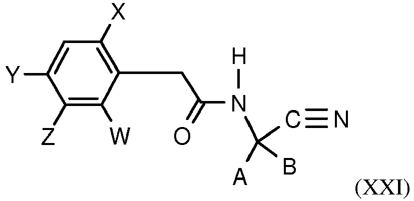 in welcher
A, B, W, X, Y und Z die in Anspruch 1 angegebenen Bedeutungen haben.

22. Verbindungen der Formel (XXIII) 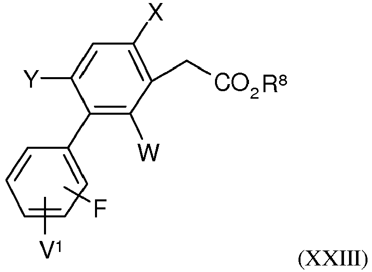 in welcher die Substituenten W, X, Y, F, V¹ und R⁸ die in der Tabelle angegebenen Bedeutungen haben
| **W** | **X** | **Y** | **F** | **V¹** | **R**⁸ |
|---|---|---|---|---|---|
| H | CH₃ | H | 4 | 3-F | CH₃ |
| H | CH₃ | H | 3-F | 4-Cl | CH₃ |
| CH₃ | CH₃ | H | 4-F | 3-F | CH₃ |
| CH₃ | CH₃ | H | 3-F | 4-Cl | CH₃ |

23. Verbindungen der Formel (XIX) 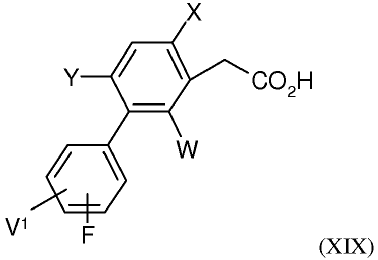 in welcher W, X, Y, F, V¹ die in der Tabelle angegebenen Bedeutungen haben
| **W** | **X** | **Y** | **F** | **V¹** |
|---|---|---|---|---|
| H | CH₃ | H | 4 | 3-F |
| H | CH₃ | H | 3 | 4-Cl |
| CH₃ | CH₃ | H | 4 | 3-F |
| CH₃ | CH₃ | H | 3 | 4-Cl |

## Claims

1. Compounds of the formula (I) 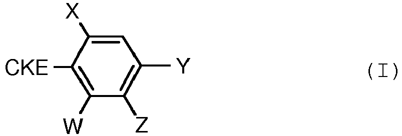 in which
W represents hydrogen or methyl,
X represents chlorine or methyl,
Y represents hydrogen or methyl,
Z represents the radical 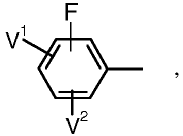
V¹ represents hydrogen, fluorine, chlorine, methyl, methoxy or trifluoromethyl,
V² represents hydrogen or fluorine,
CKE represents the group 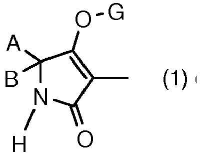
A, B and the carbon atom to which they are attached represent saturated C6-cycloalkyl in which one ring member is replaced by oxygen and which is optionally monosubstituted by methyl or ethyl,
G represents hydrogen (a) or represents one of the groups 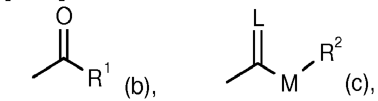
or E (f), in which
L represents oxygen,
M represents oxygen and
E represents a metal ion equivalent or an ammonium ion,
R¹ represents C₁-C₆-alkyl, C₂-C₆-alkenyl, C₁-C₂-alkoxy-C₁-alkyl, C₁-C₂-alkylthio-C₁-alkyl, each of which is optionally monosubstituted by fluorine or chlorine, or represents cyclopropyl or cyclohexyl, each of which is optionally monosubstituted by fluorine, chlorine, methyl or methoxy, represents phenyl which is optionally monosubstituted by fluorine, chlorine, bromine, cyano, nitro, methyl, methoxy, trifluoromethyl or trifluoromethoxy,
R² represents C₁-C₈-alkyl, C₂-C₆-alkenyl or C₁-C₄-alkoxy-C₂-C₃-alkyl, phenyl or benzyl, each of which is optionally monosubstituted by fluorine.

2. Process for preparing compounds of the formula (I) according to Claim 1, **characterized in that**, to obtain
(A) compounds of the formula (I-1-a) 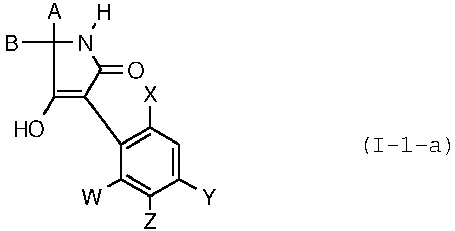 in which
A, B, W, X, Y and Z have the meanings given in Claim 1,
N-acylamino acid esters of the formula (II) 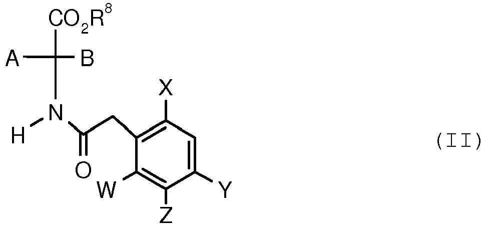 in which
A, B, W, X, Y and Z have the meanings given in Claim 1,
and
R⁸ represents alkyl
are condensed intramolecularly in the presence of a diluent and in the presence of a base,
(C) compounds of the formulae (I-1-a), (I-1-b), (I-1-c), (I-1-f), shown above in which A, B, G, W, X, Y and Z have the meaning given in Claim 1, compounds of the formulae (I-1'-a), (I-1'-b), (I-1'-c), (I-1'-f),
**(I-1'-a),** (I-1'-b), (I-1'-c), (I-1'-f) 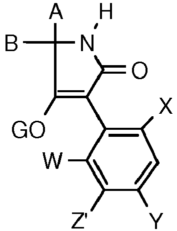 in which
A, B, G, W, X and Y have the meaning given in Claim 1 and
Z' represents chlorine, bromine, iodine, preferably bromine,
are reacted with boronic acids or boronic acid derivatives of the formula (IV) 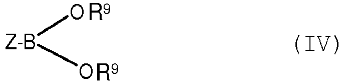 in which
R⁹ represents hydrogen, C₁-C₆-alkyl or C₂-C₆-alkanediyl
and
Z has the meaning given in Claim 1
in the presence of a solvent, a base and a catalyst, suitable catalysts being in particular palladium salts or palladium complexes,
(D) compounds of the formula (I-1-b) shown above in which A, B, R¹, W, X, Y and Z have the meanings given in Claim 1, compounds of the formulae (I-1-a) shown above in which A, B, W, X, Y and Z have the meanings given in Claim 1 are in each case reacted
(α) with acid halides of the formula (V) 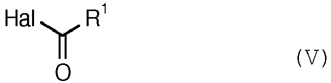 in which
R¹ has the meaning given in Claim 1 and
Hal represents halogen
or
(β) with carboxylic anhydrides of the formula (VI)
R¹-CO-O-CO-R¹ (VI)
in which
R¹ has the meaning given in Claim 1,
if appropriate in the presence of a diluent and if appropriate in the presence of an acid binder;
(E) compounds of the formula (I-1-c) shown above in which A, B, R², M, W, X, Y and Z have the meanings given in Claim 1 and L represents oxygen, compounds of the formulae (I-1-a) shown in Claim 1 in which A, B, W, X, Y and Z have the meanings given in Claim 1 are in each case reacted
with chloroformic esters of the formula (VII)
R²-M-CO-Cl (VII)
in which
R² and M have the meanings given in Claim 1,
if appropriate in the presence of a diluent and if appropriate in the presence of an acid binder;
(I) compounds of the formulae (I-1-f) shown above in which A, B, E, W, X, Y and Z have the meanings given in Claim 1, compounds of the formula (I-1-a) shown above in which A, B, W, X, Y and Z have the meanings given in Claim 1 are in each case reacted
with metal compounds or amines of the formulae (XI) and (XII), respectively,
Me(OR¹⁰)ₜ (XI) 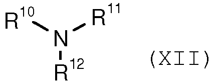
in which
Me represents a mono- or divalent metal,
t represents the number 1 or 2 and
R¹⁰, R¹¹, R¹² independently of one another represent hydrogen or alkyl,
if appropriate in the presence of a diluent.

3. Composition comprising an effective amount of an active compound combination comprising, as components,
(a') at least one biphenyl-substituted spirocyclic ketoenol of the formula (I) 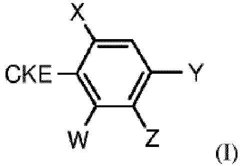 in which CKE, W, X, Y and Z have the meaning given in Claim 1
and
(b') at least one crop plant compatibility-improving compound from the following group of compounds:
4-dichloroacetyl-1-oxa-4-azaspiro[4.5]decane (AD-67, MON-4660), 1-dichloroacetylhexahydro-3,3,8a-trimethylpyrrolo[1,2-a]pyrimidin-6(2H)-one (dicyclonon, BAS-145138), 4-dichloroacetyl-3,4-dihydro-3-methyl-2H-1,4-benzoxazine (benoxacor), 1-methylhexyl 5-chloroquinoline-8-oxyacetate (cloquintocet-mexyl - cf. also related compounds in EP-A-86750, EP-A-94349, EP-A-191736, EP-A-492366), 3-(2-chlorobenzyl)-1-(1-methyl-1-phenylethyl)urea (cumyluron), a-(cyanomethoximino)phenylacetonitrile (cyometrinil), 2,4-dichlorophenoxyacetic acid (2,4-D), 4-(2,4-dichlorophenoxy)butyric acid (2,4-DB), 1-(1-methyl-1-phenylethyl)-3-(4-methylphenyl)urea (daimuron, dymron), 3,6-dichloro-2-methoxybenzoic acid (dicamba), S-1-methyl-1-phenylethyl piperidine-1-thiocarboxylate (dimepiperate), 2,2-dichloro-N-(2-oxo-2-(2-propenylamino)ethyl)-N-(2-propenyl)acetamide (DKA-24), 2,2-dichloro-N,N-di-2-propenylacetamide (dichlormid), 4,6-dichloro-2-phenylpyrimidine (fenclorim), ethyl 1-(2,4-dichlorophenyl)-5-trichloromethyl-1H-1,2,4-triazole-3-carboxylate (fenchlorazole-ethyl - cf. also related compounds in EP-A-174562 and EP-A-346620), phenylmethyl 2-chloro-4-trifluoromethylthiazole-5-carboxylate (flurazole), 4-chloro-N-(1,3-dioxolan-2-ylmethoxy)-α-trifluoroacetophenone oxime (fluxofenim), 3-dichloroacetyl-5-(2-furanyl)-2,2-dimethyloxazolidine (furilazole, MON-13900), ethyl 4,5-dihydro-5,5-diphenyl-3-isoxazolecarboxylate (isoxadifen-ethyl - cf. also related compounds in WO-A-95/07897), 1-(ethoxycarbonyl)ethyl 3,6-dichloro-2-methoxybenzoate (lactidichlor), (4-chloro-o-tolyloxy)acetic acid (MCPA), 2-(4-chloro-o-tolyloxy)propionic acid (mecoprop), diethyl 1-(2,4-dichorophenyl)-4,5-dihydro-5-methyl-1H-pyrazole-3,5-dicarboxylate (mefenpyr-diethyl - cf. also related compounds in WO-A-91/07874), 2-dichloromethyl-2-methyl-1,3-dioxolane (MG-191), 2-propenyl-1-oxa-4-azaspiro[4.5]decane-4-carbodithioate (MG-838), 1,8-naphthalic anhydride, α-(1,3-dioxolan-2-ylmethoximino)phenylacetonitrile (oxabetrinil), 2,2-dichloro-N-(1,3-dioxolan-2-ylmethyl)-N-(2-propenyl)acetamide (PPG-1292), 3-dichloroacetyl-2,2-dimethyloxazolidine (R-28725), 3-dichloroacetyl-2,2,5-trimethyloxazolidine (R-29148), 4-(4-chloro-o-tolyl)butyric acid, 4-(4-chlorophenoxy)butyric acid, diphenylmethoxyacetic acid, methyl diphenylmethoxyacetate, ethyl diphenylmethoxyacetate, methyl 1-(2-chlorophenyl)-5-phenyl-1H-pyrazole-3-carboxylate, ethyl 1-(2,4-dichlorophenyl)-5-methyl-1H-pyrazole-3-carboxylate, ethyl 1-(2,4-dichlorophenyl)-5-isopropyl-1H-pyrazole-3-carboxylate, ethyl 1-(2,4-dichlorophenyl)-5-(1,1-dimethylethyl)-1H-pyrazole-3-carboxylate, ethyl 1-(2,4-dichlorophenyl)-5-phenyl-1H-pyrazole-3-carboxylate (cf. also related compounds in EP-A-269806 and EP-A-333131), ethyl 5-(2,4-dichlorobenzyl)-2-isoxazoline-3-carboxylate, ethyl 5-phenyl-2-isoxazoline-3-carboxylate, ethyl 5-(4-fluorophenyl)-5-phenyl-2-isoxazoline-3-carboxylate (cf. also related compounds in WO-A-91/08202), 1,3-dimethylbut-1-yl 5-chloroquinoline-8-oxyacetate, 4-allyloxybutyl 5-chloroquinoline-8-oxyacetate, 1-allyloxyprop-2-yl 5-chloroquinoline-8-oxyacetate, methyl 5-chloroquinoxaline-8-oxyacetate, ethyl 5-chloroquinoline-8-oxyacetate, allyl 5-chloroquinoxaline-8-oxyacetate, 2-oxoprop-1-yl 5-chloroquinoline-8-oxyacetate, diethyl 5-chloroquinoline-8-oxymalonate, diallyl 5-chloroquinoxaline-8-oxymalonate, diethyl 5-chloroquinoline-8-oxymalonate (cf. also related compounds in EP-A-582198), 4-carboxychroman-4-ylacetic acid (AC-304415, cf. EP-A-613618), 4-chlorophenoxyacetic acid, 3,3'-dimethyl-4-methoxybenzophenone, 1-bromo-4-chloromethylsulphonylbenzene, 1-[4-(N-2-methoxybenzoylsulphamoyl)phenyl]-3-methylurea (also known as N-(2-methoxybenzoyl)-4-[(methylaminocarbonyl)amino]benzenesulphonami de), 1-[4-(N-2-methoxybenzoylsulphamoyl)phenyl]-3,3-dimethylurea, 1-[4-(N-4,5-dimethylbenzoylsulphamoyl)phenyl]-3-methylurea, 1-[4-(N-naphthylsulphamoyl)phenyl]-3,3-dimethylurea, N-(2-methoxy-5-methylbenzoyl)-4-(cyclopropylaminocarbonyl)benzenesulphonamide,
and/or one of the following compounds, defined by general formulae
of the general formula (IIa) 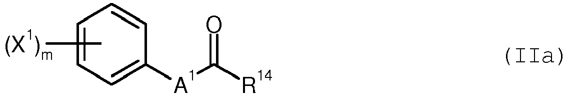 or of the general formula (IIb) 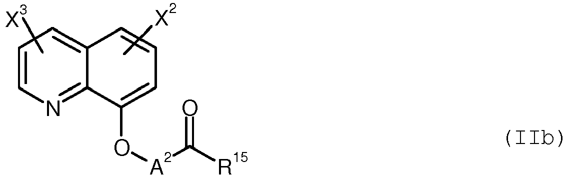 or of the formula (IIc) 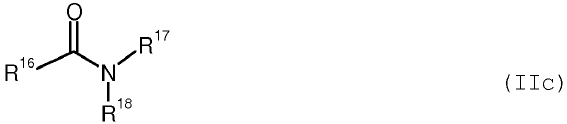 where
m represents a number 0, 1, 2, 3, 4 or 5,
A¹ represents one of the divalent heterocyclic groupings shown below 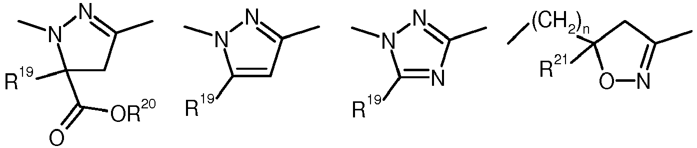
n represents a number 0, 1, 2, 3, 4 or 5,
A² represents optionally C₁-C₄-alkyl- and/or C₁-C₄-alkoxy-carbonyl- and/or C₁-C₄-alkenyloxycarbonyl-substituted alkanediyl having 1 or 2 carbon atoms,
R¹⁴ represents hydroxyl, mercapto, amino, C₁-C₆-alkoxy, C₁-C₆-alkylthio, C₁-C₆-alkylamino or di (C₁-C₄-alkyl) amino,
R¹⁵ represents hydroxyl, mercapto, amino, C₁-C₇-alkoxy, C₁-C₆-alkenyloxy, C₁-C₆-alkenyloxy-C₁-C₆-alkoxy, C₁-C₆-alkylthio, C₁-C₆-alkylamino or di (C₁-C₄-alkyl) -amino,
R¹⁶ represents optionally fluorine-, chlorine- and/or bromine-substituted C₁-C₄-alkyl,
R¹⁷ represents hydrogen, in each case optionally fluorine-, chlorine- and/or bromine-substituted C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, or optionally fluorine-, chlorine- and/or bromine- or C₁-C₄-alkyl-substituted phenyl,
R¹⁸ represents hydrogen, in each case optionally fluorine-, chlorine- and/or bromine-substituted C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, or optionally fluorine-, chlorine- and/or bromine- or C₁-C₄-alkyl-substituted phenyl, R¹⁷ and R¹⁸ also together represent C₃-C₆-alkanediyl or C₂-C₅-oxaalkanediyl, each of which is optionally substituted by C₁-C₄-alkyl, phenyl, furyl, a fused benzene ring or by two substituents which, together with the C atom to which they are attached, form a 5- or 6-membered carbocycle,
R¹⁹ represents hydrogen, cyano, halogen, or represents in each case optionally fluorine-, chlorine- and/or bromine-substituted C₁-C₄-alkyl, C₃-C₆-cycloalkyl or phenyl,
R²⁰ represents hydrogen, in each case optionally hydroxyl-, cyano-, halogen- or C₁-C₄-alkoxy-substituted C₁-C₆-alkyl, C₃-C₆-cycloalkyl or tri- (C₁-C₄-alkyl) silyl,
R²¹ represents hydrogen, cyano, halogen, or represents in each case optionally fluorine-, chlorine- and/or bromine-substituted C₁-C₄-alkyl, C₃-C₆-cycloalkyl or phenyl,
X¹ represents nitro, cyano, halogen, C₁-C₄-alkyl, C₁-C₄-haloalkyl, C₁-C₄-alkoxy or C₁-C₄-haloalkoxy,
X² represents hydrogen, cyano, nitro, halogen, C₁-C₄-alkyl, C₁-C₄-haloalkyl, C₁-C₄-alkoxy or C₁-C₄-haloalkoxy,
X³ represents hydrogen, cyano, nitro, halogen, C₁-C₄-alkyl, C₁-C₄-haloalkyl, C₁-C₄-alkoxy or C₁-C₄-haloalkoxy,
and/or the following compounds, defined by general formulae
of the general formula (IId) 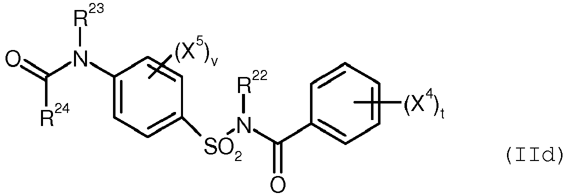 or of the general formula (IIe) 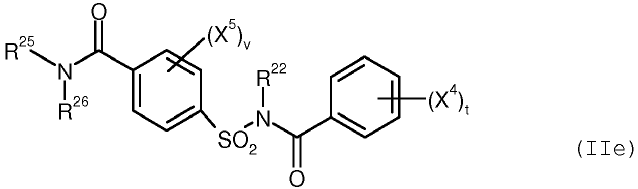 where
t represents a number 0, 1, 2, 3, 4 or 5,
v represents a number 0, 1, 2, 3, 4 or 5,
R²² represents hydrogen or C₁-C₄-alkyl,
R²³ represents hydrogen or C₁-C₄-alkyl,
R²⁴ represents hydrogen, in each case optionally cyano-, halogen- or C₁-C₄-alkoxy-substituted C₁-C₆-alkyl, C₁-C₆-alkoxy, C₁-C₆-alkylthio, C₁-C₆-alkylamino or di (C₁-C₄-alkyl) amino, or in each case optionally cyano-, halogen- or C₁-C₄-alkyl-substituted C₃-C₆-cycloalkyl, C₃-C₆-cycloalkyloxy, C₃-C₆-cycloalkylthio or C₃-C₆-cycloalkylamino,
R²⁵ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, or optionally cyano-, halogen- or C₁-C₄-alkyl-substituted C₃-C₆-cycloalkyl,
R²⁶ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, optionally cyano-, halogen- or C₁-C₄-alkyl-substituted C₃-C₆-cycloalkyl, or optionally nitro-, cyano-, halogen-, C₁-C₄-alkyl-, C₁-C₄-haloalkyl-, C₁-C₄-alkoxy- or C₁-C₄-haloalkoxy-substituted phenyl, or together with R²⁵ represents in each case optionally C₁-C₄-alkyl-substituted C₂-C₆-alkanediyl or C₂-C₅-oxaalkanediyl,
X⁴ represents nitro, cyano, carboxyl, carbamoyl, formyl, sulphamoyl, hydroxyl, amino, halogen, C₁-C₄-alkyl, C₁-C₄-haloalkyl, C₁-C₄-alkoxy or C₁-C₄-haloalkoxy, and
X⁵ represents nitro, cyano, carboxyl, carbamoyl, formyl, sulphamoyl, hydroxyl, amino, halogen, C₁-C₄-alkyl, C₁-C₄-haloalkyl, C₁-C₄-alkoxy or C₁-C₄-haloalkoxy.

4. Composition according to Claim 3 in which the crop plant compatibility-improving compound is selected from the following group of compounds:
cloquintocet-mexyl, fenchlorazole-ethyl, isoxadifen-ethyl, mefenpyr-diethyl, furilazole, fenclorim, cumyluron, dymron or the compounds 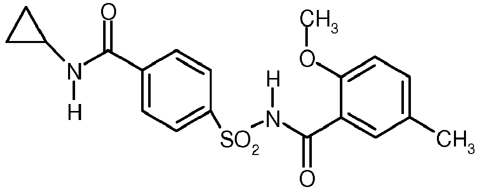 and

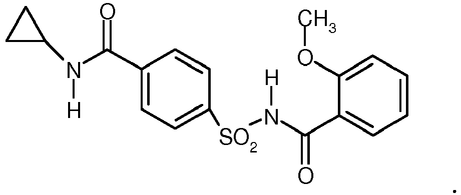

5. Composition according to one of Claims 3 and 4, in which the crop plant compatibility-improving compound is cloquintocet-mexyl.

6. Composition according to one of Claims 3 and 4, in which the crop plant compatibility-improving compound is mefenpyr-diethyl.

7. Composition comprising
- at least one compound of the formula (I) according to Claim 1 or a composition according to Claim 3 and
- at least one salt of the formula (III') 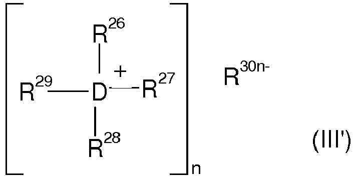 in which
D represents nitrogen or phosphorus,
R²⁶, R²⁷, R²⁸ and R²⁹ independently of one another represent hydrogen or in each case optionally substituted C₁-C₈-alkyl or mono- or polyunsaturated, optionally substituted C₁-C₈-alkylene, the substituents being selectable from halogen, nitro and cyano,
n represents 1, 2, 3 or 4,
R³⁰ represents an inorganic or organic anion.

8. Composition according to Claim 7, **characterized in that** it comprises at least one penetrant.

9. Use of compositions of the formula (I) according to Claim 1 for preparing pesticides and/or herbicides.

10. Pesticides and/or herbicides, **characterized in that** they comprise at least one compound of the formula (I) according to Claim 1.

11. Method for controlling animal pests and/or unwanted vegetation, **characterized in that** compounds of the formula (I) according to Claim 1 are allowed to act on pests and/or their habitat, with the exception of methods for the surgical or therapeutic treatment of the human or animal body.

12. Use of compounds of the formula (I) according to Claim 1, for controlling animal pests and/or unwanted vegetation, the use for surgical or therapeutic treatment of the human or animal body being excluded.

13. Process for preparing pesticides and/or herbicides, **characterized in that** compounds of the formula (I) according to Claim 1 are mixed with extenders and/or surfactants.

14. Method for controlling unwanted vegetation, **characterized in that** a composition according to Claim 3 is allowed to act on the plants or their surroundings.

15. Use of a composition according to Claim 3 for controlling unwanted vegetation.

16. Method of controlling unwanted vegetation, **characterized in that** compounds of the formula (I) according to Claim 1 and the crop plant compatibility-improving compound according to Claim 3 are caused to act, separately in close temporal succession, on the plants or their surroundings.

17. Method for increasing the action of pesticides and/or herbicides comprising an active compound of the formula (I) according to Claim 1 or a composition according to Claim 3, **characterized in that** the ready-to-use composition (spray liquor) is prepared using a salt of the formula (III') according to Claim 7, with the exception of methods for the surgical or therapeutic treatment of the human or animal body.

18. Method according to Claim 17, **characterized in that** the spray liquor is prepared using a penetrant.

19. Compounds of the formula (II) 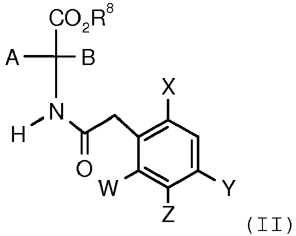 in which
A, B, R⁸, W, X, Y and Z have the meanings given in Claim 1 or 2.

20. Compounds of the formula (XVII) 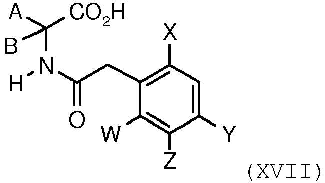 in which
A, B, W, X, Y and Z have the meanings given in Claim 1.

21. Compounds of the formula (XXI) 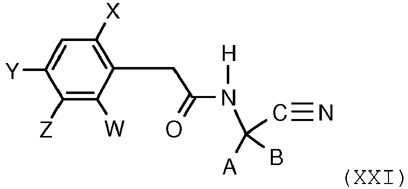 in which
A, B, W, X, Y and Z have the meanings given in Claim 1.

22. Compounds of the formula (XXIII) 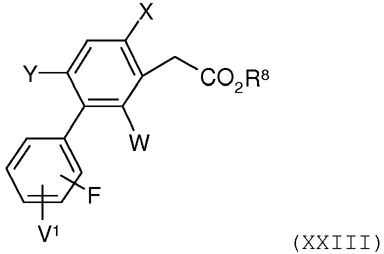 in which the substituents W, X, Y, F, V¹ and R⁸ have the meanings given in the Table
| **W** | **X** | **Y** | **F** | **V**¹ | **R**⁸ |
|---|---|---|---|---|---|
| H | CH₃ | H | 4 | 3-F | CH₃ |
| H | CH₃ | H | 3-F | 4-Cl | CH₃ |
| CH₃ | CH₃ | H | 4-F | 3-F | CH₃ |
| CH₃ | CH₃ | H | 3-F | 4-Cl | CH₃ |

23. Compounds of the formula (XIX) 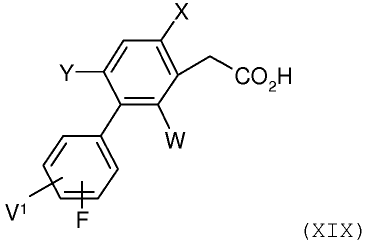 in which W, X, Y, F, V¹ have the meanings given in the Table
| **W** | **X** | **Y** | **F** | **V¹** |
|---|---|---|---|---|
| H | CH₃ | H | 4 | 3-F |
| H | CH₃ | H | 3 | 4-Cl |
| CH₃ | CH₃ | H | 4 | 3-F |
| CH₃ | CH₃ | H | 3 | 4-Cl |

## Revendications

1. Composés de formule (I) 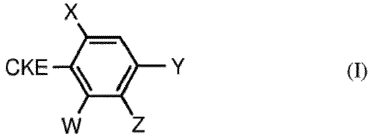 dans lesquels
W représente hydrogène ou méthyle,
X représente chlore ou méthyle,
Y représente hydrogène ou méthyle,
Z représente le radical 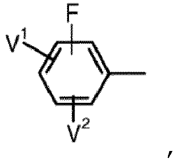
V¹ représente hydrogène, fluor, chlore, méthyle, méthoxy ou trifluorométhyle,
V² représente hydrogène ou fluor,
CKE représente le groupe 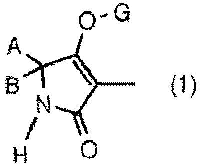
A, B et l'atome de carbone auquel ils sont reliés représentent un cycloalkyle en C₆ saturé, dans lequel un élément de cycle est remplacé par un oxygène et qui est éventuellement substitué une fois par méthyle ou éthyle,
G représente l'hydrogène (a) ou un des groupes 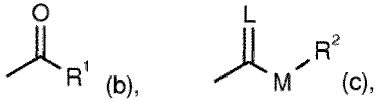 ou E (f), dans lesquels
L représente l'oxygène,
M représente l'oxygène et
E représente un équivalent d'ion métallique ou un ion ammonium,
R¹ représente alkyle en C₁-C₆, alcényle en C₂-C₆, alcoxy en C₁-C₂-alkyle en C₁, alkylthio en C₁-C₂-alkyle en C₁, chacun éventuellement substitués une fois par fluor ou chlore ; ou cyclopropyle ou cyclohexyle, chacun éventuellement substitués une fois par fluor, chlore, méthyle ou méthoxy ; phényle éventuellement substitué une fois par fluor, chlore, brome, cyano, nitro, méthyle, méthoxy, trifluorométhyle ou trifluorométhoxy,
R² représente alkyle en C₁-C₈, alcényle en C₂-C₆ ou alcoxy en C₁-C₄-alkyle en C₂-C₃, phényle ou benzyle, chacun éventuellement substitués une fois par fluor.

2. Procédé de fabrication de composés de formule (I) selon la revendication 1, **caractérisé en ce que**, pour obtenir
(A) des composés de formule (I-1-a) 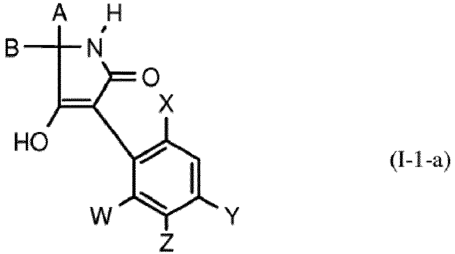 dans laquelle
A, B, W, X, Y et Z ont les significations indiquées dans la revendication 1,
des esters d'acides N-acylaminés de formule (II) 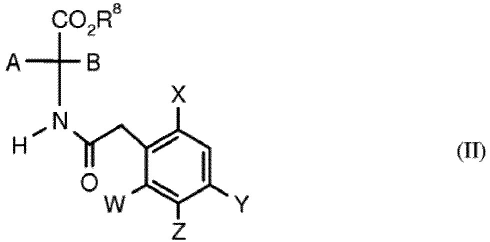 dans laquelle
A, B, W, X, Y et Z ont les significations indiquées dans la revendication 1,
et
R⁸ représente alkyle,
sont condensés intramoléculairement en présence d'un diluant et en présence d'une base,
(C) des composés des formules (I-1-a), (I-1-b), (I-1-c), (I-1-f) représentées précédemment, dans lesquelles A, B, G, W, X, Y et Z ont la signification indiquée dans la revendication 1, des composés de formule (I-1'-a), (I-1'-b), (I-1'-c), (I-1'-f), **(I-1'-a), (I-1'-b), (I-1'-c), (I-1'-f)** 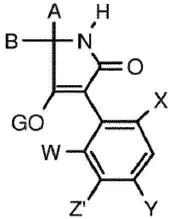 dans laquelle
A, B, G, W, X et Y ont la signification indiquée dans la revendication 1, et
Z' représente chlore, brome, iode, de préférence brome,
sont mis en réaction avec des acides boroniques ou des dérivés d'acides boroniques de formule (IV) 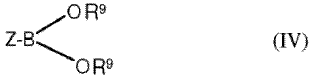 dans laquelle
R⁹ représente hydrogène, alkyle en C₁-C₆ ou alcanediyle en C₂-C₆,
et
Z a la signification indiquée dans la revendication 1,
en présence d'un solvant, d'une base et d'un catalyseur, des sels de palladium ou des complexes de palladium étant notamment envisagés en tant que catalyseur,
(D) des composés de la formule (I-1-b) représentée précédemment, dans laquelle A, B, R¹, W, X, Y et Z ont les significations indiquées dans la revendication 1, des composés de la formule (I-1-a) représentée précédemment, dans laquelle A, B, W, X, Y et Z ont les significations indiquées dans la revendication 1, sont mis en réaction respectivement
(α) avec des halogénures d'acides de formule (V) 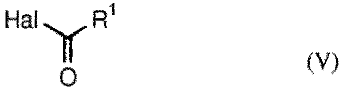 dans laquelle
R¹ a la signification indiquée dans la revendication 1 et
Hal représente halogène,
ou
(β) avec des anhydrides d'acides carboxyliques de formule (VI)
R¹-CO-O-CO-R¹ (VI)
dans laquelle
R¹ a la signification indiquée dans la revendication 1,
éventuellement en présence d'un diluant et éventuellement en présence d'un liant d'acide ;
(E) des composés de la formule (I-1-c) représentée précédemment, dans laquelle A, B, R², M, W, X, Y et Z ont les significations indiquées dans la revendication 1 et L représente l'oxygène, des composés de la formule (I-1-a) représentée précédemment, dans laquelle A, B, W, X, Y et Z ont les significations indiquées dans la revendication, sont mis en réaction respectivement avec des esters de l'acide chloroformique de formule (VII)
R²-M-CO-Cl (VII)
dans laquelle
R² et M ont les significations indiquées dans la revendication 1,
éventuellement en présence d'un diluant et éventuellement en présence d'un liant d'acide ;
(I) des composés de la formule (I-1-f) représentée précédemment, dans laquelle A, B, E, W, X, Y et Z ont les significations indiquées dans la revendication 1, des composés de la formule (I-1-a) représentée précédemment, dans laquelle A, B, W, X, Y et Z ont les significations indiquées dans la revendication 1, sont mis en réaction respectivement
avec des composés métalliques ou des amines des formules (XI) ou (XII)
**Me(OR¹⁰)ₜ** **(XI)** 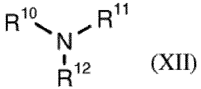
dans lesquelles
Me représente un métal mono- ou bivalent,
t représente le nombre 1 ou 2, et
R¹⁰, R¹¹, R¹² représentent indépendamment les uns des autres hydrogène ou alkyle,
éventuellement en présence d'un diluant.

3. Agent contenant une teneur efficace d'une combinaison d'agents actifs comprenant en tant que composants :
(a') au moins un céto-énol spirocyclique à substitution biphényle de formule (I) 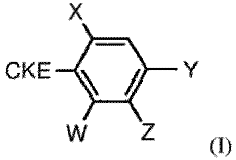 dans laquelle CKE, W, X, Y et Z ont la signification indiquée dans la revendication 1
et
(b') au moins un composé améliorant la compatibilité avec les plantes cultivées du groupe suivant de composés :
4-dichloroacétyl-1-oxa-4-aza-spiro[4.5]-décane (AD-67, MON-4660), 1-dichloro-acétyl-hexahydro-3,3,8a-triméthylpyrrolo[1,2-a]-pyrimidin-6(2H)-one (dicyclonone, BAS-145138), 4-dichloroacétyl-3,4-dihydro-3-méthyl-2H-1,4-benzoxazine (bénoxacor), ester 1-méthyl-hexylique de l'acide 5-chloro-quinolin-8-oxyacétique (cloquintocet-mexyl - voir également composés apparentés dans EP-A-86750, EP-A-94349, EP-A-191736, EP-A-492366), 3-(2-chloro-benzyl)-1-(1-méthyl-1-phényléthyl)-urée (cumyluron), α-(cyanométhoximino)-phénylacétonitrile (cyométrinil), acide 2,4-dichloro-phénoxyacétique (2,4-D), acide 4-(2,4-dichloro-phénoxy)-butyrique (2,4-DB), 1-(1-méthyl-1-phényléthyl)-3-(4-méthyl-phényl)-urée (daimuron, dymron), acide 3,6-dichloro-2-méthoxy-benzoïque (dicamba), ester S-1-méthyl-1-phényl-éthylique de l'acide pipéridine-1-thiocarboxylique (dimépipérate), 2,2-dichloro-N-(2-oxo-2-(2-propényl-amino)-éthyl)-N-(2-propényl)-acétamide (DKA-24), 2,2-dichloro-N,N-di-2-propényl-acétamide (dichlormid), 4,6-dichloro-2-phényl-pyrimidine (fenclorim), ester éthylique de l'acide 1-(2,4-dichloro-phényl)-5-trichlorométhyl-1H-1,2,4-triazole-3-carboxylique (fenchlorazole-éthyle - voir également composés apparentés dans EP-A-174562 et EP-A-346620), ester phénylméthylique de l'acide 2-chloro-4-trifluorométhyl-thiazole-5-carboxylique (flurazole), 4-chloro-N-(1,3-dioxolan-2-yl-méthoxy)-α-trifluoro-acétophénonoxime (fluxofénim), 3-dichloroacétyl-5-(2-furanyl)-2,2-diméthyl-oxazolidine (furilazole, MON-13900), carboxylate d'éthyl-4,5-dihydro-5,5-diphényl-3-isoxazole (isoxadifen-éthyle - voir également composés apparentés dans WO-A-95/07897), 3,6-dichloro-2-méthoxybenzoate de 1-(éthoxy-carbonyl)-éthyle (lactidichlor), acide (4-chloro-o-tolyl-oxy)-acétique (MCPA), acide 2-(4-chloro-o-tolyloxy)-propionique (mécoprop), di-éthyl-1-(2,4-dichloro-phényl)-4,5-dihydro-5-méthyl-1H-pyrazole-3,5-dicarboxylate (méfenpyr-diéthyl - voir également composés apparentés dans WO-A-91/07874), 2-dichlorométhyl-2-méthyl-1,3-dioxolane (MG-191), 4-carbodithioate de 2-propényl-1-oxa-4-azaspiro-[4.5]décane (MG-838), anhydride de l'acide 1,8-naphtalique, α-(1,3-dioxolan-2-yl-méthoximino)-phénylacétonitrile (oxabétrinil), 2,2-dichloro-N-(1,3-dioxolan-2-yl-méthyl)-N-(2-propényl)-acétamide (PPG-1292), 3-dichloroacétyl-2,2-diméthyl-oxazolidine (R-28725), 3-dichloroacétyl-2,2,5-triméthyl-oxazolidine (R-29148), acide 4-(4-chloro-o-tolyl)-butyrique, acide 4-(4-chloro-phénoxy)-butyrique, acide diphénylméthoxyacétique, ester méthylique de l'acide diphénylméthoxyacétique, ester éthylique de l'acide diphénylméthoxyacétique, ester méthylique de l'acide 1-(2-chloro-phényl)-5-phényl-1H-pyrazole-3-carboxylique, ester éthylique de l'acide 1-(2,4-dichloro-phényl)-5-méthyl-1H-pyrazole-3-carboxylique, ester éthylique de l'acide 1-(2,4-dichloro-phényl)-5-isopropyl-1H-pyrazole-3-carboxylique, ester éthylique de l'acide 1-(2,4-dichloro-phényl)-5-(1,1-diméthyl-éthyl)-1H-pyrazole-3-carboxylique, ester éthylique de l'acide 1-(2,4-dichloro-phényl)-5-phényl-1H-pyrazole-3-carboxylique (voir également composés apparentés dans EP-A-269806 et EP-A-333131), ester éthylique de l'acide 5-(2,4-dichloro-benzyl)-2-isoxazoline-3-carboxylique, ester éthylique de l'acide 5-phényl-2-isoxazoline-3-carboxylique, ester éthylique de l'acide 5-(4-fluorophényl)-5-phényl-2-isoxazoline-3-carboxylique (voir également composés apparentés dans WO-A-91/08202), ester 1,3-diméthyl-but-1-ylique de l'acide 5-chloro-quinolin-8-oxy-acétique, ester 4-allyloxy-butylique de l'acide 5-chloro-quinolin-8-oxy-acétique, ester 1-allyloxy-prop-2-ylique de l'acide 5-chloro-quinolin-8-oxy-acétique, ester méthylique de l'acide 5-chloro-quinoxalin-8-oxy-acétique, ester éthylique de l'acide 5-chloro-quinolin-8-oxy-acétique, ester allylique de l'acide 5-chloro-quinoxalin-8-oxy-acétique, ester 2-oxo-prop-1-ylique de l'acide 5-chloro-quinolin-8-oxy-acétique, ester diéthylique de l'acide 5-chloro-quinolin-8-oxy-malonique, ester diallylique de l'acide 5-chloro-quinoxalin-8-oxy-malonique, ester diéthylique de l'acide 5-chloro-quinolin-8-oxy-malonique (voir également composés apparentés dans EP-A-582198), acide 4-carboxy-chroman-4-yl-acétique (AC-304415, voir EP-A-613618), acide 4-chloro-phénoxy-acétique, 3,3'-diméthyl-4-méthoxy-benzophénone, 1-bromo-4-chlorométhylsulfonyl-benzène, 1-[4-(N-2-méthoxybenzoylsulfamoyl)-phényl]-3-méthyl-urée (alias N-(2-méthoxy-benzoyl)-4-[(méthylamino-carbonyl)-amino]-benzène-sulfonamide), 1-[4-(N-2-méthoxybenzoylsulfamoyl)-phényl]-3,3-diméthyl-urée, 1-[4-(N-4,5-diméthylbenzoylsulfamoyl)-phényl]-3-méthyl-urée, 1-[4-(N-naphtylsulfamoyl)-phényl]-3,3-diméthyl-urée, N-(2-méthoxy-5-méthyl-benzoyl)-4-(cyclopropylaminocarbonyl)-benzène-sulfonamide, et/ou un des composés suivants définis par les formules générales :
la formule générale (IIa) 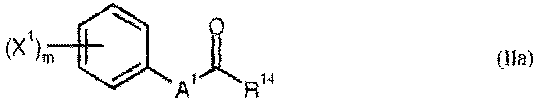
ou la formule générale (IIb) 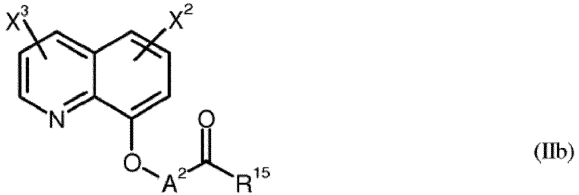 ou la formule (IIc) 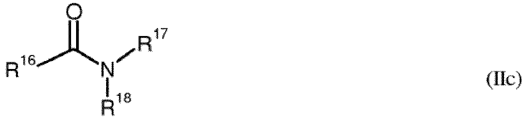 dans lesquelles
m représente un nombre 0, 1, 2, 3, 4 ou 5,
A¹ représente un des groupes hétérocycliques bivalents représentés ci-après : 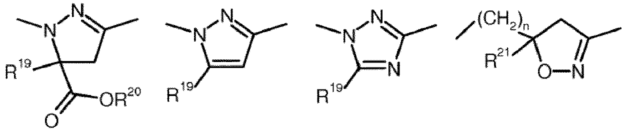
n représente un nombre 0, 1, 2, 3, 4 ou 5,
A² représente alcanediyle éventuellement substitué par alkyle en C₁-C₄ et/ou alcoxy-carbonyle en C₁-C₄ et/ou alcényloxy-carbonyle en C₁-C₄, contenant 1 ou 2 atomes de carbone,
R¹⁴ représente hydroxy, mercapto, amino, alcoxy en C₁-C₆, alkylthio en C₁-C₆, alkylamino en C₁-C₆ ou di-(alkyle en C₁-C₄)-amino,
R¹⁵ représente hydroxy, mercapto, amino, alcoxy en C₁-C₇, alcényloxy en C₁-C₆, alcényloxy en C₁-C₆-alcoxy en C₁-C₆, alkylthio en C₁-C₆, alkylamino en C₁-C₆ ou di-(alkyle en C₁-C₄)-amino,
R¹⁶ représente alkyle en C₁-C₄ éventuellement substitué par fluor, chlore et/ou brome,
R¹⁷ représente hydrogène, alkyle en C₁-C₆, alcényle en C₂-C₆ ou alcynyle en C₂-C₆, alcoxy en C₁-C₄-alkyle en C₁-C₄, dioxolanyl-alkyle en C₁-C₄, furyle, furyl-alkyle en C₁-C₄, thiényle, thiazolyle, pipéridinyle, chacun éventuellement substitués par fluor, chlore et/ou brome ; ou phényle éventuellement substitué par fluor, chlore et/ou brome ou alkyle en C₁-C₄,
R¹⁸ représente hydrogène, alkyle en C₁-C₆, alcényle en C₂-C₆ ou alcynyle en C₂-C₆, alcoxy en C₁-C₄-alkyle en C₁-C₄, dioxolanyl-alkyle en C₁-C₄, furyle, furyl-alkyle en C₁-C₄, thiényle, thiazolyle, pipéridinyle, chacun éventuellement substitués par fluor, chlore et/ou brome ; ou phényle éventuellement substitué par fluor, chlore et/ou brome ou alkyle en C₁-C₄, R¹⁷ et R¹⁸ représentant également ensemble alcanediyle en C₃-C₆ ou oxa-alcanediyle en C₂-C₅, chacun éventuellement substitués par alkyle en C₁-C₄, phényle, furyle, un cycle benzène annelé ou par deux substituants, qui forment ensemble avec l'atome C auquel ils sont reliés un carbocycle à 5 ou 6 chaînons,
R¹⁹ représente hydrogène, cyano, halogène ; ou alkyle en C₁-C₄, cycloalkyle en C₃-C₆ ou phényle, chacun éventuellement substitués par fluor, chlore et/ou brome,
R²⁰ représente hydrogène, alkyle en C₁-C₆, cycloalkyle en C₃-C₆ ou tri-(alkyle en C₁-C₄) -silyle, chacun éventuellement substitués par hydroxy, cyano, halogène ou alcoxy en C₁-C₄,
R²¹ représente hydrogène, cyano, halogène ; ou alkyle en C₁-C₄, cycloalkyle en C₃-C₆ ou phényle, chacun éventuellement substitués par fluor, chlore et/ou brome,
X¹ représente nitro, cyano, halogène, alkyle en C₁-C₄, halogénoalkyle en C₁-C₄, alcoxy en C₁-C₄ ou halogénoalcoxy en C₁-C₄,
X² représente hydrogène, cyano, nitro, halogène, alkyle en C₁-C₄, halogénoalkyle en C₁-C₄, alcoxy en C₁-C₄ ou halogénoalcoxy en C₁-C₄,
X³ représente hydrogène, cyano, nitro, halogène, alkyle en C₁-C₄, halogénoalkyle en C₁-C₄, alcoxy en C₁-C₄ ou halogénoalcoxy en C₁-C₄,
et/ou les composés suivants définis par les formules générales :
la formule générale (IId) 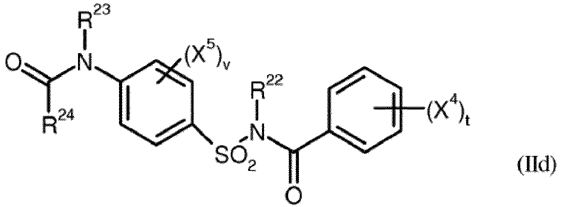
ou la formule générale (IIe) 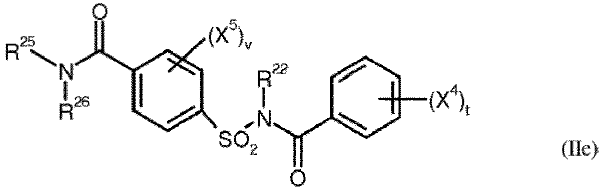 dans lesquelles
t représente un nombre 0, 1, 2, 3, 4 ou 5,
v représente un nombre 0, 1, 2, 3, 4 ou 5,
R²² représente hydrogène ou alkyle en C₁-C₄,
R²³ représente hydrogène ou alkyle en C₁-C₄,
R²⁴ représente hydrogène, alkyle en C₁-C₆, alcoxy en C₁-C₆, alkylthio en C₁-C₆, alkylamino en C₁-C₆ ou di-(alkyle en C₁-C₄)-amino, chacun éventuellement substitués par cyano, halogène ou alcoxy en C₁-C₄ ; ou cycloalkyle en C₃-C₆, cycloalkyloxy en C₃-C₆, cycloalkylthio en C₃-C₆ ou cycloalkylamino en C₃-C₆, chacun éventuellement substitués par cyano, halogène ou alkyle en C₁-C₄,
R²⁵ représente hydrogène, alkyle en C₁-C₆ éventuellement substitué par cyano, hydroxy, halogène ou alcoxy en C₁-C₄ ; alcényle en C₃-C₆ ou alcynyle en C₃-C₆, chacun éventuellement substitués par cyano ou halogène ; ou cycloalkyle en C₃-C₆ éventuellement substitué par cyano, halogène ou alkyle en C₁-C₄,
R²⁶ représente hydrogène, alkyle en C₁-C₆ éventuellement substitué par cyano, hydroxy, halogène ou alcoxy en C₁-C₄ ; alcényle en C₃-C₆ ou alcynyle en C₃-C₆, chacun éventuellement substitués par cyano ou halogène ; cycloalkyle en C₃-C₆ éventuellement substitué par cyano, halogène ou alkyle en C₁-C₄ ; ou phényle éventuellement substitué par nitro, cyano, halogène, alkyle en C₁-C₄, halogénoalkyle en C₁-C₄, alcoxy en C₁-C₄ ou halogénoalcoxy en C₁-C₄ ; ou représente conjointement avec R²⁵ alcanediyle en C₂-C₆ ou oxa-alcanediyle en C₂-C₅, chacun éventuellement substitués par alkyle en C₁-C4,
X⁴ représente nitro, cyano, carboxy, carbamoyle, formyle, sulfamoyle, hydroxy, amino, halogène, alkyle en C₁-C₄, halogénoalkyle en C₁-C₄, alcoxy en C₁-C₄ ou halogénoalcoxy en C₁-C₄, et
X⁵ représente nitro, cyano, carboxy, carbamoyle, formyle, sulfamoyle, hydroxy, amino, halogène, alkyle en C₁-C₄, halogénoalkyle en C₁-C₄, alcoxy en C₁-C₄ ou halogénoalcoxy en C₁-C₄.

4. Agent selon la revendication 3, dans lequel le composé améliorant la compatibilité avec les plantes cultivées est choisi dans le groupe suivant de composés :
cloquintocet-mexyl, fenchlorazole-éthyl, isoxadifen-éthyl, méfenpyr-diéthyl, furilazole, fenclorim, cumyluron, dymron ou les composés 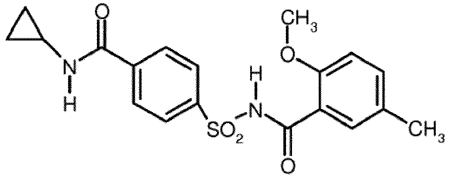 et

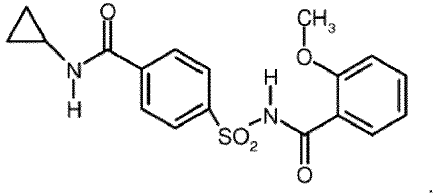

5. Agents selon l'une quelconque des revendications 3 ou 4, dans lesquels le composé améliorant la compatibilité avec les plantes cultivées est le cloquintocet-mexyl.

6. Agents selon l'une quelconque des revendications 3 ou 4, dans lesquels le composé améliorant la compatibilité avec les plantes cultivées est le méfenpyr-diéthyl.

7. Composition comprenant :
- au moins un composé de formule (I) selon la revendication 1 ou un agent selon la revendication 3, et
- au moins un sel de formule (III') 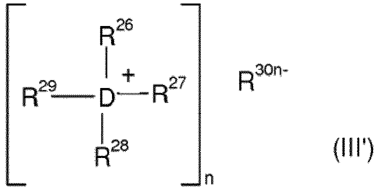 dans laquelle
D représente azote ou phosphore,
R²⁶, R²⁷, R²⁸ et R²⁹ représentent indépendamment les uns des autres hydrogène ou alkyle en C₁-C₈ éventuellement substitué ou alkylène en C₁-C₈ mono- ou polyinsaturé éventuellement substitué, les substituants pouvant être choisis parmi halogène, nitro et cyano,
n représente 1, 2, 3 ou 4,
R³⁰ représente un anion inorganique ou organique.

8. Composition selon la revendication 7, **caractérisée en ce qu'**elle contient au moins un promoteur de pénétration.

9. Utilisation de composés de formule (I) selon la revendication 1 pour la fabrication d'agents de lutte contre des nuisibles et/ou d'herbicides.

10. Agents de lutte contre des nuisibles et/ou herbicides, **caractérisés par** une teneur en au moins un composé de formule (I) selon la revendication 1.

11. Procédé de lutte contre des animaux nuisibles et/ou une végétation indésirable, **caractérisé en ce que** des composés de formule (I) selon la revendication 1 sont laissés agir sur des nuisibles et/ou leur habitat, les procédés de traitement chirurgical ou thérapeutique du corps humain ou animal étant exclus.

12. Utilisation de composés de formule (I) selon la revendication 1 pour lutter contre des animaux nuisibles et/ou une végétation indésirable, l'utilisation pour le traitement chirurgical ou thérapeutique du corps humain ou animal étant exclue.

13. Procédé de fabrication d'agents de lutte contre des nuisibles et/ou d'herbicides, **caractérisé en ce que** des composés de formule (I) selon la revendication 1 sont mélangés avec des extendeurs et/ou des substances tensioactives.

14. Procédé de lutte contre une végétation indésirable, **caractérisé en ce qu'**un agent selon la revendication 3 est laissé agir sur les plantes ou leur environnement.

15. Utilisation d'un agent selon la revendication 3 pour lutter contre une végétation indésirable.

16. Procédé de lutte contre une végétation indésirable, **caractérisé en ce que** des composés de formule (I) selon la revendication 1 et le composé améliorant la compatibilité avec les plantes cultivées selon la revendication 3 sont laissés agir séparément en succession proche dans le temps sur les plantes ou leur environnement.

17. Procédé d'augmentation de l'effet d'agents de lutte contre des nuisibles et/ou d'herbicides contenant un agent actif de formule (I) selon la revendication 1 ou un agent selon la revendication 3, **caractérisé en ce que** l'agent prêt à l'emploi (bouillie de pulvérisation) est préparé en utilisant un sel de formule (III') selon la revendication 7, les procédés de traitement chirurgical ou thérapeutique du corps humain ou animal étant exclus.

18. Procédé selon la revendication 17, **caractérisé en ce que** la bouillie de pulvérisation est préparée en utilisant un promoteur de pénétration.

19. Composés de formule (II) 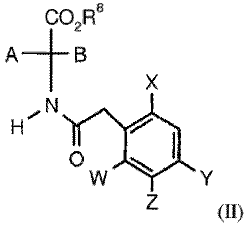 dans laquelle
A, B, R⁸, W, X, Y et Z ont les significations indiquées dans la revendication 1 ou 2.

20. Composés de formule (XVII) 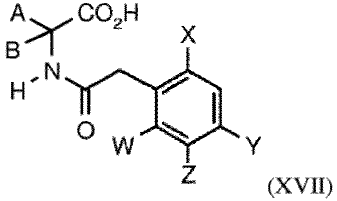 dans laquelle
A, B, W, X, Y et Z ont les significations indiquées dans la revendication 1.

21. Composés de formule (XXI) 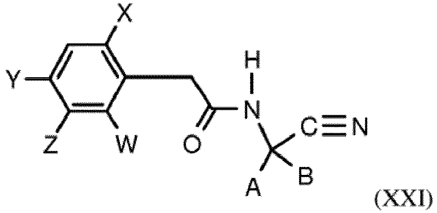 dans laquelle
A, B, W, X, Y et Z ont les significations indiquées dans la revendication 1.

22. Composés de formule (XXIII) 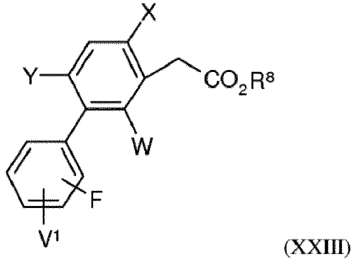 dans laquelle les substituants W, X, Y, F, V¹ et R⁸ ont les significations indiquées dans le tableau :
| W | X | Y | F | V¹ | R⁸ |
|---|---|---|---|---|---|
| H | CH₃ | H | 4 | 3-F | CH₃ |
| H | CH₃ | H | 3-F | 4-Cl | CH₃ |
| CH₃ | CH₃ | H | 4-F | 3-F | CH₃ |
| CH₃ | CH₃ | H | 3-F | 4-Cl | CH₃ |

23. Composés de formule (XIX) 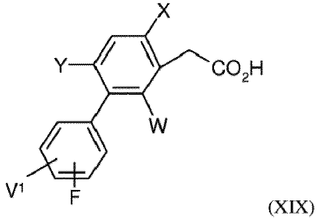 dans laquelle W, X, Y, F, V¹ ont les significations indiquées dans le tableau :
| W | X | Y | F | V¹ |
|---|---|---|---|---|
| H | CH₃ | H | 4 | 3-F |
| H | CH₃ | H | 3 | 4-Cl |
| CH₃ | CH₃ | H | 4 | 3-F |
| CH₃ | CH₃ | H | 3 | 4-Cl |
